# EUROPEAN PATENT APPLICATION

(11) **EP 4 174 092 A1**
(43) Date of publication of application: **03.05.2023**
(21) Application number: 21833675.8
(22) Date of filing: 29.06.2021
(51) Int. Cl.: C07K 16/46, C12N 15/13, A61K 39/395, A61P 35/00

(54) **MULTISPECIFIC BINDING PROTEIN OF IMMUNE CELL ENGAGER, PREPARATION THEREFOR AND APPLICATION THEREOF**

(30) Priority: 30.06.2020 CN 202010630471
(71) Applicant: Harbour Biomed (Shanghai) Co., Ltd, Shanghai 201203 (CN)
(72) Inventor: HE, Yun, Shanghai 201203 (CN); SHI, Lei, Shanghai 201203 (CN); WU, Xiaodong, Shanghai 201203 (CN); ZHONG, Chen, Shanghai 201203 (CN); HUANG, Bing, Shanghai 201203 (CN); RONG, Yiping, Shanghai 201203 (CN)
(74) Representative: Henderson, Helen Lee
(86) International application number: PCT/CN2021/103065
(87) International publication number: WO 2022/002038

(57) **Abstract**

Provided are a binding protein and an application thereof. The binding protein comprises a first protein function region bound to a targeting domain of an antigen on a target cell, and a second protein function region comprising an effect domain bound to an antigen on an immune effector cell, and is selected from the following structure: the first protein function region is a Fab structure; the second protein function region is a VH structure or a Fab structure; the binding protein comprises the first and second protein function regions from an N-terminus to a C-terminus in sequence. The binding protein can bring immune effector cells and specific target cells closer together, and efficiently and selectively activate the immune effector cells in the microenvironment of specific target cells, thereby enhancing an immune response and reducing the toxic side effects caused by non-specific activation of peripheral immune cells. The binding protein utilizes the cross-linking of costimulatory molecules mediated by target cell antigens and the activation of downstream signal transduction pathways to promote the activation and proliferation of immune cells and improve the effectiveness in killing tumors.

## Description

The present application claims priority to Chinese Patent Application No. 202010630471.6 filed on Jun. 30, 2020, the content of which is incorporated herein by reference in its entirety.

### TECHNICAL FIELD

The present invention relates to the field of biological pharmacy, in particular to a multispecific binding protein with tumor targeting function and immune cell redirecting ability for an immune cell engager, and preparation and use thereof.

### BACKGROUND

Bispecific antibodies and multispecific antibodies are a class of artificial antibodies with two or more different specific antigen-binding sites prepared by protein engineering techniques based on natural monoclonal antibodies. The natural monoclonal antibodies are monospecific, i.e., capable of recognizing and binding to only one antigen; the bispecific antibodies can bind to two different antigens or different epitopes on the same antigen; while the multispecific antibodies may recognize more antigens.

In recent years, tumor immunotherapies for the treatment of cancer by modulating immune responses have shown great potential, enabling tumor immunology-related bispecific antibodies to attract increasing attention and technical and financial support. Multispecific immune cell engager antibody molecules can simultaneously recognize a specific antigen on a tumor cell and a CD3 molecule or other costimulatory molecules on an immune cell, thereby bringing the immune cell and the tumor cell together, efficiently and selectively activating the immune cell in a tumor microenvironment, and reducing toxic and side effects caused by non-specific activation of peripheral immune cells. Bispecific T-cell engagers (BsTCEs) based on targeting CD3 are a class of immune cell engagers targeting CD3 and tumor targets. In the bispecific antibody projects currently in clinical or preclinical stage worldwide, nearly half of the bispecific antibodies are bispecific antibodies targeting CD3. BsTCE has shown potent efficacy in the field of hematological tumor diseases and has been fully verified in the clinic, e.g., blinatumomab (targeting CD19×CD3) approved by the FDA for marketing in 2014 for the treatment of Philadelphia chromosome-negative B cell lymphoma. However, the effect of BsTCE on solid tumors has not been fully verified.

Activation of T cells generally requires to be triggered by TCR-MHC interaction, but the CD3-targeted immune cell engager (CD3BsTCE) molecule activates T cells in a non-MHC restricted manner, which can overcome immune escape mechanisms caused by tumor cell MHC down-regulation or antigen-presenting cell tolerance. However, in the absence of costimulatory signals, T cell pathways in the tumor microenvironment are not fully activated. This may be the reason why the effect of CD3BsTCE on solid tumors was not fully exerted. In another aspect, immune cell engager molecules targeting costimulatory molecules on the surface of T cells can cause the crosslinking of the costimulatory molecules mediated by tumor antigens and the activation of downstream signaling pathways to provide costimulatory signals for the complete activation of the T cells, thereby fully activating the T cells and promoting proliferation, and improving the efficacy of killing the tumors.

4-1BB (TNFRSF9, CD137) is a transmembrane protein belonging to the TNF receptor superfamily. 4-1BB is a costimulatory molecule expressed on a variety of immune cells. It is a multifunctional modulator of immune activity. Its expression is induced in activated T cells, NK cells and other immune cells. 4-1BB activates T cells through trimerization mediated by its ligand 4-1BBL, thereby promoting cell proliferation and cytokine release. Anti-4-1BB agonistic antibodies have the function of inhibiting tumors. The first 4-1BB antibodies to be subjected to clinical trials were utomilumab from Pfizer and urelumab (BMS-663513) from Bristol-Myers Squibb (BMS). The initial clinical results of urelumab were published in 2008. Although encouraging efficacy was observed in some patients, the data showed urelumab to cause target and dose-associated hepatotoxicity. Utomilumab has better safety enabling the dose to be increased to 10 mg/kg, but still has a poor therapeutic effect. The core problem of the development of 4-1BB-targeted drugs is how to properly activate immune cells through 4-1BB to achieve a balance between efficacy and safety.

OX40 (TNFRSF4, CD134), one of the TNF receptor superfamily members, is involved in enhancing T cell receptor (TCR)-triggered T cell responses, and is a costimulatory receptor molecule. It is a transmembrane protein of 50 kD. OX40 is transiently expressed on human CD4⁺ and CD8⁺ T cells after TCR stimulation. However, at the tumor site, OX40 is more highly expressed on CD4⁺ T cells than on CD8⁺ T cells. Thus, CD4⁺ and CD8⁺ T cells are potential targets of OX40-directed immunotherapy of cancer. Some preclinical studies on OX40 antibodies have shown that anti-OX40 monoclonal antibodies produce deleterious immunosuppressive side effects by promoting MDSC accumulation and Th2 cytokine production.

Given the known difficulties and unmet clinical needs, there is an urgent need to develop novel antibody molecules capable of mediating the activation of specific immune cells using target cells in a tumor microenvironment to avoid the toxic and side effects caused by their activation in the peripheral system or normal tissues. Meanwhile, those novel antibody molecules need to have a relatively simple and stable molecular structures and excellent pharmaceutical properties to meet the needs of rapid development and reduced production costs.

The basic structure of antibodies of human is in a "Y"-type tetrameric form, comprising two identical heavy chains (H chains) and two identical light chains (L chains), which are also referred to as "H2L2". A heavy-chain antibody (HCAb) lacking light chains is naturally present in the sera of Camelidae species and sharks. The heavy-chain antibody derived from Camelidae species has no heavy chain constant domain CH1 region between the heavy chain variable region and the hinge region thereof, and contains only one heavy chain variable region (VHH) and two heavy chain constant domains (CH2 and CH3, i.e., Fc), in addition to the lack of light chains, as compared to the antibodies with a conventional H2L2 structure (e.g., IgG antibodies); and the basic structure thereof is a heavy chain dimer. The VHH fragment of the heavy-chain antibody of Camelidae species has different characteristics from the VH of the conventional antibodies, and the VHH structure cloned and expressed separately has structural stability and antigen-binding activity that are comparable to the original heavy-chain antibody. The heavy-chain antibody of Camelidae species has a molecular weight of only about 13 KDa, and is therefore also referred to as a nanobody or a single-domain antibody. The heavy-chain antibody or the nanobody derived therefrom have unique advantages in molecular imaging, diagnostic reagents, etc., but its therapeutic uses are limited by its non-human nature and potential immunogenic risk, which requires further antibody engineering (e.g., antibody humanization) to meet the requirements for clinical treatment.

In the H2L2 structure of the human antibody, the association of the heavy chain variable region VH and the light chain variable region VL ensures the stability and solubility of the antibody; if there is no VL, the hydrophobic groups on the VH that would otherwise be protected by VL will be exposed to aqueous solvents, which makes VH prone to aggregation, thereby leading to poor antibody solubility, and losing antigen-binding ability contributed by VL. Therefore, useful human heavy-chain antibodies cannot be obtained from natural sources. Harbour HCAb transgenic mouse (Harbour antibodies BV) was derived from a technique proposed by Frank Grosveld et al. for obtaining a fully human heavy-chain antibody using a transgenic animal (Patent Application WO2007/096779). Frank Grosveld et al. constructed a transgenic mouse, specifically, the endogenous antibody heavy chain locus and light chain locus of the mouse were both knocked out or inactivated, making it impossible to produce mouse antibodies; then, the heavy chain gene fragments of human antibody (V, D, and J fragments) were transferred into the mouse to produce an antibody with a human antibody gene sequence by the rearrangement and mutation mechanisms of the mouse itself, and the produced antibody was the human heavy-chain antibody due to the absence of the light chain. The VDJ combinations and mutations which are beneficial to the solubility of VH can be selected by introducing gene mutations and performing natural selection after VDJ rearrangement in the transgenic mouse, so as to effectively improve the solubility of VH. Therefore, a non-naturally occurring human heavy-chain dimer structure can be produced in the transgenic mouse. The fully human heavy-chain antibody obtained from the transgenic mouse and the fully human single-domain antibody derived therefrom have wide application prospect.

Heavy-chain antibodies and single-domain antibodies derived therefrom have unique advantages in the construction of bispecific or even multispecific antibodies. The heavy-chain antibody has an antigen-binding domain that is only one quarter the size of the antigen-binding fragment Fab of a conventional antibody, and does not have light chains, so that the problem of light chain mismatch is avoided. The serum half-life of the molecule can also be increased by fusing the fragments crystallizable Fcs of the antibody. Therefore, bispecific or even multispecific antibodies with smaller molecular weight, less polypeptide chains and simpler structures can be constructed using heavy-chain antibodies and single-domain antibodies derived therefrom. Furthermore, fully human heavy-chain antibodies are more advantageous in terms of immunogenicity and druggability than nanobodies of Camelidae species.

### SUMMARY

The present invention aims to solve the technical problems to overcome the defects of antibody molecules for immune cell engagers at present by providing a binding protein for an immune cell engager. The binding protein of the present invention is multivalent and multispecific, and is able to bind to two or more antigens, or two or more epitopes. The present invention further provides a method for preparing such multivalent and multispecific binding proteins and use thereof, including a method for using such binding proteins to prevent or treat various diseases, or to detect specific antigens *in vitro* or *in vivo.* The binding protein of the present invention can enhance the immune response (e.g., the activity of T cells or NK cells) against a specific target cell (e.g., a tumor cell), and can bring an immune effector cell and a specific target cell (e.g., a tumor cell) together, and efficiently and selectively activate the immune effector cell in a microenvironment of the specific target cell (e.g., a tumor cell), thereby enhancing immune responses (e.g., promoting the activation of T cells and the production of cytokines), and reducing toxic and side effects caused by non-specific activation of peripheral immune cells. By utilizing the crosslinking of the costimulatory molecules mediated by target cell antigens and the activation of downstream signaling pathways, the binding proteins promote the activation and proliferation of the immune cells, and improve the efficacy of killing the tumors. The binding protein of the present invention has excellent universality and druggability, and shows excellent biological activity, molecular stability and pharmaceutical properties on a plurality of bispecific antibody molecules with different combinations of targets.

In order to solve the above technical problems, a first aspect of the present invention provides a binding protein comprising at least two protein functional regions. The binding protein of the present invention may be generally referred to as "multispecific immune cell engager binding protein", which comprises a first protein functional region and a second protein functional region, wherein the first protein functional region comprises a targeting moiety binding to an antigen on a target cell (which may bind to specific antigens on one or more target cells), and the second protein functional region comprises an effector moiety binding to an antigen on an immune effector cell (which may bind to specific antigens on one or more immune effector cells).

The binding protein may be of structure (I):
the first protein functional region is of a Fab structure, the second protein functional region is of a VH structure, and the first protein functional region and the second protein functional region are arranged from the N-terminus to the C-terminus of the binding protein, wherein the first protein functional region and the second protein functional region are directly or indirectly connected. For example, the binding protein is of a Fab-VH-Fc (Fab-HCAb) or Fab-Fc-VH (IgG-VH) structure.

The binding protein may be of structure (II):
the first protein functional region is of a Fab structure, the second protein functional region is of a Fab structure, and the first protein functional region and the second protein functional region are arranged from the N-terminus to the C-terminus of the binding protein, wherein the first protein functional region and the second protein functional region are directly or indirectly connected. For example, the binding protein is of an FIT-Ig structure.

Preferably, the binding protein is of (a) a monomeric structure, or (b) a dimeric structure consisting of two monomers.

In the present invention, the monomeric structure refers to a monomer containing a single structure (I) or a single structure (II), which generally contains only one first protein functional region. The dimeric structure consists of two monomers, i.e., generally refers to a dimer containing two structures (I) or (II), which generally contains two first protein functional regions and at least two second protein functional regions.

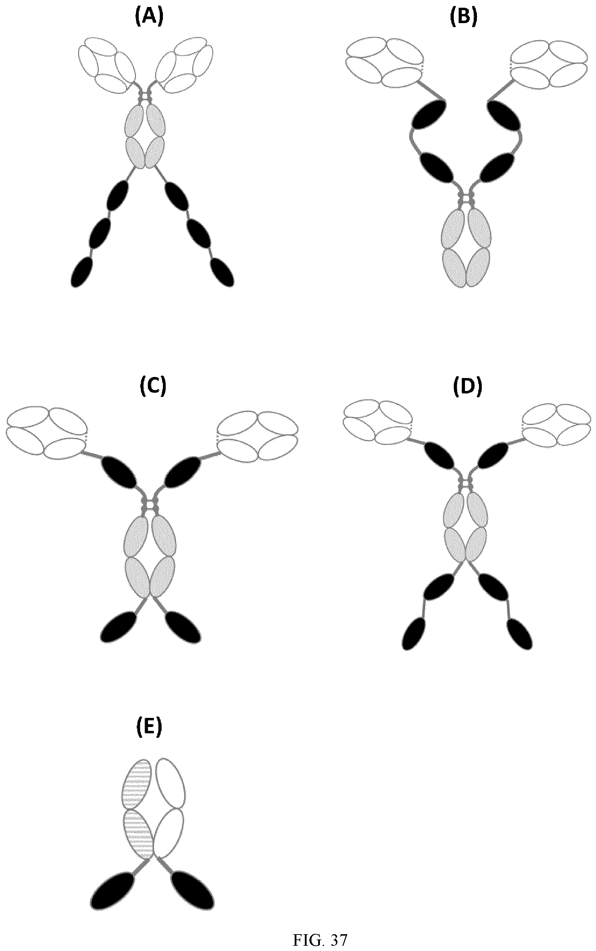

Preferably, in the monomeric structure of the (a), the binding protein has two second protein functional regions, one of which is linked to the C-terminus of the heavy chain CH1 of the Fab structure of the first protein functional region, and the other of which is linked to the C-terminus of the light chain CL of the Fab structure of the first protein functional region (e.g., as shown in FIG. 37 (E)).

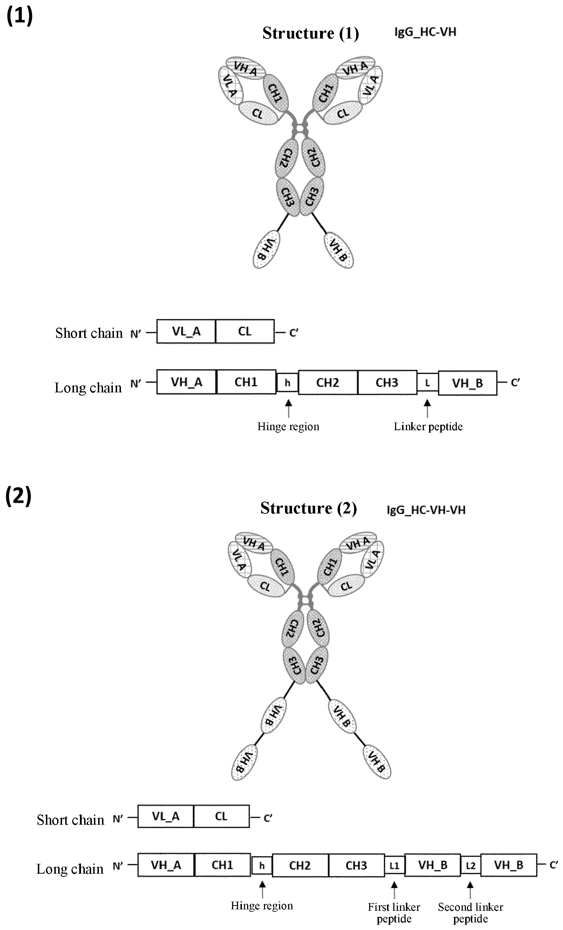

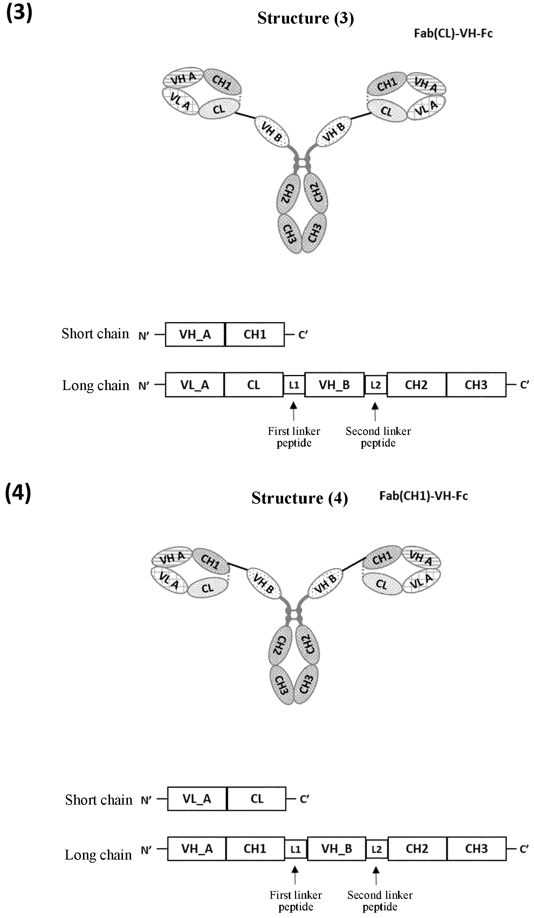

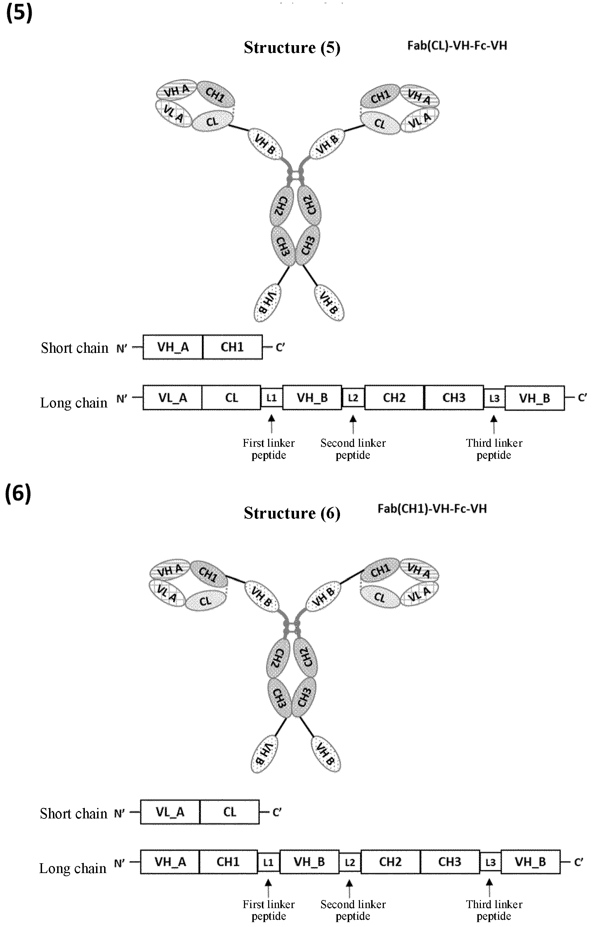

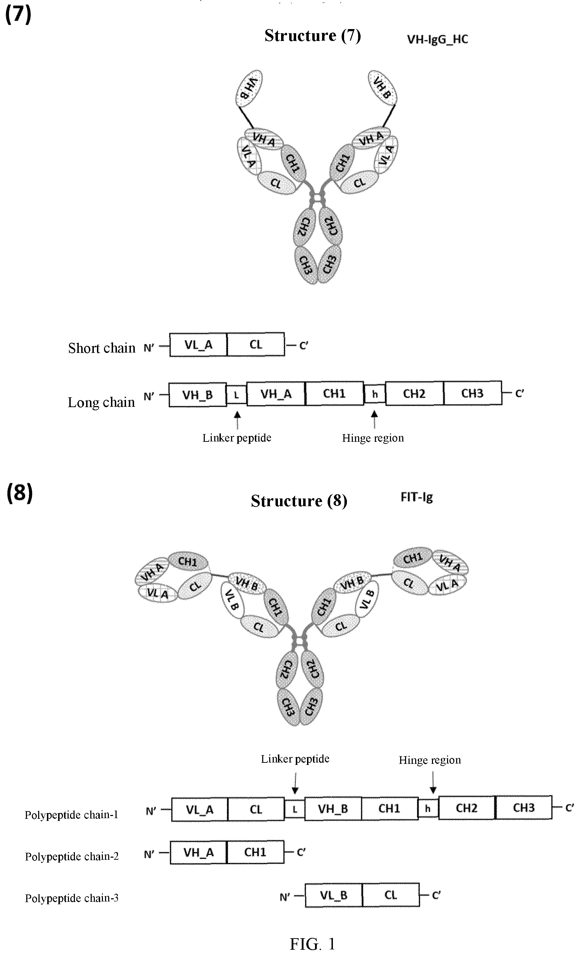

Preferably, in the dimeric structure of the (b), the binding protein generally further comprises an Fc. The Fc may be positioned between the first protein functional region and the second protein functional region (e.g., as shown in structures (1) and (2) in FIG. 1, or as shown in FIG. 37 (A)); or the Fc may be positioned at the C-terminus of the second protein functional region (e.g., as shown in structures (3), (4) and (8) in FIG. 1, or as shown in FIG. 37 (B-D)); or the Fc may be positioned between two second protein functional regions (e.g., as shown in structure (6) in FIG. 1). The number of the Fc may be generally 1 or more than one. In the present application, the Fc may be preferably a heavy chain constant region of human IgG1, human IgG2, human IgG3 or human IgG4, which may generally further comprise one, two or three mutations of L234A, L235A and P329G, for example, a mutation comprising L234A and L235A, or a mutation comprising L234A, L235A and P329G.

More preferably, in each monomer, the number of the first protein functional region is 1.

More preferably, in each monomer, the number of the second protein functional region is 1 or more than one, e.g., 1, 2 or 3.

Even more preferably, the binding protein may generally further comprise a third protein functional region, wherein the third protein functional region is generally positioned at the C- or N-terminus of the binding protein or the Fc of the binding protein. The number of the third protein functional region is 1 or more than one, e.g., 1, 2 or 3. The third protein functional region binds to an antigen on an additional target cell or to different epitopes of the same antigen as the first protein functional region on the target cell; or binds to an antigen on an additional immune effector cell or to different epitopes of the same antigen as the second protein functional region on the immune effector cell. The third protein functional region is preferably one or more VH.

In a certain preferred embodiment, the binding protein comprises a polypeptide chain 1 and a polypeptide chain 2, wherein the polypeptide chain 1 is as shown in a formula of N'-VL_A-CL-C', and the polypeptide chain 2 is as shown in a formula of N'-VH_A-CH1-h-CH2-CH3-L-VH_B-C', wherein the L is a linker peptide being preferably 0 in length or having an amino acid sequence preferably as set forth in any one of SEQ ID NOs: 244-265 or an amino acid sequence of GS (for example, as shown in structure 1 in FIG. 1).

In a certain preferred embodiment, the binding protein comprises a polypeptide chain 1 and a polypeptide chain 2, wherein the polypeptide chain 1 is as shown in a formula of N'-VL_A-CL-C', and the polypeptide chain 2 is as shown in a formula of N'-VH_A-CH1-h-CH2-CH3-L1-VH_B-L2-VH_B-C', wherein the L1 and L2 are linker peptides being preferably 0 in length or having an amino acid sequence preferably as set forth in any one of SEQ ID NOs: 244-265 or an amino acid sequence of GS (for example, as shown in structure 2 in FIG. 1).

In a certain preferred embodiment, the binding protein comprises a polypeptide chain 1 and a polypeptide chain 2, wherein the polypeptide chain 1 is as shown in a formula of N'-VH_A-CH1-C', and the polypeptide chain 2 is as shown in a formula of N'-VL_A-CL-L1-VH_B-L2-CH2-CH3-C', wherein the L1 and L2 are linker peptides being preferably 0 in length or having an amino acid sequence preferably as set forth in any one of SEQ ID NOs: 244-265 or an amino acid sequence of GS, and the L2 further has an amino acid sequence preferably as set forth in SEQ ID NO: 253, SEQ ID NO: 262 or SEQ ID NO: 263 (for example, as shown in structure 3 in FIG. 1).

In a certain preferred embodiment, the binding protein comprises a polypeptide chain 1 and a polypeptide chain 2, wherein the polypeptide chain 1 is as shown in a formula of N'-VL_A-CL-C', and the polypeptide chain 2 is as shown in a formula of N'-VH_A-CH1-L1-VH_B-L2-CH2-CH3-C', wherein the L1 and L2 are linker peptides being preferably 0 in length or having an amino acid sequence preferably as set forth in any one of SEQ ID NOs: 244-265 or an amino acid sequence of GS, and the L2 further has an amino acid sequence preferably as set forth in SEQ ID NO: 253, SEQ ID NO: 262 or SEQ ID NO: 263 (for example, as shown in structure 4 in FIG. 1).

In a certain preferred embodiment, the binding protein comprises a polypeptide chain 1 and a polypeptide chain 2, wherein the polypeptide chain 1 is as shown in a formula of N'-VH_A-CH1-C', and the polypeptide chain 2 is as shown in a formula of N'-VL_A-CL-L1-VH_B-L2-CH2-CH3-L3-VH_B-C', wherein the L1, L2 and L3 are linker peptides being preferably 0 in length or having an amino acid sequence preferably as set forth in any one of SEQ ID NOs: 244-265 or an amino acid sequence of GS, and the L2 further has an amino acid sequence preferably as set forth in SEQ ID NO: 253, SEQ ID NO: 262 or SEQ ID NO: 263 (for example, as shown in structure 5 in FIG. 1).

In a certain preferred embodiment, the binding protein comprises a polypeptide chain 1 and a polypeptide chain 2, wherein the polypeptide chain 1 is as shown in a formula of N'-VL_A-CL-C', and the polypeptide chain 2 is as shown in a formula of N'-VH_A-CH1-L1-VH_B-L2-CH2-CH3-L3-VH_B-C', wherein the L1, L2 and L3 are linker peptides being preferably 0 in length or having an amino acid sequence preferably as set forth in any one of SEQ ID NOs: 244-265 or an amino acid sequence of GS, and the L2 further has an amino acid sequence preferably as set forth in SEQ ID NO: 253, SEQ ID NO: 262 or SEQ ID NO: 263 (for example, as shown in structure 6 in FIG. 1).

In a certain preferred embodiment, the binding protein comprises a polypeptide chain 1, a polypeptide chain 2 and a polypeptide chain 3, wherein the polypeptide chain 1 is as shown in a formula of N'-VL_A-CL-L-VH_B-CH1-h-CH2-CH3-C', the polypeptide chain 2 is as shown in a formula of N'-VH_A-CH1-C', the polypeptide chain 3 is as shown in a formula of N'-VL_B-CL-C', wherein the L is a linker peptide being preferably 0 in length or having an amino acid sequence preferably as set forth in any one of SEQ ID NOs: 244-265 or an amino acid sequence of GS (for example, as shown in structure 8 in FIG. 1).

The VL_B and/or VH_B are VL and/or VH of the second protein functional region, respectively, and the VL_A and/or VH_A are VL and/or VH of the first protein functional region, respectively; the h is a hinge region or a sequence derived therefrom (e.g., a human IgG1 hinge, a human IgG1 hinge (C220S), a human IgG2 hinge, a human IgG4 hinge, a human IgG4 hinge (S228P) in Table 2); the CL is a light chain constant region domain; the CH1, CH2 and CH3 are a first domain, a second domain and a third domain of a heavy chain constant region, respectively; and the L or L1 or L2 or L3 is a linker peptide.

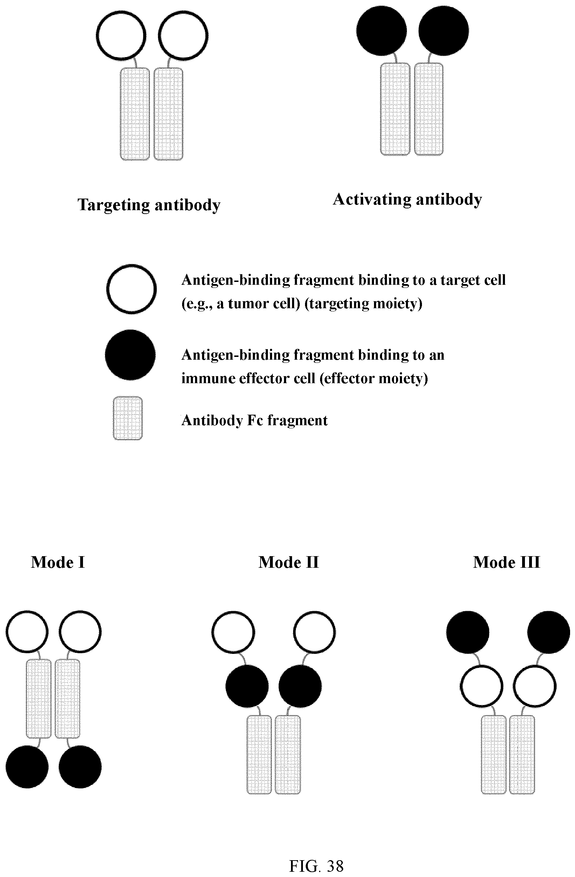

During the experiments, the inventors found that the position and distance of the antigen-binding fragment against the immune effector cell (effector moiety) relative to the antigen-binding fragment against the target cell (e.g., a tumor cell) (targeting moiety) are critical to the functional activity of the binding protein, as shown in the structure in FIG. 38.

In certain embodiments, when the effector moiety is positioned between the targeting moiety and the Fc (mode II in FIG. 38), particularly as a Fab-HCAb tetravalent symmetric structure (i.e., the structure 3 or structure 4 described in FIG. 1) or an FIT-Ig structure (i.e., the structure 8 described in FIG. 1), the binding protein has the ability to activate immune effector cells.

In certain embodiments, when the effector moiety is positioned at the C'-terminus of the Fc (mode I in FIG. 38), particularly as an IgG-VH tetravalent symmetric structure (i.e., the structure 1 described in FIG. 1) or an IgG-VH(2) hexavalent symmetric structure (i.e., the structure 2 described in FIG. 1), the binding protein has the ability to activate immune effector cells. However, when the effector moiety is at the N-terminus of the targeting moiety (mode III in FIG. 38), particularly as a VH-IgG tetravalent symmetric structure (i.e., the structure 7 described in FIG. 1), the binding protein does not have the ability to activate immune effector cells although it can bind to them.

In certain embodiments, the effector moiety may be positioned both between the targeting moiety and the Fc and at the C'-terminus of the Fc to achieve multivalent binding (which may be considered as a superposition of mode I and mode II in FIG. 38, as shown in FIG. 37 (C-D)), particularly as a Fab-VH-Fc-VH hexavalent symmetric structure (i.e., the structure 5 or structure 6 described in FIG. 1), and the binding protein has the ability to activate immune effector cells.

In the present invention, the antigen-binding fragment refers to any protein functional region that can specifically bind to an antigen, either a Fab region of a conventional antibody (H2L2) or a VH region of a heavy-chain antibody (HCAb), or other antigen-binding forms (e.g., derived protein structures such as lipocalins, neural cell adhesion molecules (NCAMs), fibronectins, and designed ankyrin repeat proteins (DARPins)).

Preferably, the (specific) antigen on the target cell is a tumor antigen and/or a co-inhibitory molecule antigen.

The tumor antigen may be conventional in the art, for example, may be CD19, BCMA, TSHR, CD171, CS-1, CLL-1, GD3, Tn Ag, FLT3, CD38, CD123, CD44v6, B7H3, B7H4, KIT, IL-13Ra2, IL-11Ra, PSCA, PSMA, PRSS21, VEGFR2, LewisY, CD24, PDGFR-beta, SSEA-4, MUC1, EGFR, NCAM, CAIX, LMP2, EphA2, fucosyl GM1, sLe, GM3, TGS5, HMWMAA, GD2, FOLR1, FOLR2, TEM1/CD248, TEM7R, CLDN6, CLDN18.2, GPRC5D, CXORF61, CD97, CD179a, ALK, polysialic acid, PLAC1, GloboH, NY-BR-1, UPK2, HAVCR1, ADRB3, PANX3, GPR20, LY6K, OR51E2, TAARP, WT1, ETV6-AML, SPA17, XAGE1, Tie 2, MAD-CT-1, MAD-CT-2, FOSL1, hTERT, ML-IAP, ERG (TMPRSS2 ETS fusion gene), NA17, PAX3, AR (androgen receptor), cyclin B1, MYCN, RhoC, CYP1B1, BORIS, SART3, PAX5, OY-TES1, LCK, AKAP-4, SSX2, CD79a, CD79b, CD72, LAIR1, FCAR, LILRA2, CD300LF, CLEC12A, BST2, EMR2, LY75, GPC3, FCRL5, IGLL1, CD20, CD30, HER2, ROR1, FLT3, TAAG72, CD22, CD33, GD2, gp100Tn, FAP, tyrosinase, EPCAM, CEA, IGF-1R, EphB2, mesothelin, cadherin17, CD32b, EGFRvIII, GPNMB, GPR64, HER3, LRP6, LYPD8, NKG2D, SLC34A2, SLC39A6, SLITRK6, GUCY2C and/or TACSTD2, etc.; preferably B7H3, B7H4, PSMA, CLDN18.2, GPC3, HER2, ROR1, CD22, EPCAM, mesothelin, EGFRvIII and/or TACSTD2.

The co-inhibitory molecule antigen may be conventional in the art, for example, may be PD-L1, PD-L2, CTLA4, B7H3, B7H4, VISTA, HHLA2 and/or CD155, preferably PD-L1, B7H3, B7H4 and/or HHLA2.

The (specific) antigen on the immune effector cell may be a stimulatory antigen or a costimulatory molecule antigen, for example, may be TCR/CD3, CD28, 4-1BB, OX40, GITR, CD27, ICOS, CD2, CD40, CD226, CD16, NKp30, NKp44, NKp46, NKG2D and/or 2B4, etc., preferably TCR/CD3, CD28, 4-1BB, OX40, GITR, CD40, CD226, CD16 and/or NKp30.

In certain embodiments, the (specific) antigen on the target cell may be CLDN18.2, HER2, B7H4, EPCAM, PSMA and/or PD-L1.

In certain embodiments, the (specific) antigen on the immune effector cell may be 4-1BB and/or OX40.

Preferably, the first protein functional region is an antibody targeting CLDN18.2 or an antigen-binding fragment thereof, an antibody targeting HER2 or an antigen-binding fragment thereof, an antibody targeting B7H4 or an antigen-binding fragment thereof, an antibody targeting EPCAM or an antigen-binding fragment thereof, an antibody targeting PSMA or an antigen-binding fragment thereof, and/or an antibody targeting PD-L1 or an antigen-binding fragment thereof.

Preferably, the second protein functional region is an antibody targeting OX40 or an antigen-binding fragment thereof, and/or an antibody targeting 4-1BB or an antigen-binding fragment thereof.

In certain embodiments, the antibody targeting CLDN18.2 or the antigen-binding fragment thereof comprises a heavy chain variable region (VH) and a light chain variable region (VL), wherein the VH comprises VH CDR1 with a sequence as set forth in SEQ ID NO: 22, VH CDR2 with a sequence as set forth in SEQ ID NO: 55 and VH CDR3 with a sequence as set forth in SEQ ID NO: 88, and the VL comprises VL CDR1 with a sequence as set forth in SEQ ID NO: 108, VL CDR2 with a sequence as set forth in SEQ ID NO: 121 and VL CDR3 with a sequence as set forth in SEQ ID NO: 142.

In certain embodiments, the antibody targeting HER2 or the antigen-binding fragment thereof comprises a heavy chain variable region (VH) and a light chain variable region (VL), wherein the VH comprises VH CDR1 with a sequence as set forth in SEQ ID NO: 13, VH CDR2 with a sequence as set forth in SEQ ID NO: 42 and VH CDR3 with a sequence as set forth in SEQ ID NO: 77, and the VL comprises VL CDR1 with a sequence as set forth in SEQ ID NO: 105, VL CDR2 with a sequence as set forth in SEQ ID NO: 117 and VL CDR3 with a sequence as set forth in SEQ ID NO: 134.

In certain embodiments, the antibody targeting PD-L1 or the antigen-binding fragment thereof comprises a heavy chain variable region (VH) and a light chain variable region (VL), wherein the VH comprises VH CDR1 with a sequence as set forth in SEQ ID NO: 11, VH CDR2 with a sequence as set forth in SEQ ID NO: 40 and VH CDR3 with a sequence as set forth in SEQ ID NO: 75, and the VL comprises VL CDR1 with a sequence as set forth in SEQ ID NO: 103, VL CDR2 with a sequence as set forth in SEQ ID NO: 117 and VL CDR3 with a sequence as set forth in SEQ ID NO: 132.

In certain embodiments, the antibody targeting PD-L1 or the antigen-binding fragment thereof comprises a heavy chain variable region (VH) and a light chain variable region (VL), wherein the VH comprises VH CDR1 with a sequence as set forth in SEQ ID NO: 14, VH CDR2 with a sequence as set forth in SEQ ID NO: 43 and VH CDR3 with a sequence as set forth in SEQ ID NO: 78, and the VL comprises VL CDR1 with a sequence as set forth in SEQ ID NO: 106, VL CDR2 with a sequence as set forth in SEQ ID NO: 119 and VL CDR3 with a sequence as set forth in SEQ ID NO: 135.

In certain embodiments, the antibody targeting B7H4 or the antigen-binding fragment thereof comprises a heavy chain variable region (VH) and a light chain variable region (VL), wherein the VH comprises VH CDR1 with a sequence as set forth in SEQ ID NO: 20, VH CDR2 with a sequence as set forth in SEQ ID NO: 53 and VH CDR3 with a sequence as set forth in SEQ ID NO: 86, and the VL comprises VL CDR1 with a sequence as set forth in SEQ ID NO: 110, VL CDR2 with a sequence as set forth in SEQ ID NO: 118 and VL CDR3 with a sequence as set forth in SEQ ID NO: 140.

In certain embodiments, the antibody targeting B7H4 or the antigen-binding fragment thereof comprises a heavy chain variable region (VH) and a light chain variable region (VL), wherein the VH comprises VH CDR1 with a sequence as set forth in SEQ ID NO: 21, VH CDR2 with a sequence as set forth in SEQ ID NO: 54 and VH CDR3 with a sequence as set forth in SEQ ID NO: 87, and the VL comprises VL CDR1 with a sequence as set forth in SEQ ID NO: 111, VL CDR2 with a sequence as set forth in SEQ ID NO: 118 and VL CDR3 with a sequence as set forth in SEQ ID NO: 141.

In certain embodiments, the antibody targeting EPCAM or the antigen-binding fragment thereof comprises a heavy chain variable region (VH) and a light chain variable region (VL), wherein the VH comprises VH CDR1 with a sequence as set forth in SEQ ID NO: 16, VH CDR2 with a sequence as set forth in SEQ ID NO: 47 and VH CDR3 with a sequence as set forth in SEQ ID NO: 81, and the VL comprises VL CDR1 with a sequence as set forth in SEQ ID NO: 108, VL CDR2 with a sequence as set forth in SEQ ID NO: 121 and VL CDR3 with a sequence as set forth in SEQ ID NO: 138.

In certain embodiments, the antibody targeting PSMA or the antigen-binding fragment thereof comprises a heavy chain variable region (VH) and a light chain variable region (VL), wherein the VH comprises VH CDR1 with a sequence as set forth in SEQ ID NO: 17, VH CDR2 with a sequence as set forth in SEQ ID NO: 48 and VH CDR3 with a sequence as set forth in SEQ ID NO: 82, and the VL comprises VL CDR1 with a sequence as set forth in SEQ ID NO: 109, VL CDR2 with a sequence as set forth in SEQ ID NO: 122 and VL CDR3 with a sequence as set forth in SEQ ID NO: 139.

In certain embodiments, the antibody targeting 4-1BB or the antigen-binding fragment thereof comprises a heavy chain variable region (VH), wherein the VH comprises VH CDR1 with a sequence as set forth in SEQ ID NO: 16, VH CDR2 with a sequence as set forth in SEQ ID NO: 49 and VH CDR3 with a sequence as set forth in SEQ ID NO: 83.

In certain embodiments, the antibody targeting 4-1BB or the antigen-binding fragment thereof comprises a heavy chain variable region (VH), wherein the VH comprises VH CDR1 with a sequence as set forth in SEQ ID NO: 18, VH CDR2 with a sequence as set forth in SEQ ID NO: 50 and VH CDR3 with a sequence as set forth in SEQ ID NO: 84.

In certain embodiments, the antibody targeting 4-1BB or the antigen-binding fragment thereof comprises a heavy chain variable region (VH), wherein the VH comprises VH CDR1 with a sequence as set forth in SEQ ID NO: 19, VH CDR2 with a sequence as set forth in SEQ ID NO: 51 and VH CDR3 with a sequence as set forth in SEQ ID NO: 84.

In certain embodiments, the antibody targeting 4-1BB or the antigen-binding fragment thereof comprises a heavy chain variable region (VH), wherein the VH comprises VH CDR1 with a sequence as set forth in SEQ ID NO: 18, VH CDR2 with a sequence as set forth in SEQ ID NO: 57 and VH CDR3 with a sequence as set forth in SEQ ID NO: 90.

In certain embodiments, the antibody targeting 4-1BB or the antigen-binding fragment thereof comprises a heavy chain variable region (VH) and a light chain variable region (VL), wherein the VH comprises VH CDR1 with a sequence as set forth in SEQ ID NO: 12, VH CDR2 with a sequence as set forth in SEQ ID NO: 41 and VH CDR3 with a sequence as set forth in SEQ ID NO: 76, and the VL comprises VL CDR1 with a sequence as set forth in SEQ ID NO: 104, VL CDR2 with a sequence as set forth in SEQ ID NO: 118 and VL CDR3 with a sequence as set forth in SEQ ID NO: 133.

In certain embodiments, the antibody targeting 4-1BB or the antigen-binding fragment thereof comprises a heavy chain variable region (VH) and a light chain variable region (VL), wherein the VH comprises VH CDR1 with a sequence as set forth in SEQ ID NO: 12, VH CDR2 with a sequence as set forth in SEQ ID NO: 44 and VH CDR3 with a sequence as set forth in SEQ ID NO: 76, and the VL comprises VL CDR1 with a sequence as set forth in SEQ ID NO: 104, VL CDR2 with a sequence as set forth in SEQ ID NO: 118 and VL CDR3 with a sequence as set forth in SEQ ID NO: 133.

In certain embodiments, the antibody targeting OX40 or the antigen-binding fragment thereof comprises a heavy chain variable region (VH), wherein the VH comprises VH CDR1 with a sequence as set forth in SEQ ID NO: 14, VH CDR2 with a sequence as set forth in SEQ ID NO: 52 and VH CDR3 with a sequence as set forth in SEQ ID NO: 85.

In certain embodiments, the antibody targeting CLDN18.2 or the antigen-binding fragment thereof comprises VH with a sequence as set forth in SEQ ID NO: 165 and VL with a sequence as set forth in SEQ ID NO: 179.

In certain embodiments, the antibody targeting HER2 or the antigen-binding fragment thereof comprises VH with a sequence as set forth in SEQ ID NO: 152 and VL with a sequence as set forth in SEQ ID NO: 170.

In certain embodiments, the antibody targeting PD-L1 or the antigen-binding fragment thereof comprises VH with a sequence as set forth in SEQ ID NO: 150 and VL with a sequence as set forth in SEQ ID NO: 168.

In certain embodiments, the antibody targeting PD-L1 or the antigen-binding fragment thereof comprises VH with a sequence as set forth in SEQ ID NO: 153 and VL with a sequence as set forth in SEQ ID NO: 171.

In certain embodiments, the antibody targeting B7H4 or the antigen-binding fragment thereof comprises VH with a sequence as set forth in SEQ ID NO: 163 and VL with a sequence as set forth in SEQ ID NO: 177.

In certain embodiments, the antibody targeting B7H4 or the antigen-binding fragment thereof comprises VH with a sequence as set forth in SEQ ID NO: 164 and VL with a sequence as set forth in SEQ ID NO: 178.

In certain embodiments, the antibody targeting EPCAM or the antigen-binding fragment thereof comprises VH with a sequence as set forth in SEQ ID NO: 157 and VL with a sequence as set forth in SEQ ID NO: 175.

In certain embodiments, the antibody targeting PSMA or the antigen-binding fragment thereof comprises VH with a sequence as set forth in SEQ ID NO: 158 and VL with a sequence as set forth in SEQ ID NO: 176.

In certain embodiments, the antibody targeting 4-1BB or the antigen-binding fragment thereof comprises VH with a sequence as set forth in SEQ ID NO: 159.

In certain embodiments, the antibody targeting 4-1BB or the antigen-binding fragment thereof comprises VH with a sequence as set forth in SEQ ID NO: 160.

In certain embodiments, the antibody targeting 4-1BB or the antigen-binding fragment thereof comprises VH with a sequence as set forth in SEQ ID NO: 161.

In certain embodiments, the antibody targeting 4-1BB or the antigen-binding fragment thereof comprises VH with a sequence as set forth in SEQ ID NO: 167.

In certain embodiments, the antibody targeting 4-1BB or the antigen-binding fragment thereof comprises VH with a sequence as set forth in SEQ ID NO: 151 and VL with a sequence as set forth in SEQ ID NO: 169.

In certain embodiments, the antibody targeting 4-1BB or the antigen-binding fragment thereof comprises VH with a sequence as set forth in SEQ ID NO: 154 and VL with a sequence as set forth in SEQ ID NO: 172.

In certain embodiments, the antibody targeting OX40 or the antigen-binding fragment thereof comprises VH with a sequence as set forth in SEQ ID NO: 162.

In certain embodiments, the antibody targeting CLDN18.2 or the antigen-binding fragment thereof comprises a heavy chain with a sequence as set forth in SEQ ID NO: 196 and a light chain with a sequence as set forth in SEQ ID NO: 210.

In certain embodiments, the antibody targeting HER2 or the antigen-binding fragment thereof comprises a heavy chain with a sequence as set forth in SEQ ID NO: 183 and a light chain with a sequence as set forth in SEQ ID NO: 201.

In certain embodiments, the antibody targeting PD-L1 or the antigen-binding fragment thereof comprises a heavy chain with a sequence as set forth in SEQ ID NO: 181 and a light chain with a sequence as set forth in SEQ ID NO: 199.

In certain embodiments, the antibody targeting PD-L1 or the antigen-binding fragment thereof comprises a heavy chain with a sequence as set forth in SEQ ID NO: 184 and a light chain with a sequence as set forth in SEQ ID NO: 202.

In certain embodiments, the antibody targeting B7H4 or the antigen-binding fragment thereof comprises a heavy chain with a sequence as set forth in SEQ ID NO: 194 and a light chain with a sequence as set forth in SEQ ID NO: 208.

In certain embodiments, the antibody targeting B7H4 or the antigen-binding fragment thereof comprises a heavy chain with a sequence as set forth in SEQ ID NO: 195 and a light chain with a sequence as set forth in SEQ ID NO: 209.

In certain embodiments, the antibody targeting EPCAM or the antigen-binding fragment thereof comprises a heavy chain with a sequence as set forth in SEQ ID NO: 188 and a light chain with a sequence as set forth in SEQ ID NO: 206.

In certain embodiments, the antibody targeting PSMA or the antigen-binding fragment thereof comprises a heavy chain with a sequence as set forth in SEQ ID NO: 189 and a light chain with a sequence as set forth in SEQ ID NO: 207.

In certain embodiments, the antibody targeting 4-1BB or the antigen-binding fragment thereof comprises a heavy chain with a sequence as set forth in SEQ ID NO: 190.

In certain embodiments, the antibody targeting 4-1BB or the antigen-binding fragment thereof comprises a heavy chain with a sequence as set forth in SEQ ID NO: 191.

In certain embodiments, the antibody targeting 4-1BB or the antigen-binding fragment thereof comprises a heavy chain with a sequence as set forth in SEQ ID NO: 192.

In certain embodiments, the antibody targeting 4-1BB or the antigen-binding fragment thereof comprises a heavy chain with a sequence as set forth in SEQ ID NO: 198.

In certain embodiments, the antibody targeting 4-1BB or the antigen-binding fragment thereof comprises a heavy chain with a sequence as set forth in SEQ ID NO: 182 and a light chain with a sequence as set forth in SEQ ID NO: 200.

In certain embodiments, the antibody targeting 4-1BB or the antigen-binding fragment thereof comprises a heavy chain with a sequence as set forth in SEQ ID NO: 185 and a light chain with a sequence as set forth in SEQ ID NO: 203.

In certain embodiments, the antibody targeting OX40 or the antigen-binding fragment thereof comprises a heavy chain with a sequence as set forth in SEQ ID NO: 193.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 105, 117 and 134, respectively; and the heavy chain variable region VH comprises VH CDR1, VH CDR2 and VH CDR3 with amino acid sequences as set forth in SEQ ID NOs: 13, 42 and 77, respectively. The second protein functional region comprises a heavy chain variable region, wherein the heavy chain variable region VH comprises VH CDR1, VH CDR2 and VH CDR3 with amino acid sequences as set forth in SEQ ID NOs: 19, 51 and 84, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 110, 118 and 140, respectively; and the heavy chain variable region VH comprises VH CDR1, VH CDR2 and VH CDR3 with amino acid sequences as set forth in SEQ ID NOs: 20, 53 and 86, respectively. The second protein functional region comprises a heavy chain variable region, wherein the heavy chain variable region VH comprises VH CDR1, VH CDR2 and VH CDR3 with amino acid sequences as set forth in SEQ ID NOs: 16, 49 and 83, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 110, 118 and 140, respectively; and the heavy chain variable region VH comprises VH CDR1, VH CDR2 and VH CDR3 with amino acid sequences as set forth in SEQ ID NOs: 20, 53 and 86, respectively. The second protein functional region comprises a heavy chain variable region, wherein the heavy chain variable region VH comprises VH CDR1, VH CDR2 and VH CDR3 with amino acid sequences as set forth in SEQ ID NOs: 18, 50 and 84, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 110, 118 and 140, respectively; and the heavy chain variable region VH comprises VH CDR1, VH CDR2 and VH CDR3 with amino acid sequences as set forth in SEQ ID NOs: 20, 53 and 86, respectively. The second protein functional region comprises a heavy chain variable region, wherein the heavy chain variable region VH comprises VH CDR1, VH CDR2 and VH CDR3 with amino acid sequences as set forth in SEQ ID NOs: 18, 57 and 90, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 106, 119 and 135, respectively; and the heavy chain variable region VH comprises VH CDR1, VH CDR2 and VH CDR3 with amino acid sequences as set forth in SEQ ID NOs: 14, 43 and 78, respectively. The second protein functional region comprises a heavy chain variable region, wherein the heavy chain variable region VH comprises VH CDR1, VH CDR2 and VH CDR3 with amino acid sequences as set forth in SEQ ID NOs: 16, 49 and 83, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 106, 119 and 135, respectively; and the heavy chain variable region VH comprises VH CDR1, VH CDR2 and VH CDR3 with amino acid sequences as set forth in SEQ ID NOs: 14, 43 and 78, respectively. The second protein functional region comprises a heavy chain variable region, wherein the heavy chain variable region VH comprises VH CDR1, VH CDR2 and VH CDR3 with amino acid sequences as set forth in SEQ ID NOs: 18, 50 and 84, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 106, 119 and 135, respectively; and the heavy chain variable region VH comprises VH CDR1, VH CDR2 and VH CDR3 with amino acid sequences as set forth in SEQ ID NOs: 14, 43 and 78, respectively. The second protein functional region comprises a heavy chain variable region, wherein the heavy chain variable region VH comprises VH CDR1, VH CDR2 and VH CDR3 with amino acid sequences as set forth in SEQ ID NOs: 19, 51 and 84, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 108, 121 and 138, respectively; and the heavy chain variable region VH comprises VH CDR1, VH CDR2 and VH CDR3 with amino acid sequences as set forth in SEQ ID NOs: 16, 47 and 81, respectively. The second protein functional region comprises a heavy chain variable region, wherein the heavy chain variable region VH comprises VH CDR1, VH CDR2 and VH CDR3 with amino acid sequences as set forth in SEQ ID NOs: 16, 49 and 83, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 108, 121 and 138, respectively; and the heavy chain variable region VH comprises VH CDR1, VH CDR2 and VH CDR3 with amino acid sequences as set forth in SEQ ID NOs: 16, 47 and 81, respectively. The second protein functional region comprises a heavy chain variable region, wherein the heavy chain variable region VH comprises VH CDR1, VH CDR2 and VH CDR3 with amino acid sequences as set forth in SEQ ID NOs: 18, 50 and 84, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 108, 121 and 138, respectively; and the heavy chain variable region VH comprises VH CDR1, VH CDR2 and VH CDR3 with amino acid sequences as set forth in SEQ ID NOs: 16, 47 and 81, respectively. The second protein functional region comprises a heavy chain variable region, wherein the heavy chain variable region VH comprises VH CDR1, VH CDR2 and VH CDR3 with amino acid sequences as set forth in SEQ ID NOs: 19, 51 and 84, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 109, 122 and 139, respectively; and the heavy chain variable region VH comprises VH CDR1, VH CDR2 and VH CDR3 with amino acid sequences as set forth in SEQ ID NOs: 17, 48 and 82, respectively. The second protein functional region comprises a heavy chain variable region, wherein the heavy chain variable region VH comprises VH CDR1, VH CDR2 and VH CDR3 with amino acid sequences as set forth in SEQ ID NOs: 16, 49 and 83, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 109, 122 and 139, respectively; and the heavy chain variable region VH comprises VH CDR1, VH CDR2 and VH CDR3 with amino acid sequences as set forth in SEQ ID NOs: 17, 48 and 82, respectively. The second protein functional region comprises a heavy chain variable region, wherein the heavy chain variable region VH comprises VH CDR1, VH CDR2 and VH CDR3 with amino acid sequences as set forth in SEQ ID NOs: 18, 50 and 84, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 109, 122 and 139, respectively; and the heavy chain variable region VH comprises VH CDR1, VH CDR2 and VH CDR3 with amino acid sequences as set forth in SEQ ID NOs: 17, 48 and 82, respectively. The second protein functional region comprises a heavy chain variable region, wherein the heavy chain variable region VH comprises VH CDR1, VH CDR2 and VH CDR3 with amino acid sequences as set forth in SEQ ID NOs: 19, 51 and 84, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 108, 121 and 142, respectively; and the heavy chain variable region VH comprises VH CDR1, VH CDR2 and VH CDR3 with amino acid sequences as set forth in SEQ ID NOs: 22, 55 and 88, respectively. The second protein functional region comprises a heavy chain variable region, wherein the heavy chain variable region VH comprises VH CDR1, VH CDR2 and VH CDR3 with amino acid sequences as set forth in SEQ ID NOs: 16, 49 and 83, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 108, 121 and 142, respectively; and the heavy chain variable region VH comprises VH CDR1, VH CDR2 and VH CDR3 with amino acid sequences as set forth in SEQ ID NOs: 22, 55 and 88, respectively. The second protein functional region comprises a heavy chain variable region, wherein the heavy chain variable region VH comprises VH CDR1, VH CDR2 and VH CDR3 with amino acid sequences as set forth in SEQ ID NOs: 18, 50 and 84, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 108, 121 and 142, respectively; and the heavy chain variable region VH comprises VH CDR1, VH CDR2 and VH CDR3 with amino acid sequences as set forth in SEQ ID NOs: 22, 55 and 88, respectively. The second protein functional region comprises a heavy chain variable region, wherein the heavy chain variable region VH comprises VH CDR1, VH CDR2 and VH CDR3 with amino acid sequences as set forth in SEQ ID NOs: 19, 51 and 84, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 106, 119 and 135, respectively; and the heavy chain variable region VH comprises VH CDR1, VH CDR2 and VH CDR3 with amino acid sequences as set forth in SEQ ID NOs: 14, 43 and 78, respectively. The second protein functional region comprises a heavy chain variable region, wherein the heavy chain variable region VH comprises VH CDR1, VH CDR2 and VH CDR3 with amino acid sequences as set forth in SEQ ID NOs: 14, 52 and 85, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 110, 118 and 140, respectively; and the heavy chain variable region VH comprises VH CDR1, VH CDR2 and VH CDR3 with amino acid sequences as set forth in SEQ ID NOs: 20, 53 and 86, respectively. The second protein functional region comprises a heavy chain variable region, wherein the heavy chain variable region VH comprises VH CDR1, VH CDR2 and VH CDR3 with amino acid sequences as set forth in SEQ ID NOs: 14, 52 and 85, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 108, 121 and 138, respectively; and the heavy chain variable region VH comprises VH CDR1, VH CDR2 and VH CDR3 with amino acid sequences as set forth in SEQ ID NOs: 16, 47 and 81, respectively. The second protein functional region comprises a heavy chain variable region, wherein the heavy chain variable region VH comprises VH CDR1, VH CDR2 and VH CDR3 with amino acid sequences as set forth in SEQ ID NOs: 14, 52 and 85, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 109, 122 and 139, respectively; and the heavy chain variable region VH comprises VH CDR1, VH CDR2 and VH CDR3 with amino acid sequences as set forth in SEQ ID NOs: 17, 48 and 82, respectively. The second protein functional region comprises a heavy chain variable region, wherein the heavy chain variable region VH comprises VH CDR1, VH CDR2 and VH CDR3 with amino acid sequences as set forth in SEQ ID NOs: 14, 52 and 85, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 108, 121 and 142, respectively; and the heavy chain variable region VH comprises VH CDR1, VH CDR2 and VH CDR3 with amino acid sequences as set forth in SEQ ID NOs: 22, 55 and 88, respectively. The second protein functional region comprises a heavy chain variable region, wherein the heavy chain variable region VH comprises VH CDR1, VH CDR2 and VH CDR3 with amino acid sequences as set forth in SEQ ID NOs: 14, 52 and 85, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 111, 118 and 141, respectively; and the heavy chain variable region VH comprises VH CDR1, VH CDR2 and VH CDR3 with amino acid sequences as set forth in SEQ ID NOs: 21, 54 and 87, respectively. The second protein functional region comprises a heavy chain variable region, wherein the heavy chain variable region VH comprises VH CDR1, VH CDR2 and VH CDR3 with amino acid sequences as set forth in SEQ ID NOs: 18, 50 and 84, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 106, 119 and 135, respectively; and the heavy chain variable region VH comprises VH CDR1, VH CDR2 and VH CDR3 with amino acid sequences as set forth in SEQ ID NOs: 14, 43 and 78, respectively. The second protein functional region comprises a light chain variable region and a heavy chain variable region, wherein the light chain variable region VL comprises VL CDR1, VL CDR2 and VL CDR3 with amino acid sequences as set forth in SEQ ID NOs: 104, 118 and 133, respectively, and the heavy chain variable region VH comprises VH CDR1, VH CDR2 and VH CDR3 with amino acid sequences as set forth in SEQ ID NOs: 12, 41 and 76, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises VL CDR1, VL CDR2 and VL CDR3 with amino acid sequences as set forth in SEQ ID NOs: 103, 117 and 132, respectively; and the heavy chain variable region VH comprises VH CDR1, VH CDR2 and VH CDR3 with amino acid sequences as set forth in SEQ ID NOs: 11, 40 and 75, respectively. The second protein functional region comprises a light chain variable region and a heavy chain variable region, wherein the light chain variable region VL comprises VL CDR1, VL CDR2 and VL CDR3 with amino acid sequences as set forth in SEQ ID NOs: 104, 118 and 133, respectively, and the heavy chain variable region VH comprises VH CDR1, VH CDR2 and VH CDR3 with amino acid sequences as set forth in SEQ ID NOs: 12, 44 and 76, respectively. The amino acid sequences of the listed CDRs are shown according to the Chothia scheme.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 170, and the heavy chain variable region VH comprises an amino acid sequence as set forth in SEQ ID NO: 152. The second protein functional region comprises a heavy chain variable region, wherein the heavy chain variable region VH comprises an amino acid sequence as set forth in SEQ ID NO: 161.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 177, and the heavy chain variable region VH comprises an amino acid sequence as set forth in SEQ ID NO: 163. The second protein functional region comprises a heavy chain variable region, wherein the heavy chain variable region VH comprises an amino acid sequence as set forth in SEQ ID NO: 159.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 177, and the heavy chain variable region VH comprises an amino acid sequence as set forth in SEQ ID NO: 163. The second protein functional region comprises a heavy chain variable region, wherein the heavy chain variable region VH comprises an amino acid sequence as set forth in SEQ ID NO: 160.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 177, and the heavy chain variable region VH comprises an amino acid sequence as set forth in SEQ ID NO: 163. The second protein functional region comprises a heavy chain variable region, wherein the heavy chain variable region VH comprises an amino acid sequence as set forth in SEQ ID NO: 167.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 171, and the heavy chain variable region VH comprises an amino acid sequence as set forth in SEQ ID NO: 153. The second protein functional region comprises a heavy chain variable region, wherein the heavy chain variable region VH comprises an amino acid sequence as set forth in SEQ ID NO: 159.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 171, and the heavy chain variable region VH comprises an amino acid sequence as set forth in SEQ ID NO: 153. The second protein functional region comprises a heavy chain variable region, wherein the heavy chain variable region VH comprises an amino acid sequence as set forth in SEQ ID NO: 160.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 171, and the heavy chain variable region VH comprises an amino acid sequence as set forth in SEQ ID NO: 153. The second protein functional region comprises a heavy chain variable region, wherein the heavy chain variable region VH comprises an amino acid sequence as set forth in SEQ ID NO: 161.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 175, and the heavy chain variable region VH comprises an amino acid sequence as set forth in SEQ ID NO: 157. The second protein functional region comprises a heavy chain variable region, wherein the heavy chain variable region VH comprises an amino acid sequence as set forth in SEQ ID NO: 159.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 175, and the heavy chain variable region VH comprises an amino acid sequence as set forth in SEQ ID NO: 157. The second protein functional region comprises a heavy chain variable region, wherein the heavy chain variable region VH comprises an amino acid sequence as set forth in SEQ ID NO: 160.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 175, and the heavy chain variable region VH comprises an amino acid sequence as set forth in SEQ ID NO: 157. The second protein functional region comprises a heavy chain variable region, wherein the heavy chain variable region VH comprises an amino acid sequence as set forth in SEQ ID NO: 161.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 176, and the heavy chain variable region VH comprises an amino acid sequence as set forth in SEQ ID NO: 158. The second protein functional region comprises a heavy chain variable region, wherein the heavy chain variable region VH comprises an amino acid sequence as set forth in SEQ ID NO: 159.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 176, and the heavy chain variable region VH comprises an amino acid sequence as set forth in SEQ ID NO: 158. The second protein functional region comprises a heavy chain variable region, wherein the heavy chain variable region VH comprises an amino acid sequence as set forth in SEQ ID NO: 160.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 176, and the heavy chain variable region VH comprises an amino acid sequence as set forth in SEQ ID NO: 158. The second protein functional region comprises a heavy chain variable region, wherein the heavy chain variable region VH comprises an amino acid sequence as set forth in SEQ ID NO: 161.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 179, and the heavy chain variable region VH comprises an amino acid sequence as set forth in SEQ ID NO: 165. The second protein functional region comprises a heavy chain variable region, wherein the heavy chain variable region VH comprises an amino acid sequence as set forth in SEQ ID NO: 159.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 179, and the heavy chain variable region VH comprises an amino acid sequence as set forth in SEQ ID NO: 165. The second protein functional region comprises a heavy chain variable region, wherein the heavy chain variable region VH comprises an amino acid sequence as set forth in SEQ ID NO: 160.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 179, and the heavy chain variable region VH comprises an amino acid sequence as set forth in SEQ ID NO: 165. The second protein functional region comprises a heavy chain variable region, wherein the heavy chain variable region VH comprises an amino acid sequence as set forth in SEQ ID NO: 161.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 171, and the heavy chain variable region VH comprises an amino acid sequence as set forth in SEQ ID NO: 153. The second protein functional region comprises a heavy chain variable region, wherein the heavy chain variable region VH comprises an amino acid sequence as set forth in SEQ ID NO: 162.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 177, and the heavy chain variable region VH comprises an amino acid sequence as set forth in SEQ ID NO: 163. The second protein functional region comprises a heavy chain variable region, wherein the heavy chain variable region VH comprises an amino acid sequence as set forth in SEQ ID NO: 162.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 175, and the heavy chain variable region VH comprises an amino acid sequence as set forth in SEQ ID NO: 157. The second protein functional region comprises a heavy chain variable region, wherein the heavy chain variable region VH comprises an amino acid sequence as set forth in SEQ ID NO: 162.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 176, and the heavy chain variable region VH comprises an amino acid sequence as set forth in SEQ ID NO: 158. The second protein functional region comprises a heavy chain variable region, wherein the heavy chain variable region VH comprises an amino acid sequence as set forth in SEQ ID NO: 162.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 179, and the heavy chain variable region VH comprises an amino acid sequence as set forth in SEQ ID NO: 165. The second protein functional region comprises a heavy chain variable region, wherein the heavy chain variable region VH comprises an amino acid sequence as set forth in SEQ ID NO: 162.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 178, and the heavy chain variable region VH comprises an amino acid sequence as set forth in SEQ ID NO: 164. The second protein functional region comprises a heavy chain variable region, wherein the heavy chain variable region VH comprises an amino acid sequence as set forth in SEQ ID NO: 160.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 171, and the heavy chain variable region VH comprises an amino acid sequence as set forth in SEQ ID NO: 153. The second protein functional region comprises a light chain variable region and a heavy chain variable region, wherein the light chain variable region VL comprises an amino acid sequence as set forth in SEQ ID NO: 169, and the heavy chain variable region VH comprises an amino acid sequence as set forth in SEQ ID NO: 151.

In certain embodiments, the binding protein comprises two protein functional regions: a first protein functional region and a second protein functional region. The first protein functional region comprises a light chain variable region and a heavy chain variable region, wherein the light chain variable region VL comprises an amino acid sequence as set forth in SEQ ID NO: 168, and the heavy chain variable region VH comprises an amino acid sequence as set forth in SEQ ID NO: 150. The second protein functional region comprises a light chain variable region and a heavy chain variable region, wherein the light chain variable region VL comprises an amino acid sequence as set forth in SEQ ID NO: 172, and the heavy chain variable region VH comprises an amino acid sequence as set forth in SEQ ID NO: 154.

In certain embodiments, the binding protein comprises two different polypeptide chains, wherein a polypeptide chain 1 comprises an amino acid sequence as set forth in SEQ ID NO: 201; and a polypeptide chain 2 comprises an amino acid sequence as set forth in SEQ ID NO: 214.

In certain embodiments, the binding protein comprises two different polypeptide chains, wherein a polypeptide chain 1 comprises an amino acid sequence as set forth in SEQ ID NO: 208; and a polypeptide chain 2 comprises an amino acid sequence as set forth in SEQ ID NO: 217.

In certain embodiments, the binding protein comprises two different polypeptide chains, wherein a polypeptide chain 1 comprises an amino acid sequence as set forth in SEQ ID NO: 208; and a polypeptide chain 2 comprises an amino acid sequence as set forth in SEQ ID NO: 218.

In certain embodiments, the binding protein comprises two different polypeptide chains, wherein a polypeptide chain 1 comprises an amino acid sequence as set forth in SEQ ID NO: 208; and a polypeptide chain 2 comprises an amino acid sequence as set forth in SEQ ID NO: 219.

In certain embodiments, the binding protein comprises two different polypeptide chains, wherein a polypeptide chain 1 comprises an amino acid sequence as set forth in SEQ ID NO: 202; and a polypeptide chain 2 comprises an amino acid sequence as set forth in SEQ ID NO: 221.

In certain embodiments, the binding protein comprises two different polypeptide chains, wherein a polypeptide chain 1 comprises an amino acid sequence as set forth in SEQ ID NO: 202; and a polypeptide chain 2 comprises an amino acid sequence as set forth in SEQ ID NO: 222.

In certain embodiments, the binding protein comprises two different polypeptide chains, wherein a polypeptide chain 1 comprises an amino acid sequence as set forth in SEQ ID NO: 202; and a polypeptide chain 2 comprises an amino acid sequence as set forth in SEQ ID NO: 223.

In certain embodiments, the binding protein comprises two different polypeptide chains, wherein a polypeptide chain 1 comprises an amino acid sequence as set forth in SEQ ID NO: 206; and a polypeptide chain 2 comprises an amino acid sequence as set forth in SEQ ID NO: 224.

In certain embodiments, the binding protein comprises two different polypeptide chains, wherein a polypeptide chain 1 comprises an amino acid sequence as set forth in SEQ ID NO: 206; and a polypeptide chain 2 comprises an amino acid sequence as set forth in SEQ ID NO: 225.

In certain embodiments, the binding protein comprises two different polypeptide chains, wherein a polypeptide chain 1 comprises an amino acid sequence as set forth in SEQ ID NO: 206; and a polypeptide chain 2 comprises an amino acid sequence as set forth in SEQ ID NO: 226.

In certain embodiments, the binding protein comprises two different polypeptide chains, wherein a polypeptide chain 1 comprises an amino acid sequence as set forth in SEQ ID NO: 207; and a polypeptide chain 2 comprises an amino acid sequence as set forth in SEQ ID NO: 227.

In certain embodiments, the binding protein comprises two different polypeptide chains, wherein a polypeptide chain 1 comprises an amino acid sequence as set forth in SEQ ID NO: 207; and a polypeptide chain 2 comprises an amino acid sequence as set forth in SEQ ID NO: 228.

In certain embodiments, the binding protein comprises two different polypeptide chains, wherein a polypeptide chain 1 comprises an amino acid sequence as set forth in SEQ ID NO: 207; and a polypeptide chain 2 comprises an amino acid sequence as set forth in SEQ ID NO: 229.

In certain embodiments, the binding protein comprises two different polypeptide chains, wherein a polypeptide chain 1 comprises an amino acid sequence as set forth in SEQ ID NO: 210; and a polypeptide chain 2 comprises an amino acid sequence as set forth in SEQ ID NO: 230.

In certain embodiments, the binding protein comprises two different polypeptide chains, wherein a polypeptide chain 1 comprises an amino acid sequence as set forth in SEQ ID NO: 210; and a polypeptide chain 2 comprises an amino acid sequence as set forth in SEQ ID NO: 231.

In certain embodiments, the binding protein comprises two different polypeptide chains, wherein a polypeptide chain 1 comprises an amino acid sequence as set forth in SEQ ID NO: 210; and a polypeptide chain 2 comprises an amino acid sequence as set forth in SEQ ID NO: 232.

In certain embodiments, the binding protein comprises two different polypeptide chains, wherein a polypeptide chain 1 comprises an amino acid sequence as set forth in SEQ ID NO: 202; and a polypeptide chain 2 comprises an amino acid sequence as set forth in SEQ ID NO: 233.

In certain embodiments, the binding protein comprises two different polypeptide chains, wherein a polypeptide chain 1 comprises an amino acid sequence as set forth in SEQ ID NO: 208; and a polypeptide chain 2 comprises an amino acid sequence as set forth in SEQ ID NO: 236.

In certain embodiments, the binding protein comprises two different polypeptide chains, wherein a polypeptide chain 1 comprises an amino acid sequence as set forth in SEQ ID NO: 206; and a polypeptide chain 2 comprises an amino acid sequence as set forth in SEQ ID NO: 241.

In certain embodiments, the binding protein comprises two different polypeptide chains, wherein a polypeptide chain 1 comprises an amino acid sequence as set forth in SEQ ID NO: 207; and a polypeptide chain 2 comprises an amino acid sequence as set forth in SEQ ID NO: 242.

In certain embodiments, the binding protein comprises two different polypeptide chains, wherein a polypeptide chain 1 comprises an amino acid sequence as set forth in SEQ ID NO: 210; and a polypeptide chain 2 comprises an amino acid sequence as set forth in SEQ ID NO: 243.

In certain embodiments, the binding protein comprises two different polypeptide chains, wherein a polypeptide chain 1 comprises an amino acid sequence as set forth in SEQ ID NO: 208; and a polypeptide chain 2 comprises an amino acid sequence as set forth in SEQ ID NO: 220.

In certain embodiments, the binding protein comprises two different polypeptide chains, wherein a polypeptide chain 1 comprises an amino acid sequence as set forth in SEQ ID NO: 209; and a polypeptide chain 2 comprises an amino acid sequence as set forth in SEQ ID NO: 234.

In certain embodiments, the binding protein comprises two different polypeptide chains, wherein a polypeptide chain 1 comprises an amino acid sequence as set forth in SEQ ID NO: 212; and a polypeptide chain 2 comprises an amino acid sequence as set forth in SEQ ID NO: 235.

In certain embodiments, the binding protein comprises two different polypeptide chains, wherein a polypeptide chain 1 comprises an amino acid sequence as set forth in SEQ ID NO: 237; and a polypeptide chain 2 comprises an amino acid sequence as set forth in SEQ ID NO: 240.

In certain embodiments, the binding protein comprises two different polypeptide chains, wherein a polypeptide chain 1 comprises an amino acid sequence as set forth in SEQ ID NO: 237; and a polypeptide chain 2 comprises an amino acid sequence as set forth in SEQ ID NO: 238.

In certain embodiments, the binding protein comprises three different polypeptide chains, wherein a polypeptide chain 1 comprises an amino acid sequence as set forth in SEQ ID NO: 213; a polypeptide chain 2 comprises an amino acid sequence as set forth in SEQ ID NO: 212; and a polypeptide chain 3 comprises an amino acid sequence as set forth in SEQ ID NO: 203.

In certain embodiments, the binding protein comprises three different polypeptide chains, wherein a polypeptide chain 1 comprises an amino acid sequence as set forth in SEQ ID NO: 216; a polypeptide chain 2 comprises an amino acid sequence as set forth in SEQ ID NO: 215; and a polypeptide chain 3 comprises an amino acid sequence as set forth in SEQ ID NO: 203.

In certain embodiments, the binding protein comprises two different polypeptide chains, wherein a polypeptide chain 1 comprises an amino acid sequence as set forth in SEQ ID NO: 237; and a polypeptide chain 2 comprises an amino acid sequence as set forth in SEQ ID NO: 266.

In certain embodiments, the binding protein comprises two different polypeptide chains, wherein a polypeptide chain 1 comprises an amino acid sequence as set forth in SEQ ID NO: 237; and a polypeptide chain 2 comprises an amino acid sequence as set forth in SEQ ID NO: 267.

In certain embodiments, the binding protein comprises two different polypeptide chains, wherein a polypeptide chain 1 comprises an amino acid sequence as set forth in SEQ ID NO: 237; and a polypeptide chain 2 comprises an amino acid sequence as set forth in SEQ ID NO: 268.

In certain embodiments, the binding protein comprises two different polypeptide chains, wherein a polypeptide chain 1 comprises an amino acid sequence as set forth in SEQ ID NO: 237; and a polypeptide chain 2 comprises an amino acid sequence as set forth in SEQ ID NO: 269.

In certain embodiments, the binding protein comprises two different polypeptide chains, wherein a polypeptide chain 1 comprises an amino acid sequence as set forth in SEQ ID NO: 202; and a polypeptide chain 2 comprises an amino acid sequence as set forth in SEQ ID NO: 270.

In certain embodiments, the binding protein comprises two different polypeptide chains, wherein a polypeptide chain 1 comprises an amino acid sequence as set forth in SEQ ID NO: 212; and a polypeptide chain 2 comprises an amino acid sequence as set forth in SEQ ID NO: 271.

In certain embodiments, the binding protein comprises two different polypeptide chains, wherein a polypeptide chain 1 comprises an amino acid sequence as set forth in SEQ ID NO: 208; and a polypeptide chain 2 comprises an amino acid sequence as set forth in SEQ ID NO: 272.

In certain embodiments, the binding protein comprises two different polypeptide chains, wherein a polypeptide chain 1 comprises an amino acid sequence as set forth in SEQ ID NO: 208; and a polypeptide chain 2 comprises an amino acid sequence as set forth in SEQ ID NO: 273.

In certain embodiments, the binding protein comprises two different polypeptide chains, wherein a polypeptide chain 1 comprises an amino acid sequence as set forth in SEQ ID NO: 237; and a polypeptide chain 2 comprises an amino acid sequence as set forth in SEQ ID NO: 274.

In the present application, the CDRs may comprise mutations based on the defined sequences. The mutation is an insertion, deletion or substitution of 3, 2 or 1 amino acids on the basis of the amino acid sequences of the VH CDR1, the VH CDR2, the VH CDR3, the VL CDR1, the VL CDR2 and the VL CDR3. In the present application, "amino acid mutation" in the context like "insertion, deletion or substitution of 3, 2 or 1 amino acids" refers to a mutation of an amino acid in the sequence of a variant as compared to the sequence of an original amino acid, including the insertion, deletion or substitution of amino acids on the basis of the original amino acid sequence. An exemplary explanation is that the mutations to the CDRs may comprise 3, 2 or 1 amino acid mutations, and that the same or different numbers of amino acid residues can be optionally selected for the mutations to those CDRs, e.g., 1 amino acid mutation to CDR1, and no amino acid mutation to CDR2 and CDR3.

In the present application, the VH and VL or the polypeptide chain may comprise mutations based on the defined sequences. The mutation is a deletion, substitution or addition of one or more amino acid residues on the defined amino acid sequence, and the amino acid sequence with the mutation has at least 85% sequence identity to the defined amino acid sequence and maintains or improves the binding activity of the antibody or the antigen-binding fragment thereof and the binding protein, wherein the at least 85% sequence identity is preferably at least 90% sequence identity, more preferably at least 95% sequence identity, and most preferably at least 99% sequence identity.

In order to solve the above technical problems, a second aspect of the present invention provides an isolated nucleic acid encoding the binding protein according to the first aspect of the present invention.

In order to solve the above technical problems, a third aspect of the present invention provides a recombinant expression vector comprising the isolated nucleic acid according to the second aspect of the present invention.

Preferably, the expression vector comprises a eukaryotic cell expression vector and/or a prokaryotic cell expression vector.

In order to solve the above technical problems, a fourth aspect of the present invention provides a transformant comprising the isolated nucleic acid according to the second aspect of the present invention or the recombinant expression vector according to the third aspect of the present invention.

Preferably, the transformant has a host cell being a prokaryotic cell and/or a eukaryotic cell, wherein the prokaryotic cell is preferably an *E. coli* cell such as TG1 and BL21, and the eukaryotic cell is preferably an HEK293 cell or a CHO cell.

In order to solve the above technical problems, a fifth aspect of the present invention provides a method for preparing a binding protein, which comprises culturing the transformant according to the fourth aspect of the present invention, and obtaining the binding protein from a culture.

In order to solve the above technical problems, a sixth aspect of the present invention provides a pharmaceutical composition comprising the binding protein according to the first aspect of the present invention, and optionally a pharmaceutically acceptable carrier.

Preferably, the pharmaceutical composition further comprises an additional anti-tumor antibody as an active ingredient.

In order to solve the above technical problems, a seventh aspect of the present invention provides a kit comprising the binding protein according to the first aspect of the present invention and/or the pharmaceutical composition according to the sixth aspect of the present invention.

Preferably, the kit further comprises (i) a device for administering the binding protein or the pharmaceutical composition; and/or (ii) instructions.

In order to solve the above technical problems, an eighth aspect of the present invention provides a combination of kitscombination of kits comprising a kit I and a kit II, wherein the kit I comprises the binding protein according to the first aspect of the present invention and/or the pharmaceutical composition according to the sixth aspect of the present invention, and the kit II comprises an additional antibody or pharmaceutical composition. The kit I and the kit II may be used simultaneously, or the kit I may be used prior to the use of the kit II, or the kit II may be used prior to the use of the kit I. The sequence of use can be determined according to actual requirements in a specific application, i.e., corresponding to the case of combination therapy.

In order to solve the above technical problems, a ninth aspect of the present invention provides an administration device comprising the binding protein according to the first aspect of the present invention and/or the pharmaceutical composition according to the sixth aspect of the present invention.

Preferably, the administration device further comprises a component, such as a syringe, an infusion device or an implantable administration device, for containing or administering to a subject the binding protein and/or the pharmaceutical composition.

In order to solve the above technical problems, a tenth aspect of the present invention provides use of the binding protein according to the first aspect of the present invention, the pharmaceutical composition according to the sixth aspect of the present invention, the kit according to the seventh aspect of the present invention, the combination of kits according to the eighth aspect of the present invention, and/or the administration device according to the ninth aspect of the present invention in the preparation of a medicament for the diagnosis, prevention and/or treatment of a tumor.

Preferably, the tumor is a hematologic tumor or solid tumor, such as breast cancer, ovarian cancer, endometrial cancer, renal cancer, melanoma, pancreatic cancer, prostate cancer, lung cancer, gastric cancer, liver cancer, esophageal cancer, cervical cancer, head and neck neoplasm, cholangiocarcinoma, gallbladder cancer, bladder cancer, sarcoma and/or colorectal cancer.

In order to solve the above technical problems, an eleventh aspect of the present invention provides a method for detecting a specific antigen *in vitro* or *in vivo* comprising, which comprises detecting with the binding protein according to the first aspect of the present invention and/or the pharmaceutical composition according to the sixth aspect of the present invention.

In order to solve the above technical problems, a twelfth aspect of the present invention provides a method for diagnosing, preventing and/or treating a tumor, which comprises the step of administering to a patient in need thereof the binding protein according to the first aspect of the present invention, the pharmaceutical composition according to the sixth aspect of the present invention, the kit according to the seventh aspect of the present invention, the combination of kits according to the eighth aspect of the present invention and/or the administration device according to the ninth aspect of the present invention.

Preferably, the tumor is a hematologic tumor or solid tumor, such as breast cancer, ovarian cancer, endometrial cancer, renal cancer, melanoma, pancreatic cancer, prostate cancer, lung cancer, gastric cancer, liver cancer, esophageal cancer, cervical cancer, head and neck neoplasm, cholangiocarcinoma, gallbladder cancer, bladder cancer, sarcoma and/or colorectal cancer.

On the basis of the general knowledge in the art, the above preferred conditions can be combined arbitrarily to obtain preferred embodiments of the present invention.

The reagents and starting materials used in the present invention are commercially available.

The beneficial effects of the present invention are as follows:

The binding protein of the present invention can enhance the immune response (e.g., the activity of T cells or NK cells) against a specific target cell (e.g., a tumor cell), and can bring an immune effector cell and a specific target cell (e.g., a tumor cell) together, and efficiently and selectively activate the immune effector cell in a microenvironment of the specific target cell (e.g., a tumor cell), thereby enhancing immune responses (e.g., promoting the activation of T cells and the production of cytokines), and reducing toxic and side effects caused by non-specific activation of peripheral immune cells. By utilizing the crosslinking of the costimulatory molecules mediated by target cell antigens and the activation of downstream signaling pathways, the binding proteins promote the activation and proliferation of the immune cells, and improve the efficacy of killing the tumors. The binding protein of the present invention has excellent universality and druggability, and shows excellent biological activity, molecular stability and pharmaceutical properties on a plurality of bispecific antibody molecules with different combinations of targets.

### BRIEF DESCRIPTION OF THE DRAWINGS

FIG. 1 shows the schematic diagrams of molecular structures.
FIG.2 shows the binding activity of PD-L1×4-1BB to human 4-1BB cells CHO-K1/hu 4-1BB.
FIG.3 shows the binding activity of B7H4×4-1BB to human 4-1BB cells CHO-K1/hu 4-1BB.
FIG. 4shows the binding activity of HER2×4-1BB to human 4-1BB cells CHO-K1/hu 4-1BB.
FIG.5 shows the binding activity of EPCAM×4-1BB to human 4-1BB cells CHO-K1/hu 4-1BB.
FIG.6 shows the binding activity of PSMA×4-1BB to human 4-1BB cells CHO-K1/hu 4-1BB.
FIG.7 shows the binding activity of CLDN18.2×4-1BB to human 4-1BB cells CHO-K1/hu 4-1BB.
FIG. 8 shows the binding activity of the antibody molecules to cynomolgus monkey 4-1BB cells CHO-K1/cyno 4-1BB.
FIG.9 shows the binding activity of the antibody molecules to cynomolgus monkey OX40 proteins.
FIG. 10 shows the binding activity of the antibody molecules to human OX40 cells CHO-K1/hu OX40.
FIG. 11 shows the binding activity of PD-L1×4-1BB to human PD-L1 cells CHO-K1/hPD-L1.
FIG.12 shows the binding activity of B7H4×4-1BB to tumor cells SK-BR-3.
FIG.13 shows the binding activity of HER2×4-1BB to tumor cells SK-BR-3.
FIG.14 shows the binding activity of EPCAM×4-1BB to tumor cells Capan-2.
FIG.15 Bshows the binding activity of PSMA×4-1BB to tumor cells LNCaP.
FIG.16 shows the binding activity of PD-L1×OX40 to human PD-L1 cells CHO-K1/hPD-L1.
FIG. 17 shows the binding activity of B7H4×OX40 to tumor cells SK-BR-3.
FIG. 18 shows the binding activity of EPCAM×OX40 to tumor cells Capan-2.
FIG. 19 shows the binding activity of PSMA×OX40 to tumor cells LNCaP.
FIG.20 shows the binding activity of CLDN18.2×OX40 to tumor cells NUGC-4.
FIG.21 shows the T cell specific activation mediated by CLDN18.2×4-1BB in NUGC-4 cells.
FIG.22 shows the T cell specific activation mediated by CLDN18.2×OX40 in NUGC-4 cells.
FIG.23 shows the T cell specific activation mediated by PSMA×4-1BB in LNCaP cells.
FIG.24 shows the T cell specific activation mediated by PSMA×OX40 in HEK293/hPSMA cells.
FIG.25 shows the T cell specific activation mediated by EPCAM×4-1BB in Capan-2 cells.
FIG.26 shows the T cell specific activation mediated by EPCAM×OX40 in Capan-2 cells.
FIG.27 shows the T cell specific activation mediated by PD-L1×4-1BB in CHO-K1/hPD-L1 cells.
FIG.28 shows the T cell specific activation mediated by PD-L1×4-1BB in MDA-MB-231 cells.
FIG.29 shows the T cell specific activation mediated by PD-L1×OX40 in MDA-MB-231 cells.
FIG.30 shows the T cell specific activation mediated by HER2×4-1BB in SK-BR-3 cells.
FIG.31 shows the T cell specific activation mediated by B7H4×OX40 in CHO-K1/hB7H4 cells.
FIG.32 shows the T cell specific activation mediated by B7H4×4-1BB in SK-BR-3 cells.
FIG.33 shows that the T cell activation by B7H4×4-1BB is specifically dependent on the expression of B7H4: (A) in the presence of SK-BR-3 cells highly expressing B7H4, B7H4×4-1BB is able to activate T cells; (B) in the presence of JIMT-1 cells not expressing B7H4, B7H4×4-1BB is unable to activate T cells.
FIG.34 shows the T cell activation by PD-L1×4-1BB in mixed lymphocyte reaction (MLR) assays: the IFNγ levels on day 5 (A) and the IL-2 levels on day 3 (B) determined in the first MLR assay; the IFNγ levels on day 5 (C) and the IL-2 levels on day 3 (D) determined in the second MLR assay.
FIG.35 shows the pharmacokinetics of the bispecific antibody molecules in mice.
FIG.36 shows the anti-tumor effect of B7H4×4-1BB in a BALB/c hCD137-KI CT26-hB7H4 tumor mouse model.
FIG.37 shows the schematic diagrams of other molecular structures.
FIG.38 shows the schematic diagrams of the combination of targeting moiety and effector moiety.

### DETAILED DESCRIPTION

The embodiments of the present invention are described below with reference to specific examples, and other advantages and effects of the present invention will be readily apparent to those skilled in the art from the disclosure of the present specification.

In the present application, the term "binding protein" or "antigen-binding protein" generally refers to a protein comprising an antigen-binding moiety, and optionally a scaffold or framework moiety that allows the antigen-binding moiety to adopt a conformation that facilitates the binding of the antigen-binding protein to the antigen. An antibody may typically comprise an antibody light chain variable region (VL) or an antibody heavy chain variable region (VH), or both. The VH and VL regions can be further divided into hypervariable regions termed complementarity determining regions (CDRs), which are scattered over more conserved regions termed framework regions (FRs). Each VH and VL can consist of three CDR regions and four FR regions arranged from amino-terminus to carboxyl-terminus in the following order: FR-1, CDR1, FR-2, CDR2, FR-3, CDR3 and FR-4. The variable regions of the heavy and light chains comprise binding domains that interact with antigens. The three CDRs of VH are denoted as HCDR1, HCDR2 and HCDR3, respectively, and may also be denoted as VH CDR1, VH CDR2 and VH CDR3, respectively; and the three CDRs of VL are denoted as LCDR1, LCDR2 and LCDR3, respectively, and may also be denoted as VL CDR1, VL CDR2 and VL CDR3, respectively. Examples of the antigen-binding proteins include, but are not limited to, antibodies, antigen-binding fragments (Fab, Fab', F(ab)₂, Fv fragment, F(ab')₂, scFv, di-scFv and/or dAb), immunoconjugates, multispecific antibodies (e.g., bispecific antibodies), antibody fragments, antibody derivatives, antibody analogs or fusion proteins, as long as they exhibit the desired antigen-binding activity.

In the present application, the amino acid sequences of the CDRs are shown according to the Chothia scheme. However, it is well known to those skilled in the art that the CDRs of an antibody can be defined in the art using a variety of methods, such as the Kabat scheme based on sequence variability (see Kabat et al., Sequences of Proteins of Immunological Interest, Fifth Edition, National Institutes of Health (U.S.), Bethesda, Maryland (1991)), and the Chothia scheme based on the location of the structural loop regions (see J Mol Biol 273: 927-948, 1997). In the technical solution of the present invention, the Combined scheme comprising the Kabat scheme and the Chothia scheme can also be used to determine the amino acid residues in a variable domain sequence. The Combined scheme combines the Kabat scheme with the Chothia scheme to obtain a larger range. See the table below for details. It will be understood by those skilled in the art that unless otherwise specified, the terms "CDR" and "complementarity determining region" of a given antibody or a region (e.g., variable region) thereof are construed as encompassing complementary determining regions as defined by any one of the above known schemes described herein. Although the scope claimed in the present invention is the sequences shown based on the Chothia scheme, the amino acid sequences corresponding to the other schemes for numbering CDRs shall also fall within the scope of the present invention.

**Table-I. The schemes for numbering the CDRs of the antibody of the present application**

| | Kabat | Chothia | Combined |
|---|---|---|---|
| LCDR1 | L24--L34 | L24--L34 | L24-L34 |
| LCDR2 | L50--L56 | L50--L56 | L50-L56 |
| LCDR3 | L89--L97 | L89--L97 | L89-L97 |
| HCDR1 | H31--H35 | H26--H32 | H26-H35 |
| HCDR2 | H50--H65 | H52--H56 | H50-H65 |
| HCDR3 | H95--H102 | H95--H102 | H95-H102 |

Laa-Lbb can refer to an amino acid sequence from position aa (the Chothia scheme) to position bb (the Chothia scheme) beginning at the N-terminus of the light chain of the antibody; and Haa-Hbb can refer to an amino acid sequence from position aa (the Chothia scheme) to position bb (the Chothia scheme) beginning at the N-terminus of the heavy chain of the antibody. For example, L24-L34 can refer to the amino acid sequence from position 24 to position 34 according to the Chothia scheme beginning at the N-terminus of the light chain of the antibody; H26-H32 can refer to the amino acid sequence from position 26 to position 32 according to the Chothia scheme beginning at the N-terminus of the heavy chain of the antibody. It should be known to those skilled in the art that there are positions where insertion sites are present in numbering CDRs with the Chothia scheme (see http://bioinf.org.uk/abs/).

In the present application, the term "monoclonal antibody" generally refers to an antibody obtained from a population of substantially homogeneous antibodies, that is, the individual antibodies in the population are identical except for a small amount of natural mutations that may exist. Monoclonal antibodies are generally highly specific for a single antigenic site. Moreover, unlike conventional polyclonal antibody preparations (which generally have different antibodies directed against different determinants), each monoclonal antibody is directed against a single determinant on the antigen. In addition to their specificity, monoclonal antibodies have the advantage that they can be synthesized by hybridoma culture without contamination by other immunoglobulins. The modifier "monoclonal" indicates the characteristic of the antibody obtained from a population of substantially homogeneous antibodies, and is not to be construed as requiring production of the antibody by any particular method. For example, monoclonal antibodies used according to the present invention can be prepared in hybridoma cells or can be prepared by the recombinant DNA method.

In the present application, the term "fully human antibody" generally refers to an antibody that is expressed by a genetically engineered antibody gene-deleted animal into which the entire gene that encode an antibody in human is transferred. All parts of the antibody (including the variable and constant regions of the antibody) are encoded by genes of human origin. The fully human antibody can greatly reduce the immune side effects caused in the human body by the heterologous antibody. Methods for obtaining fully human antibodies in the art can include phage display, transgenic mice, and the like.

In the present application, the term "specifically bind to" generally refers to that an antibody binds to an epitope via its antigen-binding domain, and that the binding requires some complementarity between the antigen-binding domain and the epitope. According to this definition, an antibody is said to "specifically bind to" an antigen when the antibody more easily binds to an epitope via its antigen-binding domain than binds to a random, unrelated epitope. "Epitope" refers to a specific atomic group (e.g., saccharide side chain, phosphoryl, sulfonyl) or an amino acid on an antigen that binds to an antigen-binding protein (e.g., an antibody).

In the present application, the term "Fab" generally refers to the portion of a conventional antibody (e.g., IgG) that binds to an antigen, including the heavy chain variable region VH, the light chain variable region VL, the heavy chain constant region domain CH1 and the light chain constant region CL of the antibody. In conventional antibodies, the C-terminus of VH is linked to the N-terminus of CH1 to form a heavy chain Fd fragment, the C-terminus of VL is linked to the N-terminus of CL to form a light chain, and the C-terminus of CH1 is further linked to the hinge region and other constant region domains of the heavy chain to form a heavy chain. In some embodiments, "Fab" also refers to a variant structure of the Fab. For example, in certain embodiments, the C-terminus of VH is linked to the N-terminus of CL to form one polypeptide chain, and the C-terminus of VL is linked to the N-terminus of CH1 to form the other polypeptide chain, in which case an Fab (cross VH/VL) structure is formed; in certain embodiments, CH1 of the Fab is not linked to the hinge region, but rather the C-terminus of CL is linked to the hinge region of the heavy chain, in which case a Fab (cross Fd/LC) structure is formed.

In the present application, the term "VH" generally refers to the heavy chain variable region VH domain of an antibody, i.e., the heavy chain variable region VH of a conventional antibody (H2L2 structure) from human or other animals, the heavy chain variable region VHH of a heavy-chain antibody (HCAb structure) from animals such as those of Camelidae species, or the heavy chain variable region VH of a fully human heavy-chain antibody (HCAb structure) produced using a Harbour HCAb transgenic mouse.

In the present application, the term "antigen-binding fragment" generally refers to any protein functional region that can specifically bind to the antigen, either "Fab" or "VH", or other antigen-binding forms (e.g., derived protein structures such as lipocalins, neuronal cell adhesion molecules (NCAMs), fibronectins, and designed ankyrin repeat proteins (DARPins)).

In the present application, the term "tumor antigen" may be either a tumor specific antigen (TSA) or a tumor-associated antigen (TAA). Tumor specific antigen refers to an antigen that is specific to tumor cells and is not present in normal cells or tissues. Tumor-associated antigen is not specific to tumor cells and is also present in normal cells or tissues, but is highly expressed when tumor cells proliferate.

In the present application, the term "target cell" refers to a cell that needs to be eliminated, mainly a tumor cell, and may be an immunosuppressive cell or the like.

In the present application, the term "immune effector cell" generally refers to an immune cell involved in the clearance of foreign antigens and performing effector functions in an immune response, e.g., a plasma cell, a cytotoxic T cell, a NK cell, and the like.

In the present application, the term "costimulatory molecule", "costimulatory molecule antigen" or "stimulatory antigen" refers to a cell surface molecule and its ligand that provides a costimulatory signal for the complete activation of an immune cell, such as a T cell or B cell, which positively regulate the activation of the immune cell, e.g., CD28, 4-1BB, ICOS, OX40, CD40, and the like.

In the present application, the term "co-inhibitory molecule" or "co-inhibitory molecule antigen" is a class of cell surface molecules and their ligands that negatively regulate the functions of immune cells, e.g., CTLA-4, PD-L1, PD-1, and the like.

In the present application, the term "effector moiety" refers to an antigen-binding fragment binding to an immune effector cell.

In the present application, the term "targeting moiety" refers to an antigen-binding fragment binding to a target cell (e.g., a tumor cell).

In the present application, the term "PD-L1" generally refers to the programmed death ligand 1 protein, a functional variant thereof and/or a functional fragment thereof. PD-L1 is also known as cluster of differentiation 274 (CD274) or B7 homologue 1 (B7-H1), and is a protein encoded by the CD274 gene (in human). The sequence of PD-L1 is known in the art. For example, the amino acid sequence of an exemplary full-length human PD-L1 protein can be found under NCBI accession No. NP_054862 or UniProt accession No. Q9NZQ7; and the sequence of an exemplary full-length cynomolgus monkey PD-L1 protein can be found under NCBI accession No. XP_005581836 or Uniprot accession No. G7PSE7.

In the present application, the term "HER2" generally refers to the receptor tyrosine kinase erbB-2 (also known as ERBB2), a functional variant thereof and/or a functional fragment thereof. The sequence of HER2 is known in the art. For example, the sequence of an exemplary full-length human HER2 can be found under Uniprot accession No. P04626; and the sequence of an exemplary full-length cynomolgus monkey HER2 can be found under NCBI accession No. XP_005584091.

In the present application, the term "B7H4" generally refers to the V-Set domain-containing T-cell activation inhibitor 1 (also known as VTCN1), a functional variant thereof and/or a functional fragment thereof. The sequence of B7H4 is known in the art. For example, the sequence of an exemplary full-length human B7H4 can be found under Uniprot accession No. Q7Z7D3; the sequence of an exemplary full-length cynomolgus monkey B7H4 can be found under NCBI accession No. XP_005542249; and the sequence of an exemplary full-length mouse B7H4 can be found under Uniprot accession No. Q7TSP5.

In the present application, the term "4-1BB" generally refers to the tumor necrosis factor receptor superfamily member 9 (also known as CD137, 4-1BBL receptor), a functional variant thereof and/or a functional fragment thereof. The sequence of 4-1BB is known in the art. For example, the sequence of an exemplary full-length human 4-1BB can be found under Uniprot accession No. Q07011; and the sequence of an exemplary full-length cynomolgus monkey 4-1BB can be found under NCBI accession No. XP_005544945.

In the present application, the term "OX40" generally refers to the tumor necrosis factor receptor superfamily member 4 (also known as CD134 or OX40L receptor), a functional variant thereof and/or a functional fragment thereof. The sequence of OX40 is known in the art. For example, the sequence of an exemplary full-length human OX40 can be found under Uniprot accession No. P43489; and the sequence of an exemplary full-length cynomolgus monkey OX40 can be found under NCBI accession No. XP_005545179.

In the present application, the term "EPCAM" generally refers to the epithelial cell adhesion molecule (also known as CD326), a functional variant thereof and/or a functional fragment thereof. The sequence of EPCAM is known in the art. For example, the sequence of an exemplary full-length human EPCAM can be found under Uniprot accession No. P16422; and the sequence of an exemplary full-length cynomolgus monkey or rhesus monkey EPCAM can be found under NCBI accession No. NP_001035118.

In the present application, the term "PSMA" generally refers to the prostate specific membrane antigen (also known as FOLH1, glutamate carboxypeptidase 2), a functional variant thereof and/or a functional fragment thereof. The sequence of PSMA is known in the art. For example, the sequence of an exemplary full-length human PSMA can be found under Uniprot accession No. Q04609; and the sequence of an exemplary full-length cynomolgus monkey PSMA can be found under NCBI accession No. XP_005579379.

In the present application, the term "Claudin-18" or "CLDN18" generally refers to a protein encoded by the CLDN18 gene in humans, which belongs to the cellular tight junction protein family, and can control the flow of molecules between layer cells. Claudin-18 has two splice variants, Claudin 18.1 and Claudin 18.2, with only eight amino acids different between the two sequences. The expression profiles of Claudin 18.1 and Claudin 18.2 are different. Claudin 18.1 is selectively expressed in cells of the normal lung, and Claudin 18.2 is highly restricted in normal cells, but is activated and highly expressed in a variety of tumors. The sequence of Claudin-18 is known in the art. For example, the sequence of an exemplary full-length human Claudin-18 can be found under Uniprot accession No. P56856; and correspondingly, the sequence of the splice variant Claudin 18.1 is P56856-1, and the sequence of the splice variant Claudin 18.2 is P56856-2.

In the present application, the term "CLDN18.1" refers specifically to the splice variant Claudin 18.1 of "Claudin-18".

In the present application, the term "CLDN18.2" refers specifically to the splice variant Claudin 18.2 of "Claudin-18".

### Examples

The present invention is further illustrated by the following examples, which are not intended to limit the present invention. The examples do not include detailed descriptions of conventional methods, such as those methods for constructing vectors and plasmids, methods for inserting genes encoding proteins into such vectors and plasmids, or methods for introducing plasmids into host cells. Such methods are well known to those of ordinary skill in the art and are described in numerous publications. Experimental procedures without specified conditions in the following examples are performed in accordance with conventional procedures and conditions, or in accordance with instructions.

### Example 1. Multispecific Immune Cell Engager Binding Proteins based on Heavy-Chain Antibody

In this example, the structures of several multispecific immune cell engager binding proteins constructed using fully human heavy-chain antibodies (HCAbs) and single-domain antibodies (sdAbs) derived therefrom were listed. In some structures, the domains were linked via linker peptides. In some structures, amino acid mutations were introduced into the Fc region of the heavy chain to alter its binding to Fc receptors, thereby altering the associated effector functions or other properties.

The molecular structures of the multispecific binding proteins to which the present application relates are list in Table 1 and FIG. 1, each of which will be further described below. The preferred structures are structure 1, structure 2 and structure 3. The sequences of the linker peptides that may be used in the structural design of the present application are listed in Table 2. The molecular structures of other derived bi- (multi-) specific antibodies based on heavy-chain antibodies or single-domain antibodies are listed in FIG. 37.

**Table 1. Molecular structures of multispecific binding proteins listed in the present application**

| **Structur e No.** | **Structure mode** | **Structure type** | **Binding valence** | **Symmetr y** | **Number of different polypeptid e chains** |
|---|---|---|---|---|---|
| 1 | IgG_HC-VH | IgG-VH | Tetravale nt | Symmetri c | 2 |
| 2 | IgG_HC-VH-VH | IgG-VH(2) | Hexavale nt | Symmetri c | 2 |
| 3 | Fab(CL)-VH-Fc | Fab-HCAb | Tetravale nt | Symmetri c | 2 |
| 4 | Fab(CH1)-VH-Fc | Fab-HCAb | Tetravale nt | Symmetri c | 2 |
| 5 | Fab(CL)-VH-Fc-VH | Fab-VH-Fc-VH | Hexavale nt | Symmetri c | 2 |
| 6 | Fab(CH1)-VH-Fc-VH | Fab-VH-Fc-VH | Hexavale nt | Symmetri c | 2 |
| 7 | VH-IgG_HC | VH-IgG | Tetravale nt | Symmetri c | 2 |
| 8 | FIT-Ig | FIT-Ig | Tetravale nt | Symmetri c | 3 |

**Table 2. Sequences of linker peptides**

| **Name of linker peptide** | **Lengt h** | | **Sequence** | **SEQ ID NO** |
|---|---|---|---|---|
| GS_2 | 2 | | GS | |
| GS_4 | 4 | | GSGS | 244 |
| GS_5 | 5 | | GGGGS | 245 |
| GS_7 | 7 | | GGGGSGS | 246 |
| GS_15 | 15 | | GGGGSGGGGSGGGGS | 247 |
| GS_20 | 20 | | GGGGSGGGGSGGGGSGGGGS | 248 |
| GS_25 | 25 | | GGGGSGGGGSGGGGSGGGGSGGGGS | 249 |
| H1_15 | 15 | | EPKSSDKTHTPPPPP | 250 |
| LH1 | 10 | | DKTHTCPPCP | 251 |
| G5-LH | 15 | | GGGGGDKTHTCPPCP | 252 |
| H1_15-RT | 17 | | EPKSSDKTHTPPPPPRT | 253 |
| L-GS_15-RT | 18 | | LGGGGSGGGGSGGGGSRT | 254 |
| L-H1_15-RT | 18 | | LEPKSSDKTHTPPPPPRT | 255 |
| KL-H1_15-R | 19 | | KLEPKSSDKTHTPPPPPRT | 256 |
| KL-H1_15-A S | 19 | | KLEPKSSDKTHTPPPPPAS | 257 |
| RT-GS_5-KL | 9 | | RTGGGGSKL | 258 |
| RT-GS_15-K L | 19 | | RTGGGGSGGGGSGGGGSKL | 259 |
| RT-GS_25-K L | 29 | | RTGGGGSGGGGSGGGGSGGGGSGGGGS KL | 260 |
| Human IgG1 hinge | 15 | | EPKSCDKTHTCPPCP | 261 |
| Human IgG1 hinge (C220S) | 15 | | EPKSSDKTHTCPPCP | 262 |
| Human IgG2 hinge | 12 | | ERKCCVECPPCP | 263 |
| Human IgG4 hinge | 12 | | ESKYGPPCPSCP | 264 |
| Human IgG4 hinge (S228P) | 12 | | ESKYGPPCPPCP | 265 |

### Example 1.1 Structures of bispecific binding proteins containing heavy-chain antibody VH domains

The present invention provides a method for constructing a bispecific binding protein using two parent monoclonal antibodies: a conventional antibody A binding to a first antigen and a heavy-chain antibody B binding to a second antigen.

As shown in structure (1) to structure (7) in FIG. 1, the Fab end is derived from a conventional antibody A, wherein VH_A and VL_A are heavy chain and light chain variable regions of the antibody A, respectively. The VH end is derived from a heavy-chain antibody B, wherein VH_B is a heavy chain variable region of the heavy-chain antibody B. CL is a light chain constant region domain. CH1, CH2 and CH3 are first, second and third domains of a heavy chain constant region, respectively. h is a hinge region or derived sequence of an IgG antibody, and L or L1 or L2 or L3 is a linker peptide.

### Example 1.1.1 Structure (1): IgG_HC-VH

The binding protein with the structure (1) comprises two different polypeptide chains: a polypeptide chain 1, also known as a short chain, comprising VL_A-CL from the amino-terminus to the carboxyl-terminus; and a polypeptide chain 2, also known as a long chain, comprising VH_A-CH1-h-CH2-CH3-L-VH_B from the amino-terminus to the carboxyl-terminus.

In one embodiment, CH3 is fusion-linked directly to VH_B in the polypeptide chain 2, i.e., L is 0 in length. In another embodiment, CH3 is linked to VH_B via a linker peptide L in the polypeptide chain 2; and L may be the sequence listed in Table 2.

### Example 1.1.2 Structure (2): IgG_HC-VH-VH

The binding protein with the structure (2) comprises two different polypeptide chains: a polypeptide chain 1, also known as a short chain, comprising VL_A-CL from the amino-terminus to the carboxyl-terminus; and a polypeptide chain 2, also known as a long chain, comprising VH_A-CH1-h-CH2-CH3-L1-VH_B-L2-VH_B from the amino-terminus to the carboxyl-terminus.

In one embodiment, CH3 is fusion-linked directly to VH_B in the polypeptide chain 2, i.e., L1 is 0 in length. In another embodiment, CH3 is linked to VH_B via a linker peptide L1 in the polypeptide chain 2; and L1 may be the sequence listed in Table 2.

In one embodiment, a first VH_B is fusion-linked directly to a second VH_B in the polypeptide chain 2, i.e., L2 is 0 in length. In another embodiment, a first VH_B is linked to a second VH_B via a linker peptide L2 in the polypeptide chain 2; and L2 may be the sequence listed in Table 2.

### Example 1.1.3 Structure (3): Fab(CL)-VH-Fc

The binding protein with the structure (3) comprises two different polypeptide chains: a polypeptide chain 1, also known as a short chain, comprising VB_A-CH1 from the amino-terminus to the carboxyl-terminus; and a polypeptide chain 2, also known as a long chain, comprising VL_A-CL-L1-VH_B-L2-CH2-CH3 from the amino-terminus to the carboxyl-terminus. In the structure (3), VL_A of the antibody A and VH_B of the heavy-chain antibody B are fused on the same polypeptide chain, so that the mismatched byproducts generated by the association of VL_A and VH_B can be avoided.

VH_B is linked to CH2 via a linker peptide L2 in the polypeptide chain 2; L2 may be a hinge region or a hinge region-derived linker peptide sequence of IgG or the sequence listed in Table 2, preferably the sequence of human IgG1 hinge region, human IgG1 hinge (C220S) or G5-LH.

In one embodiment, CL is fusion-linked directly to VH_B in the polypeptide chain 2, i.e., L1 is 0 in length. In another embodiment, CL is linked to VH_B via a linker peptide L1 in the polypeptide chain 2; and L1 may be the sequence listed in Table 2.

### Example 1.1.4 Structure (4): Fab(CH1)-VH-Fc

The binding protein with the structure (4) comprises two different polypeptide chains: a polypeptide chain 1, also known as a short chain, comprising VL_A-CL from the amino-terminus to the carboxyl-terminus; and a polypeptide chain 2, also known as a long chain, comprising VH_A-CH1-L1-VH_B-L2-CH2-CH3 from the amino-terminus to the carboxyl-terminus.

VH_B is linked to CH2 via a linker peptide L2 in the polypeptide chain 2; L2 may be a hinge region or a hinge region-derived linker peptide sequence of IgG or the sequence listed in Table 2, preferably the sequence of human IgG1 hinge region, human IgG1 hinge (C220S) or G5-LH.

In one embodiment, CH1 is fusion-linked directly to VH_B in the polypeptide chain 2, i.e., L1 is 0 in length. In another embodiment, CH1 is linked to VH_B via a linker peptide L1 in the polypeptide chain 2; and L1 may be the sequence listed in Table 2.

### Example 1.1.5 Structure (5): Fab(CL)-VH-Fc-VH

The binding protein with the structure (5) comprises two different polypeptide chains: a polypeptide chain 1, also known as a short chain, comprising VH_A-CH1 from the amino-terminus to the carboxyl-terminus; and a polypeptide chain 2, also known as a long chain, comprising VL_A-CL-L1-VH_B-L2-CH2-CH3-L3-VH_B from the amino-terminus to the carboxyl-terminus. In the structure (5), VL_A of the antibody A and VH_B of the heavy-chain antibody B are fused on the same polypeptide chain, so that the mismatched byproducts generated by the association of VL_A and VH_B can be avoided.

VH_B is linked to CH2 via a linker peptide L2 in the polypeptide chain 2; L2 may be a hinge region or a hinge region-derived linker peptide sequence of IgG or the sequence listed in Table 2, preferably the sequence of human IgG1 hinge region, human IgG1 hinge (C220S) or G5-LH.

In one embodiment, CL is fusion-linked directly to a first VH_B in the polypeptide chain 2, i.e., L1 is 0 in length. In another embodiment, CL is linked to a first VH_B via a linker peptide L1 in the polypeptide chain 2; and L1 may be the sequence listed in Table 2.

In one embodiment, CH3 is fusion-linked directly to a second VH_B in the polypeptide chain 2, i.e., L3 is 0 in length. In another embodiment, CH3 is linked to a second VH_B via a linker peptide L3 in the polypeptide chain 2; and L3 may be the sequence listed in Table 2.

### Example 1.1.6 Structure (6): Fab(CH1)-VH-Fc-VH

The binding protein with the structure (6) comprises two different polypeptide chains: a polypeptide chain 1, also known as a short chain, comprising VL_A-CL from the amino-terminus to the carboxyl-terminus; and a polypeptide chain 2, also known as a long chain, comprising VH_A-CH1-L1-VH_B-L2-CH2-CH3-L3-VH_B from the amino-terminus to the carboxyl-terminus.

VH_B is linked to CH2 via a linker peptide L2 in the polypeptide chain 2; L2 may be a hinge region or a hinge region-derived linker peptide sequence of IgG or the sequence listed in Table 2, preferably the sequence of human IgG1 hinge region, human IgG1 hinge (C220S) or G5-LH.

In one embodiment, CH1 is fusion-linked directly to a first VH_B in the polypeptide chain 2, i.e., L1 is 0 in length. In another embodiment, CH1 is linked to a first VH_B via a linker peptide L1 in the polypeptide chain 2; and L1 may be the sequence listed in Table 2.

In one embodiment, CH3 is fusion-linked directly to a second VH_B in the polypeptide chain 2, i.e., L3 is 0 in length. In another embodiment, CH3 is linked to a second VH_B via a linker peptide L3 in the polypeptide chain 2; and L3 may be the sequence listed in Table 2.

### Example 1.1.7 Structure (7): VH-IgG_HC

The binding protein with the structure (7) comprises two different polypeptide chains: a polypeptide chain 1, also known as a short chain, comprising VL_A-CL from the amino-terminus to the carboxyl-terminus; and a polypeptide chain 2, also known as a long chain, comprising VH_B-L-VH_A-CH1-h-CH2-CH3 from the amino-terminus to the carboxyl-terminus.

In one embodiment, VH_B is fusion-linked directly to VH_A in the polypeptide chain 2, i.e., L is 0 in length. In another embodiment, VH_B is linked to VB_A via a linker peptide L in the polypeptide chain 2; and L may be the sequence listed in Table 2.

### Example 1.2 FIT-Ig bispecific binding protein structure

The FIT-Ig structure can be designed by referring to WO2015/103072A1, and the structure is as shown in the structure (8) in FIG. 1. The bispecific antibody molecules with the FIT-Ig structure can be constructed from: a conventional antibody A binding to a first antigen and a conventional antibody B binding to a second antigen.

As shown in the structure (8) in FIG. 1, VH_A and VL_A are heavy chain and light chain variable regions of the antibody A, respectively. VH_B and VL_B are heavy chain and light chain variable regions of the antibody B, respectively. CL is a light chain constant region domain. CH1, CH2 and CH3 are first, second and third domains of a heavy chain constant region, respectively. h is a hinge region or a derived sequence of an IgG antibody; and L is a linker peptide.

### Example 1.2.1 Structure (8): FIT-Ig

The binding protein with the structure (8) comprises three polypeptide chains: a polypeptide chain 1 comprising VL_A-CL-L-VH_B-CH1-h-CH2-CH3 from the amino-terminus to the carboxyl-terminus; a polypeptide chain 2 comprising VH_A-CH1 from the amino-terminus to the carboxyl-terminus; and a polypeptide chain 3 comprising VL_B-CL from the amino-terminus to the carboxyl-terminus.

In one embodiment, CL is fusion-linked directly to VH_B in the polypeptide chain 1, i.e., L is 0 in length. In another embodiment, CL is linked to VH_B via a linker peptide L in the polypeptide chain 1; and L may be the sequence listed in Table 2.

### Example 2. Sequence Analysis, Expression and Purification, and Characterization and Analysis of Physicochemical Properties of Antibodies

### Example 2.1 Expression and purification of antibodies

In this example, a general method for preparing antibodies in mammalian host cells (e.g., human embryonic kidney cell HEK293 or Chinese hamster ovary CHO cells and cells derived therefrom) by such techniques as transient transfection and expression, and affinity capture and separation was described. This method is applicable to an antibody of interest comprising an Fc region. The antibody of interest may consist of one or more protein polypeptide chains, and may be derived from one or more expression plasmids.

The amino acid sequences of the polypeptide chains of the antibody were converted into nucleotide sequences by codon optimization. The encoding nucleotide sequences were synthesized and cloned into expression vectors compatible with the host cell. The mammalian host cells were transfected simultaneously with plasmids encoding the polypeptide chains of the antibody in a particular ratio, and the recombinant antibody with correct folding and assembly of polypeptide chains could be obtained by the conventional recombinant protein expression and purification techniques. Specifically, FreeStyle^{™} 293-F cells (Thermo, #R79007) were expanded in FreeStyle^{™} F17 Expression Medium (Thermo, #A1383504). Before transient transfection, the cells were adjusted to a concentration of 6-8×10⁵ cells/mL, and cultured in a shaker at 37 °C with 8% CO₂ for 24 h to make a concentration of 1.2×10⁶ cells/mL. 30 mL of cultured cells were taken. Plasmids encoding the polypeptide chains of the antibody were mixed in a certain ratio, and a total of 30 µg of the plasmids (the ratio of the plasmids to cells was 1 µg : 1 mL) were dissolved in 1.5 mL of Opti-MEM reduced serum medium (Thermo, #31985088). The resulting mixture was filtered through a 0.22 µm filter membrane for sterilization. Then, 1.5 mL of Opti-MEM was dissolved in 120 µL of 1 mg/mL PEI (Polysciences, #23966-2), and the mixture was left to stand for 5 min. PEI was slowly added to the plasmids, and the mixture was incubated at room temperature for 10 min. The mixed solution of plasmids and PEI was slowly added dropwise while shaking the culture flask, and the cells were cultured in a shaker at 37 °C with 8% CO₂ for 5 days. Cell viability was measured after 5 days. The culture was collected and centrifuged at 3300 g for 10 min, and then the supernatant was collected and centrifuged at high speed to remove impurities. A gravity column (Bio-Rad, #7311550) containing MabSelect^{™} (GE Healthcare, #71-5020-91) was equilibrated with a PBS buffer (pH 7.4) and rinsed with 2-5 column volumes of PBS. The column was loaded with the supernatant sample, and rinsed with 5-10 column volumes of PBS buffer, followed by 0.1 M glycine at pH 3.5 to elute the target protein. The eluate was adjusted to neutrality with Tris-HCl at pH 8.0, and concentrated and buffer exchanged into PBS buffer or a buffer with other components with an ultrafiltration tube (Millipore, #UFC901024) to obtain a purified solution of the recombinant antibody. Finally, the purified antibody solution was determined for concentration using NanoDrop (Thermo, NanoDrop^{™} One), subpackaged and stored for later use.

### Example 2.2 Analysis of protein purity and polymers by SEC-HPLC

In this example, analytical size-exclusion chromatography (SEC) was used to analyze the protein sample for purity and polymer form. An analytical chromatography column TSKgel G3000SWxl (Tosoh Bioscience, #08541, 5 µm, 7.8 mm×30 cm) was connected to a high-pressure liquid chromatograph HPLC (Agilent Technologies, Agilent 1260 Infinity II) and equilibrated with a PBS buffer at room temperature for at least 1 h. A proper amount of the protein sample (at least 10 µg) was filtered through a 0.22 µm filter membrane and then injected into the system, and an HPLC program was set: the sample was eluted in the chromatography column with a PBS buffer at a flow rate of 1.0 mL/min for a maximum of 25 min. An analysis report was generated by the HPLC, with the retention time of the components with different molecular sizes in the sample reported.

### Example 2.3 Determination of molecular stability and molecular aggregation of proteins by Uncle

Uncle (Unchained Labs) is a multifunctional one-stop protein stability analysis platform that characterizes protein stability by total fluorescence, static light scattering (SLS) and dynamic light scattering (DLS) assay methods. The parameters of melting temperature (Tm), aggregation temperature (Tagg) and particle size (diameter) can be obtained simultaneously for the same group of samples. In this example, an "Tm & Tagg with optional DLS" application of Uncle was selected for the determination. 9 µL of sample was added to a Uni tube, and the temperature was set to gradually increase from 25 °C to 95 °C at a gradient of 0.3 °C/min. Four acquisitions of data were performed at the beginning and end of DLS assay, each for 5 s. After the experimental run was finished, the Tm value of each sample was calculated according to a barycentric mean (BCM) formula using the Uncle analysis software, the Tagg value was calculated from a curve (aggregation curve) of fluorescence intensity of SLS at the wavelength of 266 nm or 473 nm, and the particle size and dispersion of the samples were calculated from DLS related functions.

### Example 3. Construction of Bispecific Immune Cell Engager Binding Proteins Targeting Costimulatory Molecules

In this example, several bispecific immune cell engager binding protein molecules were listed, one end of which can bind to a costimulatory molecule on the surface of an immune cell (e.g., 4-1BB, OX40, etc.), and the other end of which can bind to a specific antigen molecule on the surface of a tumor cell (e.g., HER2, PSMA, EPCAM, CLDN18.2, B7H4, etc.) or a co-inhibitory molecule on the surface of an immune cell (e.g., PD-L1, etc.).

In this example, several bispecific immune cell engager binding protein molecules targeting 4-1BB and a tumor antigen (or a co-inhibitory molecule) were constructed, in which the 4-1BB antibody was redirected to tumor cells using the tumor antigen, and the immune response in a tumor microenvironment was specifically activated, so that side effects such as hepatotoxicity were avoided. In addition, 4-1BB was highly expressed on the surface of both CD8⁺ T cells and NK cells. The activation of 4-1BB can increase the infiltration of cytotoxic T cells to tumors, and can also enhance the activity of NK cells, thereby converting a "cold tumor" into a "hot tumor". In this example, bispecific binding protein molecules targeting both 4-1BB and tumor antigens (or co-inhibitory molecules) were constructed using an antigen-binding fragment VH of an HCAb antibody binding to 4-1BB (PR001758, PR001760, PR001836 or PR004469), and an antigen-binding fragment Fab of an IgG antibody binding to a tumor antigen HER2 (trastuzumab), or an antigen-binding fragment Fab of an IgG antibody binding to B7H4 (PR002408 or PR002410), or an antigen-binding fragment Fab of an IgG antibody binding to EPCAM (PR001081), or an antigen-binding fragment Fab of an IgG antibody binding to PSMA (PR001331), or an antigen-binding fragment Fab of an IgG antibody binding to CLDN18.2 (PR002726) or an antigen-binding fragment Fab of an IgG antibody binding to PD-L1 (PR000265).

In this example, several bispecific immune cell engager binding protein molecules targeting OX40 and a tumor antigen (or a co-inhibitory molecule) were constructed, in which the OX40 antibody was redirected to tumor cells using the tumor antigen, and the immune response in a tumor microenvironment was specifically activated. In this example, bispecific binding protein molecules targeting both OX40 and a tumor antigen (or a co-inhibitory molecule) were constructed using an antigen-binding fragment VH of an HCAb antibody binding to OX40 (PR002067), and an antigen-binding fragment Fab of an IgG antibody binding to tumor antigen EPCAM (PR001081), or an antigen-binding fragment Fab of an IgG antibody binding to PSMA (PR001331), or an antigen-binding fragment Fab of an IgG antibody binding to CLDN18.2 (PR002726), or an antigen-binding fragment Fab of an IgG antibody binding to B7H4 (PR002408) or an antigen-binding fragment Fab of an IgG antibody binding to PD-L1 (PR000265).

In this example, anti-4-1BB HCAb antibodies (PR001758, PR001760, PR001836 and PR004469) and anti-OX40 HCAb antibody (PR002067) were obtained by using Harbour HCAb transgenic mice (Harbour antibodies BV) to immunize related antigens, screening and optimizing. The anti-PD-L1 IgG antibody (PR000265), anti-EPCAM IgG antibody (PR001081), anti-PSMA IgG antibody (PR001331), anti-CLDN18.2 IgG antibody (PR002726), anti-B7H4 IgG antibody (PR002408 or PR002410), anti-4-1BB IgG antibody (PR000197 or PR000448) and the like were obtained by immunization of Harbour H2L2 transgenic mice (Harbour Antibodies BV) with related antigens, screening and optimization. The sequences of the anti-HER2 IgG antibody trastuzumab analog (antibody No. PR000210), the anti-PD-L1 IgG antibody atezolizumab (antibody No. PR000151), the anti-4-1BB IgG antibody utomilumab analog (antibody No. PR000483) and urelumab analog (antibody No. PR000628), the anti-OX40 IgG antibody pogalizumab analog (also known as vonlerolizumab, antibody No. PR003475) and the like were derived from existing literature or public databases. Those antibodies as parent antibodies or reference antibody molecules of the bispecific immune cell engager binding protein molecules constructed in this example are listed in Table 3, and the sequence numbers of their corresponding amino acid sequences are listed in Table 10.

In this example, each bispecific immune cell engager binding protein molecule had two specific antigen-binding fragments, which were a first antigen-binding fragment, from a targeting antibody binding to an antigen specific of a target cell (e.g., tumor cell), and a second antigen-binding fragment, from an activating antibody binding to an antigen specific of an immune effector cell, respectively. The bispecific binding protein molecules with different structures and the corresponding antigen-binding fragments and connection modes thereof are listed in Table 4, Table 5, Table 6, Table 7 and Table 8, and the structure numbers in the tables correspond to Table 1 and FIG. 1. The codes for the mutation sites in the tables are (AAG: L234A, L235A and P329G; LALA: L234A and L235A). The sequence numbers of the polypeptide chain amino acid sequences of the bispecific binding protein molecules are listed in Table 11, and the sequence numbers of the corresponding CDR sequences of the first and second antigen-binding fragments of the bispecific binding protein molecules are listed in Table 12.

In this example, a plurality of bispecific binding proteins with the structures as described in Example 1 were designed, and corresponding protein samples were prepared and analyzed according to the methods as described in Example 2, with the results summarized in Table 9.

**Table 3. Control molecules and parent monoclonal antibodies**

| **Molecule No.** | **Antibody** |
|---|---|
| PR000151 | Anti-PD-L1 monoclonal antibody atezolizumab analog |
| PR000265 | Anti-PD-L1 91G3H5H3(D54E), hIgG1(N297A) |
| PR000210 | Anti-HER2 monoclonal antibody trastuzumab analog |
| PR001081 | Anti-EPCAM monoclonal antibody 16A8B3 hIgG1 |
| PR001331 | Anti-PSMA monoclonal antibody 2E8A1B1 hIgG1 |
| PR002408 | Anti-B7H4 monoclonal antibody 80C8-2E9 (H: G55A; L: N92Q) hIgG1 |
| PR002410 | Anti-B7H4 monoclonal antibody B-1H11 (L: C87Y) hIgG I |
| PR002726 | Anti-CLDN18.2 monoclonal antibody 13E6F4 hIgG1 |
| PR000628 | Anti-4-1BB monoclonal antibody urelumab analog |
| PR000483 | Anti-4-1BB monoclonal antibody utomilumab analog |
| PR003475 | Anti-OX40 monoclonal antibody vonlerolizumab analog |
| PR000197 | Anti-4-1BB monoclonal antibody 79B10G8D4, hIgG4 |
| PR000448 | Anti-4-1BB monoclonal antibody 79B10G8D4 (H: N52Q; L: F2I) hIgG4 |
| PR001758 | Anti-4-1BB heavy-chain antibody 1016P0010B11 |
| PR001760 | Anti-4-1BB heavy-chain antibody 1016P0011G10 |
| PR001836 | Anti-4-1BB heavy-chain antibody 1016P0020G4 |
| PR004469 | Anti-4-1BB heavy-chain antibody PR001838_G53A |
| PR002067 | Anti-OX40 heavy-chain antibody R1026P079E12 |

**Table 4. Bispecific antibody molecules with an IgG-VH or VH-IgG tetravalent symmetric structure**

| **Structur e No.** | **Bispecific antibody molecule** | **Targeting antigen** | **Targeting antibody** | **Immune cell antigen** | **Activating antibody** | **Linker peptide** | **Fc type (mutation)** |
|---|---|---|---|---|---|---|---|
| 1 | PR002827 | HER2 | PR000210 | 4-1BB | PR001836 | H1_15-RT | Human IgG1 (AAG) |
| 1 | PR003334 | B7H4 | PR002408 | 4-1BB | PR001758 | H1_15-RT | Human IgG1 (LALA) |
| 1 | PR003335 | B7H4 | PR002408 | 4-1BB | PR001760 | H1_15-RT | Human IgG1 (LALA) |
| 1 | PR003338 | B7H4 | PR002408 | 4-1BB | PR004469 | H1_15-RT | Human IgG1 (LALA) |
| 1 | PR003549 | PD-L1 | PR000265 | 4-1BB | PR001758 | H1_15-RT | Human IgG1 (AAG) |
| 1 | PR003550 | PD-L1 | PR000265 | 4-1BB | PR001760 | H1_15-RT | Human IgG1 (AAG) |
| 1 | PR003551 | PD-L1 | PR000265 | 4-1BB | PR001836 | H1_15-RT | Human IgG1 (AAG) |
| 1 | PR003552 | EPCAM | PR001081 | 4-1BB | PR001758 | H1_15-RT | Human IgG1 (AAG) |
| 1 | PR003553 | EPCAM | PR001081 | 4-1BB | PR001760 | H1_15-RT | Human IgG1 (AAG) |
| 1 | PR003554 | EPCAM | PR001081 | 4-1BB | PR001836 | H1_15-RT | Human IgG1 (AAG) |
| 1 | PR003555 | PSMA | PR001331 | 4-1BB | PR001758 | H1_15-RT | Human IgG1 (AAG) |
| 1 | PR003556 | PSMA | PR001331 | 4-1BB | PR001760 | H1_15-RT | Human IgG1 (AAG) |
| 1 | PR003557 | PSMA | PR001331 | 4-1BB | PR001836 | H1_15-RT | Human IgG1 (AAG) |
| 1 | PR003562 | CLDN18. 2 | PR002726 | 4-1BB | PR001758 | H1_15-RT | Human IgG1 (AAG) |
| 1 | PR003563 | CLDN18. 2 | PR002726 | 4-1BB | PR001760 | H1_15-RT | Human IgG1 (AAG) |
| 1 | PR003564 | CLDN18. 2 | PR002726 | 4-1BB | PR001836 | H1_15-RT | Human IgG1 (AAG) |
| 1 | PR003789 | PD-L1 | PR000265 | OX40 | PR002067 | H1_15 | Human IgG1 (AAG) |
| 1 | PR004276 | B7H4 | PR002408 | OX40 | PR002067 | H1_15-RT | Human IgG1 (LALA) |
| 1 | PR004283 | EPCAM | PR001081 | OX40 | PR002067 | H1_15-RT | Human IgG1 (LALA) |
| 1 | PR004284 | PSMA | PR001331 | OX40 | PR002067 | H1_15-RT | Human IgG1 (LALA) |
| 1 | PR004285 | CLDN18. 2 | PR002726 | OX40 | PR002067 | H1_15-RT | Human IgG1 (LALA) |
| 7 | PR004278 | B7H4 | PR002408 | 4-1BB | PR001760 | GS_15 | Human IgG1 (LALA) |

**Table 5. Bispecific antibody molecules with an IgG-VH(2) hexavalent symmetric structure**

| **Structur e No.** | **Bispecifi c antibody molecule** | **Targetin g antigen** | **Targetin g antibody** | **Immune cell antigen** | **Activatin g antibody** | **First linker peptide** | **Second linker peptide** | **Fc type (mutation )** |
|---|---|---|---|---|---|---|---|---|
| 2 | PR00348 7 | B7H4 | PR00240 8 | 4-1BB | PR00176 0 | H1_15-R T | None | Human IgG1 (LALA) |
| 2 | PR00415 8 | B7H4 | PR00241 0 | 4-1BB | PR00176 0 | H1_15-R T | GS_5 | Human IgG1 (LALA) |

**Table 6. Bispecific antibody molecules with a Fab-HCAb tetravalent symmetric structure**

| **Structur e No.** | **Bispecifi c antibody molecule** | **Targetin g antigen** | **Targetin g antibody** | **Immun e cell antigen** | **Activatin g antibody** | **First linker peptide** | **Second linker peptide** | **Fc type (mutation)** |
|---|---|---|---|---|---|---|---|---|
| 3 | PR00427 0 | PD-L1 | PR00026 5 | 4-1BB | PR00176 0 | H1_15 | Human IgG1 hinge (C220S) | Human IgG1 (LALA) |
| 3 | PR00427 9 | B7H4 | PR00240 8 | 4-1BB | PR00176 0 | H1_15 | Human IgG1 hinge (C220S) | Human IgG1 (LALA) |
| 3 | PR00427 7 | B7H4 | PR00240 8 | OX40 | PR00206 7 | H1_15 | Human IgG1 hinge (C220S) | Human IgG1 (LALA) |
| 3 | PR00518 9 | B7H4 | PR00240 8 | 4-1BB | PR00176 0 | None | Human IgG1 hinge (C220S) | Human IgG1 (LALA) |
| 3 | PR00564 9 | B7H4 | PR00240 8 | 4-1BB | PR00176 0 | H1_15 | Human IgG2 hinge | Human IgG1 (LALA) |
| 4 | PR00716 3 | PD-L1 | PR00026 5 | 4-1BB | PR00176 0 | None | Human IgG1 hinge (C220S) | Human IgG1 (LALA) |
| 3 | PR00716 4 | PD-L1 | PR00026 5 | 4-1BB | PR00176 0 | None | Human IgG1 hinge (C220S) | Human IgG1 (LALA) |
| 4 | PR00716 5 | B7H4 | PR00240 8 | 4-1BB | PR00176 0 | None | Human IgG1 hinge (C220S) | Human IgG1 (LALA) |
| 4 | PR00716 6 | B7H4 | PR00240 8 | OX40 | PR00206 7 | None | Human IgG1 hinge (C220S) | Human IgG1 (LALA) |
| 3 | PR00716 7 | B7H4 | PR00240 8 | OX40 | PR00206 7 | None | Human IgG1 hinge (C220S) | Human IgG1 (LALA) |

**Table 7. B7H4×4-1BB bispecific antibody molecules with a Fab-VH-Fc-VH hexavalent symmetric structure**

| **Structur e No.** | **Bispecific antibody molecule** | **B7H4 antibody** | **4-1BB antibody** | **First linker peptide** | **Second linker peptide** | **Third linker peptide** | **Fc type (mutation )** |
|---|---|---|---|---|---|---|---|
| 5 | PR005650 | PR002408 | PR001760 | None | Human IgG1 hinge (C220S) | None | Human IgG1 (LALA) |
| 5 | PR005651 | PR002408 | PR004469 | None | Human IgG1 hinge (C220S) | None | Human IgG1 (LALA) |

**Table 8. Bispecific antibody molecules with an FIT-Ig symmetric structure**

| **Struct ure No.** | **Bispecific antibody molecule** | **Targetin g antigen** | **Targetin g antibody** | **Immune cell antigen** | **Activatin g antibody** | **Description of structures** | **Fc type (mutation )** |
|---|---|---|---|---|---|---|---|
| 8 | PR000701 | PD-L1 | PR00026 5 | 4-1BB | PR000197 | 4-1BB Fab close to Fc; no linker peptide | Human IgG4 (S228P) |
| 8 | PR003052 | PD-L1 | PR00015 1 | 4-1BB | PR000448 | 4-1BB Fab close to Fc; no linker peptide | Human IgG4 (S228P) |

**Table 9. Expression of bispecific antibody molecule proteins**

| **Structure No.** | **Bispecific antibody molecule** | **Expression system and volume** | **Yield (mg/L) after first purification** | **SEC-HPLC purity (%)** |
|---|---|---|---|---|
| 1 | PR002827 | Expi293F (10ml) | 29.5 | 73.75 |
| 1 | PR003334 | ExpiCHO (200ml) | 38.2 | 97.98 |
| 1 | PR003335 | ExpiCHO (200ml) | 68.9 | 99.29 |
| 1 | PR003338 | HEK293-F (10ml) | 74.6 | 98.02 |
| 2 | PR003487 | HEK293-F (30ml) | 19 | 84 |
| 1 | PR003549 | HEK293-F (30ml) | 24.5 | 98.31 |
| 1 | PR003550 | HEK293-F (30ml) | 42.2 | 95.41 |
| 1 | PR003551 | HEK293-F (30ml) | 2.3 | 99.39 |
| 1 | PR003552 | HEK293-6E (40ml) | 44 | 89.71 |
| 1 | PR003553 | HEK293-6E (40ml) | 44.8 | 88.77 |
| 1 | PR003554 | HEK293-6E (40ml) | 6.5 | 79.47 |
| 1 | PR003555 | HEK293-6E (40ml) | 52.8 | 98.73 |
| 1 | PR003556 | HEK293-6E (40ml) | 67.8 | 98.42 |
| 1 | PR003557 | HEK293-6E (40ml) | 9 | 97.73 |
| 1 | PR003562 | HEK293-F (30ml) | 173 | 97.87 |
| 1 | PR003563 | HEK293-F (30ml) | 142.7 | 95.63 |
| 1 | PR003564 | HEK293-F (30ml) | 20 | 93.72 |
| 1 | PR003789 | HEK293-6E (40ml) | 3.3 | 96.08 |
| 1 | PR004276 | HEK293-6E (40ml) | 17.7 | 95.46 |
| 1 | PR004283 | HEK293-6E (40ml) | 27.5 | 95.42 |
| 1 | PR004284 | HEK293-6E (40ml) | 21 | 99.43 |
| 1 | PR004285 | HEK293-6E (40ml) | 60.8 | 98.15 |
| 2 | PR004158 | HEK293-F (30ml) | 31.9 | 63.09 |
| 3 | PR004270 | HEK293-6E (40ml) | 77.5 | 92.33 |
| 3 | PR004279 | HEK293-F (30ml) | 11.4 | 79.18 |
| 3 | PR004279 | CHO (100ml) | 3.6 | 95.46 |
| 3 | PR004277 | CHO (100ml) | 48 | 93.03 |
| 5 | PR005650 | HEK293 (100ml) | 21.5 | 97.48 |
| 5 | PR005651 | HEK293 (100ml) | 18.2 | 98.13 |
| 7 | PR004278 | HEK293-6E (40ml) | 11.5 | 98.08 |
| 8 | PR003052 | HEK293-6E (40ml) | 47.5 | 87.76 |

**Table 10. Sequence numbers of sequences and CDR sequences of control molecules and parent monoclonal antibodies**

| **Molecule No.** | **Light chain** | **Heavy chain** | **VL** | **VH** | **LCDR 1** | **LCDR 2** | **LCDR 3** | **HCD R1** | **HCD R2** | **HCD R3** |
|---|---|---|---|---|---|---|---|---|---|---|
| PR000151 | 199 | 181 | 168 | 150 | 103 | 117 | 132 | 11 | 40 | 75 |
| PR000265 | 202 | 184 | 171 | 153 | 106 | 119 | 135 | 14 | 43 | 78 |
| PR000210 | 201 | 183 | 170 | 152 | 105 | 117 | 134 | 13 | 42 | 77 |
| PR001081 | 206 | 188 | 175 | 157 | 108 | 121 | 138 | 16 | 47 | 81 |
| PR001331 | 207 | 189 | 176 | 158 | 109 | 122 | 139 | 17 | 48 | 82 |
| PR002408 | 208 | 194 | 177 | 163 | 110 | 118 | 140 | 20 | 53 | 86 |
| PR002410 | 209 | 195 | 178 | 164 | 111 | 118 | 141 | 21 | 54 | 87 |
| PR002726 | 210 | 196 | 179 | 165 | 108 | 121 | 142 | 22 | 55 | 88 |
| PR000628 | 205 | 187 | 174 | 156 | 108 | 121 | 137 | 12 | 46 | 80 |
| PR000483 | 204 | 186 | 173 | 155 | 107 | 120 | 136 | 15 | 45 | 79 |
| PR003475 | 211 | 197 | 180 | 166 | 112 | 123 | 143 | 23 | 56 | 89 |
| PR000197 | 200 | 182 | 169 | 151 | 104 | 118 | 133 | 12 | 41 | 76 |
| PR000448 | 203 | 185 | 172 | 154 | 104 | 118 | 133 | 12 | 44 | 76 |
| PR001758 | | 190 | | 159 | | | | 16 | 49 | 83 |
| PR001760 | | 191 | | 160 | | | | 18 | 50 | 84 |
| PR001836 | | 192 | | 161 | | | | 19 | 51 | 84 |
| PR004469 | | 198 | | 167 | | | | 18 | 57 | 90 |
| PR002067 | | 193 | | 162 | | | | 14 | 52 | 85 |

**Table 11. Sequence numbers of polypeptide chains of bispecific antibody molecules constructed in this example**

| **Structure No.** | **Molecule No.** | **Polypeptide chain 1** | **Polypeptide chain 2** | **Polypeptide chain 3** |
|---|---|---|---|---|
| 1 | PR002827 | 201 | 214 | |
| 1 | PR003334 | 208 | 217 | |
| 1 | PR003335 | 208 | 218 | |
| 1 | PR003338 | 208 | 219 | |
| 2 | PR003487 | 208 | 220 | |
| 1 | PR003549 | 202 | 221 | |
| 1 | PR003550 | 202 | 222 | |
| 1 | PR003551 | 202 | 223 | |
| 1 | PR003552 | 206 | 224 | |
| 1 | PR003553 | 206 | 225 | |
| 1 | PR003554 | 206 | 226 | |
| 1 | PR003555 | 207 | 227 | |
| 1 | PR003556 | 207 | 228 | |
| 1 | PR003557 | 207 | 229 | |
| 1 | PR003562 | 210 | 230 | |
| 1 | PR003563 | 210 | 231 | |
| 1 | PR003564 | 210 | 232 | |
| 1 | PR003789 | 202 | 233 | |
| 2 | PR004158 | 209 | 234 | |
| 3 | PR004270 | 212 | 235 | |
| 1 | PR004276 | 208 | 236 | |
| 3 | PR004277 | 237 | 238 | |
| 7 | PR004278 | 208 | 239 | |
| 3 | PR004279 | 237 | 240 | |
| 1 | PR004283 | 206 | 241 | |
| 1 | PR004284 | 207 | 242 | |
| 1 | PR004285 | 210 | 243 | |
| 8 | PR000701 | 213 | 212 | 203 |
| 8 | PR003052 | 216 | 215 | 203 |
| 3 | PR005189 | 237 | 266 | |
| 3 | PR005649 | 237 | 267 | |
| 5 | PR005650 | 237 | 268 | |
| 5 | PR005651 | 237 | 269 | |
| 4 | PR007163 | 202 | 270 | |
| 3 | PR007164 | 212 | 271 | |
| 4 | PR007165 | 208 | 272 | |
| 4 | PR007166 | 208 | 273 | |
| 3 | PR007167 | 237 | 274 | |

**Table 12. Sequence numbers of CDRs of antigen-binding fragments of bispecific antibody molecules**

| **Molecule No.** | **Antigen-bindin g domain No.** | **LCDR1** | **LCDR2** | **LCDR3** | **HCDR1** | **HCDR2** | **HCDR3** |
|---|---|---|---|---|---|---|---|
| PR002827 | #1(HER2) | 105 | 117 | 134 | 13 | 42 | 77 |
| | #2(4-1BB) | | | | 19 | 51 | 84 |
| PR003334 | #1(B7H4) | 110 | 118 | 140 | 20 | 53 | 86 |
| | #2(4-1BB) | | | | 16 | 49 | 83 |
| PR003335, PR004278, PR003487, PR004279, PR005189, PR005649, PR005650, PR007165 | #1(B7H4) | 110 | 118 | 140 | 20 | 53 | 86 |
| | #2(4-1BB) | | | | 18 | 50 | 84 |
| PR003338, PR005651 | #1(B7H4) | 110 | 118 | 140 | 20 | 53 | 86 |
| | #2(4-1BB) | | | | 18 | 57 | 90 |
| PR003549 | #1(PD-L1) | 106 | 119 | 135 | 14 | 43 | 78 |
| | #2(4-1BB) | | | | 16 | 49 | 83 |
| PR003550, PR004270, PR007163, PR007164 | #1(PD-L1) | 106 | 119 | 135 | 14 | 43 | 78 |
| | #2(4-1BB) | | | | 18 | 50 | 84 |
| PR003551 | #1(PD-L1) | 106 | 119 | 135 | 14 | 43 | 78 |
| | #2(4-1BB) | | | | 19 | 51 | 84 |
| PR003552 | #1(EPCAM) | 108 | 121 | 138 | 16 | 47 | 81 |
| | #2(4-1BB) | | | | 16 | 49 | 83 |
| PR003553 | #1(EPCAM) | 108 | 121 | 138 | 16 | 47 | 81 |
| | #2(4-1BB) | | | | 18 | 50 | 84 |
| PR003554 | #1(EPCAM) | 108 | 121 | 138 | 16 | 47 | 81 |
| | #2(4-1BB) | | | | 19 | 51 | 84 |
| PR003555 | #1(PSMA) | 109 | 122 | 139 | 17 | 48 | 82 |
| | #2(4-1BB) | | | | 16 | 49 | 83 |
| PR003556 | #1(PSMA) | 109 | 122 | 139 | 17 | 48 | 82 |
| | #2(4-1BB) | | | | 18 | 50 | 84 |
| PR003557 | #1(PSMA) | 109 | 122 | 139 | 17 | 48 | 82 |
| | #2(4-1BB) | | | | 19 | 51 | 84 |
| PR003562 | #1(CLDN18.2) | 108 | 121 | 142 | 22 | 55 | 88 |
| | #2(4-1BB) | | | | 16 | 49 | 83 |
| PR003563 | #1(CLDN18.2) | 108 | 121 | 142 | 22 | 55 | 88 |
| | #2(4-1BB) | | | | 18 | 50 | 84 |
| PR003564 | #1(CLDN18.2) | 108 | 121 | 142 | 22 | 55 | 88 |
| | #2(4-1BB) | | | | 19 | 51 | 84 |
| PR003789 | #1(PD-L1) | 106 | 119 | 135 | 14 | 43 | 78 |
| | #2(OX40) | | | | 14 | 52 | 85 |
| PR004276, PR004277, PR007166, PR007167 | #1(B7H4) | 110 | 118 | 140 | 20 | 53 | 86 |
| | #2(OX40) | | | | 14 | 52 | 85 |
| PR004283 | #1(EPCAM) | 108 | 121 | 138 | 16 | 47 | 81 |
| | #2(OX40) | | | | 14 | 52 | 85 |
| PR004284 | #1(PSMA) | 109 | 122 | 139 | 17 | 48 | 82 |
| | #2(OX40) | | | | 14 | 52 | 85 |
| PR004285 | #1(CLDN18.2) | 108 | 121 | 142 | 22 | 55 | 88 |
| | #2(OX40) | | | | 14 | 52 | 85 |
| PR004158 | #1(B7H4) | 111 | 118 | 141 | 21 | 54 | 87 |
| | #2(4-1BB) | | | | 18 | 50 | 84 |
| PR000701 | #1(PD-L1) | 106 | 119 | 135 | 14 | 43 | 78 |
| | #2(4-1BB) | 104 | 118 | 133 | 12 | 41 | 76 |
| PR003052 | #1(PD-L1) | 103 | 117 | 132 | 11 | 40 | 75 |
| | #2(4-1BB) | 104 | 118 | 133 | 12 | 44 | 76 |

### Example 4. Binding to 4-1BB

This example is intended to investigate the binding activity of the antibodies targeting 4-1BB and the bispecific immune cell engager binding proteins to 4-1BB.

The binding ability of the binding protein to a CHO-K1 cell line CHO-K1/hu 4-1BB (GenScript, M00538) highly expressing human 4-1BB and a CHO-K1 cell line CHO-K1/cyno 4-1BB (GenScript, M00569) highly expressing cynomolgus monkey 4-1BB were determined by flow cytometry FACS. Specifically, the cells were digested and resuspended in a complete medium, and the cell density was adjusted to 2×10⁶ cells/mL. Thereafter, the cells were seeded in a 96-well V-bottom plate (Corning, #3894) at 100 µL/well (2×10⁵ cells/well) and centrifuged at 4 °C for 5 min, and the supernatant was discarded. Then, the binding proteins diluted in a gradient were added to the 96-well plate at 100 µL/well, and the mixture was mixed well, wherein the binding proteins may have a total of 12 concentrations obtained by a 3-fold gradient dilution from the highest final concentration of 200 nM. hIgG1 iso (CrownBio, #C0001) was used as an isotype control. The cells were incubated at 4 °C for 1 h away from light. Then, the cells in each well were rinsed twice with 100 µL of pre-cooled FACS buffer (PBS buffer containing 0.5% BSA) and centrifuged at 500 g at 4 °C for 5 min, and then the supernatant was discarded. Thereafter, a fluorescent secondary antibody (Goat human IgG (H+L) Alexa Fluor 488 conjugation, Thermo, #A11013, diluted in a 1:1000 ratio) was added at 100 µL/well, and the plate was incubated at 4 °C for 1 h away from light. Then, the cells in each well were then rinsed twice with 200 µL of pre-cooled FACS buffer and centrifuged at 500 g at 4 °C for 5 min, and then the supernatant was discarded. Finally, a pre-cooled FACS buffer was added at 200 µL/well to resuspend the cells. Fluorescence signal values were read using a BD FACS CANTOII flow cytometer or an ACEA NovoCyte flow cytometer, and the data were processed and analyzed using FlowJo v10 (FlowJo, LLC) software.

The data were processed and analyzed by plotting using GraphPad Prism 8 software, and binding curves of the antibodies to target cells, EC₅₀ values and other parameters were obtained through four-parameter nonlinear fitting.

In this example, the anti-4-1BB monoclonal antibody urelumab or utomilumab was used as a positive control molecule.

### Example 4.1 Binding to CHO-K1/hu 4-1BB highly expressing human 4-1BB

### Example 4.1.1 Binding of PD-L1×4-1BB to CHO-K1/hu 4-1BB

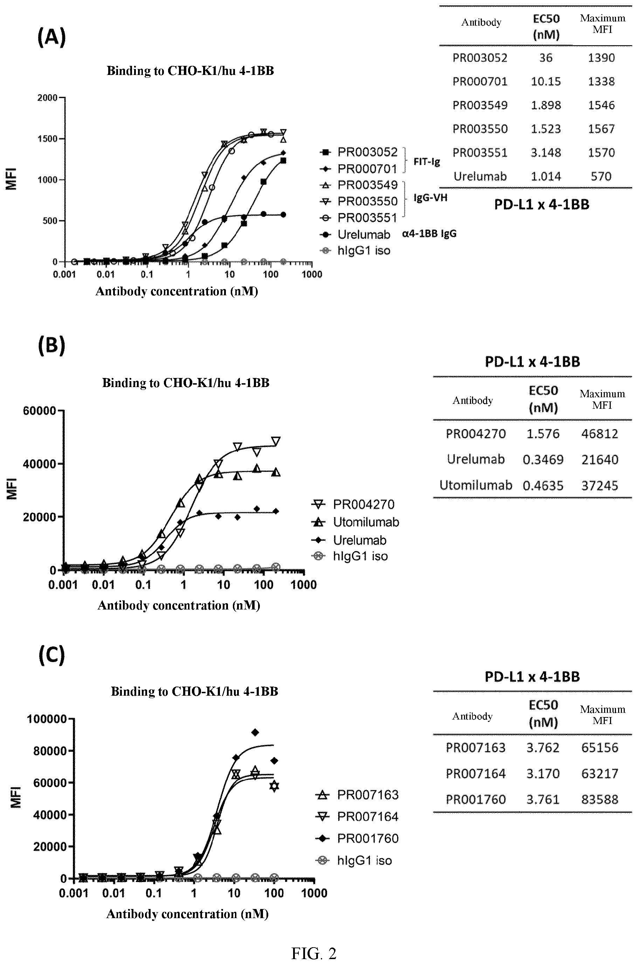

As shown in FIG. 2, the PD-L1×4-1BB bispecific antibody molecules were all able to bind to human 4-1BB cells CHO-K1/hu 4-1BB.

As shown in FIG. 2 (A), the PD-L1×4-1BB bispecific antibody molecules with the IgG-VH tetravalent symmetric structure (PR003549, PR003550 and PR003551) had superior binding ability to human 4-1BB to that of the bispecific antibody molecules with the FIT-Ig structure (PR000701 and PR003052), and were superior to the positive control urelumab in terms of maximum MFIs.

As shown in FIG. 2 (B), the PD-L1×4-1BB bispecific antibody molecule with the Fab-HCAb symmetric structure (PR004270) had superior binding ability to human 4-1BB to that of the positive controls urelumab and utomilumab in terms of maximum MFIs.

As shown in FIG. 2 (C), the PD-L1×4-1BB bispecific antibody molecules with the Fab-HCAb symmetric structure, whether the molecule with the structure (3) as described in Example 1.1.3 (PR007164) or the molecule with the structure (4) as described in Example 1.1.4 (PR007163), had almost identical binding activity to 4-1BB, and were comparable to the parent monoclonal antibody PR001760.

### Example 4.1.2 Binding of B7H4×4-1BB to CHO-K1/hu 4-1BB

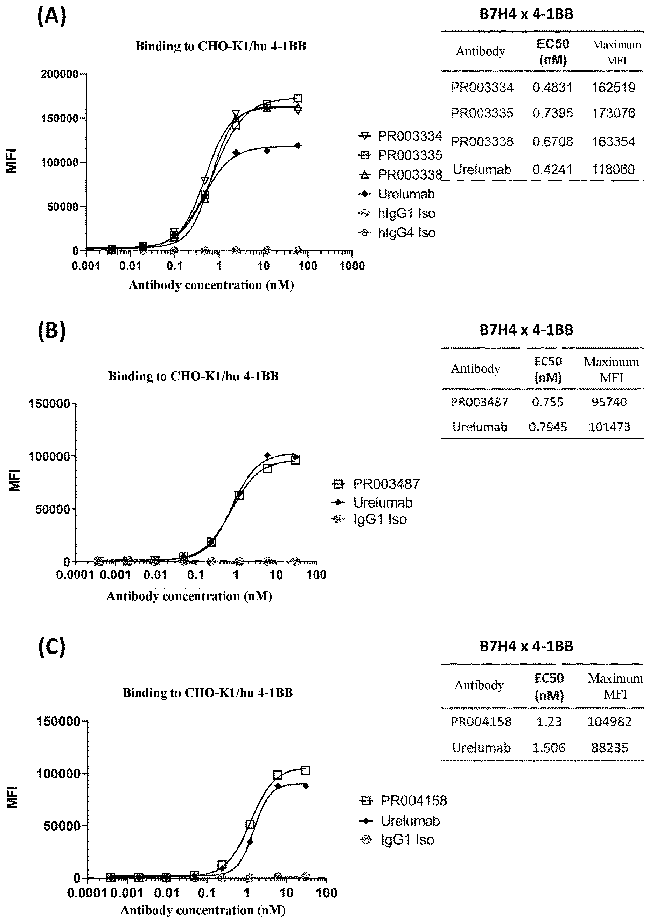

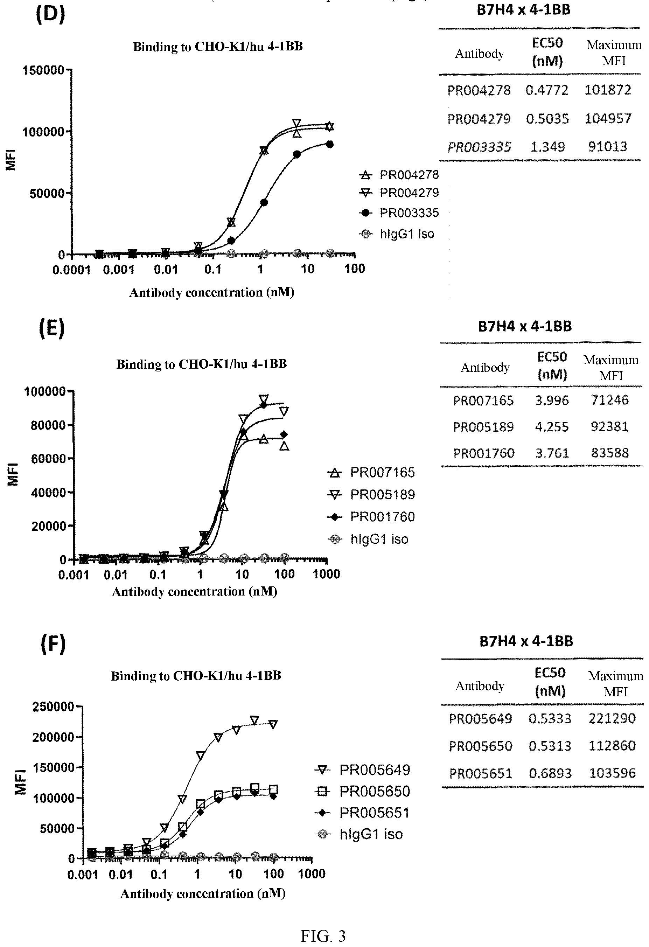

As shown in FIG. 3, the B7H4×4-1BB bispecific antibody molecules were all able to bind to human 4-1BB cells CHO-K1/hu 4-1BB.

As shown in FIG. 3 (A), the B7H4×4-1BB bispecific antibody molecules with the IgG-VH tetravalent symmetric structure (PR003334, PR003335 and PR003338) were all able to bind to CHO-K1/hu 4-1BB, and were superior to urelumab in terms of maximum MFIs.

As shown in FIG. 3 (B-C), the B7H4×4-1BB bispecific antibody molecules with the IgG-VH(2) hexavalent symmetric structure (PR003487 and PR004158) were all able to bind to CHO-K1/hu 4-1BB.

As shown in FIG. 3 (D), the B7H4×4-1BB with the Fab-HCAb symmetric structure (PR004279) and the molecule with the VH-IgG structure (PR004278) had superior binding activity to 4-1BB to that of the molecule with the IgG-VH structure (PR003335).

As shown in FIG. 3 (E), the B7H4×4-1BB bispecific antibody molecules with the Fab-HCAb symmetric structure, whether the molecule with the structure (3) as described in Example 1.1.3 (PR005189) or the molecule with the structure (4) as described in Example 1.1.4 (PR007165), had similar binding activity to 4-1BB, and were comparable to the parent monoclonal antibody PR001760.

As shown in FIG. 3 (F), the bispecific antibody molecule with the Fab-HCAb tetravalent symmetric structure (PR005649) and the bispecific antibody molecules with the Fab-VH-Fc-VH hexavalent symmetric structure (PR005650 and PR005651) were all able to bind to CHO-K1/hu 4-1BB, but the bispecific antibody molecule with the Fab-HCAb structure had higher MFI.

### Example 4.1.3 Binding of HER2×4-1BB to CHO-K1/hu 4-1BB

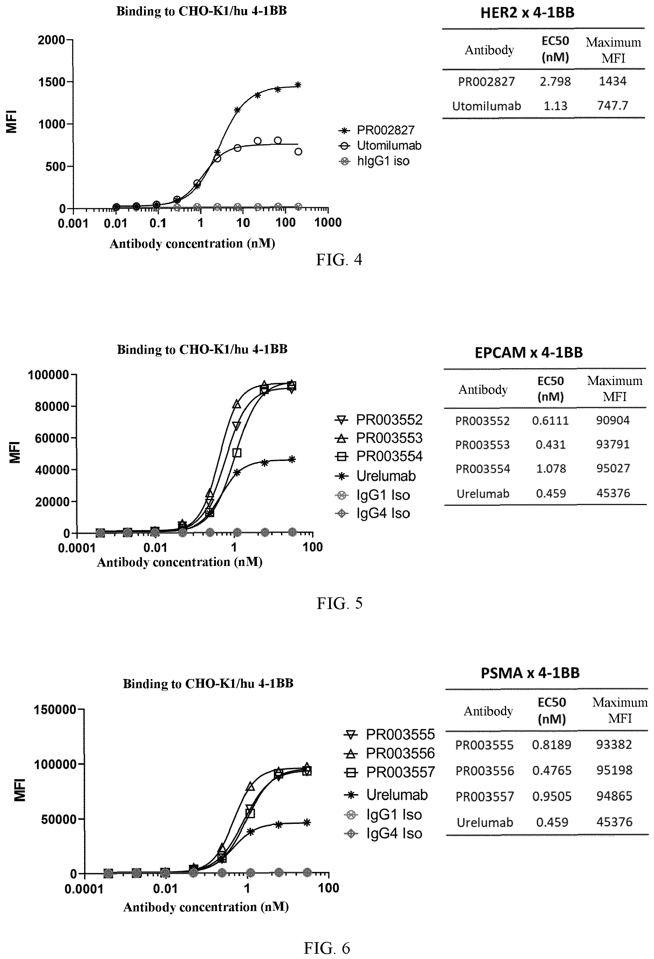

As shown in FIG. 4, the HER2×4-1BB bispecific antibody molecule (PR002827) was able to bind to human 4-1BB cells CHO-K1/hu 4-1BB.

### Example 4.1.4 Binding of EPCAM×4-1BB to CHO-K1/hu 4-1BB

As shown in FIG. 5, the EPCAM×4-1BB bispecific antibody molecules (PR003552, PR003553 and PR003554) were all able to bind to human 4-1BB cells CHO-K1/hu 4-1BB.

### Example 4.1.5 Binding of PSMA×4-1BB to CHO-K1/hu 4-1BB

As shown in FIG. 6, the PSMA×4-1BB bispecific antibody molecules (PR003555, PR003556 and PR003557) were all able to bind to human 4-1BB cells CHO-K1/hu 4-1BB.

### Example 4.1.6 Binding of CLDN18.2×4-1BB to CHO-K1/hu 4-1BB

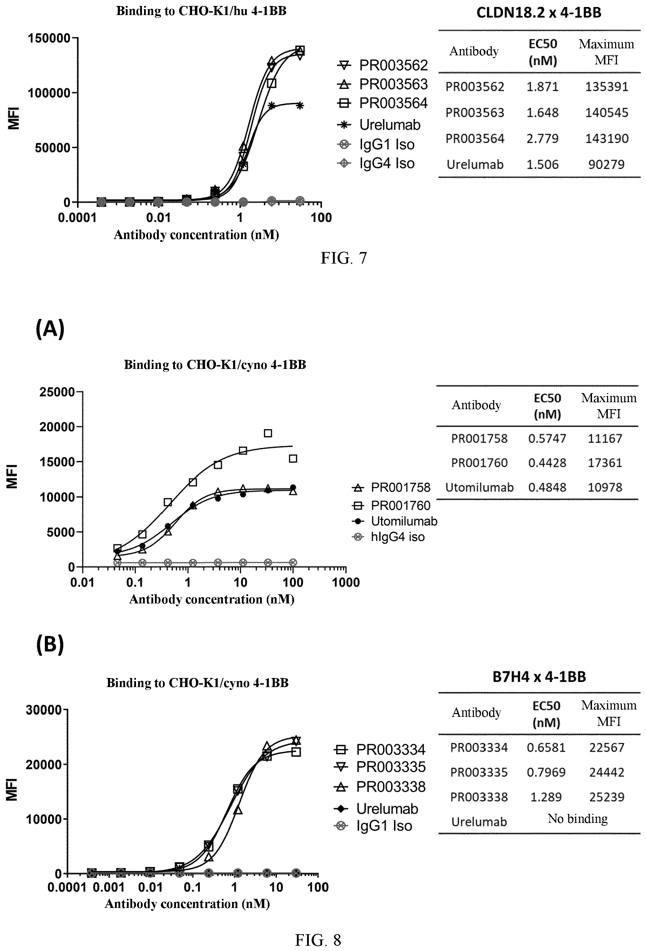

As shown in FIG. 7, the CLDN18.2×4-1BB bispecific antibody molecules (PR003562, PR003563 and PR003564) were all able to bind to human 4-1BB cells CHO-K1/hu 4-1BB.

### Example 4.2 Binding to CHO-K1/cyno 4-1BB highly expressing cynomolgus monkey 4-1BB

As shown in FIG. 8, the anti-4-1BB HCAb antibodies (PR001758 and PR001760) and the B7H4×4-1BB bispecific antibody molecules (PR003334, PR003335 and PR003338) were all able to bind to cynomolgus monkey 4-1BB cells CHO-K1/cyno 4-1BB. However, urelumab was unable to bind to cynomolgus monkey 4-1BB.

### Example 5. Binding to OX40

This example is intended to investigate the binding activity of the antibodies targeting OX40 and the bispecific immune cell engager binding proteins to OX40.

### Example 5.1 Binding to cynomolgus monkey OX40 protein

The binding ability of the binding proteins to cynomolgus monkey OX40 recombinant protein was determined by enzyme-linked immunosorbent assay (ELISA). Specifically, a 96-well plate was firstly coated with 2 µg/mL cynomolgus monkey OX40-His protein (NovoProtein, #CB17) at 100 µL/well and incubated at 4 °C overnight. The plate was then rinsed 3 times with a PBST buffer (a PBS buffer containing 0.05% Tween-20), added with a blocking buffer (a PBS buffer containing 5% skim milk powder), and incubated at 37 °C for 1 h. The plate was then rinsed 3 times with a PBST buffer. Then, binding proteins at a total of 8 concentrations obtained by a 3-fold gradient dilution were added at 100 µL/well, and the mixture was mixed well and incubated at 37 °C for 1 h. hIgG1 iso (CrownBio, #C0001) was used as an isotype control. The plate was then rinsed 3 times with a PBST buffer. Then, the HRP-labeled goat anti-human IgG (H+L) antibody (Invitrogen, A18805, diluted in a 1:4000 ratio) was added at 100 µL/well, and the mixture was incubated at 37 °C for 0.5 h. The plate was then rinsed 3 times with a PBST buffer. Then, TMB chromogenic solution was added at 100 µL/well for reaction for 15 min. Finally, a stop buffer was added to stop the reaction. The absorbance value (OD value) was read at 450 nM using an Enspire^{™} multifunctional microplate reader (Perkin Elmer, Inc.).

The data were processed and analyzed by plotting using GraphPad Prism 8 software, and binding curves of the antibodies to target cells, EC₅₀ values and other parameters were obtained through four-parameter nonlinear fitting.

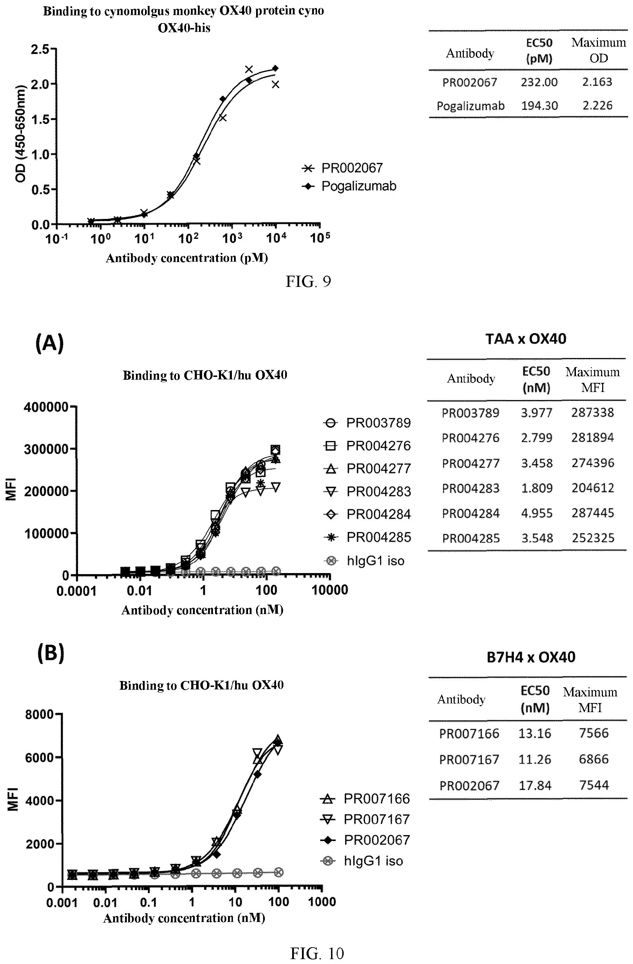

As shown in FIG. 9, the anti-OX40 HCAb antibody PR002067 was able to bind to cynomolgus monkey OX40, and had similar binding ability to that of the positive control pogalizumab.

### Example 5.2 Binding to CHO-K1/hu OX40 highly expressing human OX40

The antibody binding assay at the cellular level was performed using a CHO-K1 cell line CHO-K1/hu OX40 (GenScript, #M00561) highly expressing human OX40. Briefly, the CHO-K1/hu OX40 cells were digested and resuspended in an F12K complete medium, and the cell density was adjusted to 1×10⁶ cells/mL. The cells were seeded in a 96-well V-bottom plate (Corning, #3894) at 100 µL/well, followed by the addition of test antibodies diluted in a 3-fold gradient at concentrations that were 2 times the final concentrations at 100 µL/well. The cells were incubated at 4 °C for 1 h away from light. Thereafter, the cells in each well were rinsed twice with 100 µL of pre-cooled PBS, and centrifuged at 500 g at 4 °C for 5 min, and then the supernatant was discarded. Then, 100 µL of fluorescent secondary antibody (Alexa Fluor 488-conjugated AffiniPure Goat Anti-Human IgG, Fcy Fragment Specific, Jackson ImmunoResearch, #109-545-06, diluted in a 1:1000 ratio) was added to each well. The plate was incubated away from light at 4 °C for 30 min. The cells in each well were rinsed twice with 100 µL of pre-cooled PBS, and centrifuged at 500 g at 4 °C for 5 min, and then the supernatant was discarded. Finally, a pre-cooled PBS was added at 200 µL/well to resuspend the cells. Fluorescence signal values were read using a BD FACS CANTOII flow cytometer or an ACEA NovoCyte flow cytometer, and the data were processed and analyzed using FlowJo v10 (FlowJo, LLC) software.

The data were processed and analyzed by plotting using GraphPad Prism 8 software, and binding curves of the antibodies to target cells, EC₅₀ values and other parameters were obtained through four-parameter nonlinear fitting.

As shown in FIG. 10, the bispecific antibody molecules targeting OX40 were all able to bind to human OX40. Moreover, the B7H4×OX40 bispecific antibody molecules with the Fab-HCAb structure (PR007166 and PR007167) had comparable binding ability to OX40 to the parent monoclonal antibody PR002067.

### Example 6. Binding to Tumor Antigens

This example is intended to investigate the binding activity of the bispecific immune cell engager binding proteins to tumor antigens. In this example, the tumor antigen molecules may be HER2, PSMA, EPCAM, CLDN18.2, B7H4, etc., or co-inhibitory molecules on the surface of immune cells, e.g., PD-L1, etc.

In this example, the binding ability of the binding proteins to cells highly expressing specific tumor antigens was determined by flow cytometry FACS. Briefly, a cell suspension was collected, and the cell density was adjusted to 2×10⁶ cells/mL. Then, the cells were seeded in a 96-well V-bottom plate (Corning, #3894) at 50 µL/well. Then, binding proteins diluted in a 3-fold gradient at a total of 8 concentrations that were 2 times the final concentrations were added at 50 µL/well, and the mixture was mixed well. hIgG1 iso (CrownBio, #C0001) was used as an isotype control. The cells were incubated at 4 °C for 2 h away from light. Then, the cells in each well were then rinsed twice with 100 µL of pre-cooled PBS buffer and centrifuged at 500 g at 4 °C for 5 min, and then the supernatant was discarded. Thereafter, a fluorescent secondary antibody (Alexa Fluor 647-conjugated AffiniPure Goat Anti-Human IgG, Fcy Fragment Specific, Jackson ImmunoResearch, #109-605-098, diluted in a 1:1000 ratio) was added at 100 µL/well, and the plate was incubated at 4 °C for 1 h away from light. Then, the cells in each well were then rinsed twice with 100 µL of pre-cooled PBS buffer and centrifuged at 500 g at 4 °C for 5 min, and then the supernatant was discarded. Finally, a pre-cooled FACS buffer (PBS buffer containing 0.5% BSA) was added at 200 µL/well to resuspend the cells. Fluorescence signal values were read using an ACEA NovoCyte flow cytometer or a BD FACS CANTOII flow cytometer, and the data were processed and analyzed using FlowJo v10 (FlowJo, LLC) software.

The data were processed and analyzed by plotting using GraphPad Prism 8 software, and binding curves of the antibodies to target cells, EC₅₀ values and other parameters were obtained through four-parameter nonlinear fitting.

### Example 6.1 Binding to CLDN18.2

In this example, the binding ability of the antibodies targeting CLDN18.2 to NUGC-4 cells highly expressing human CLDN18.2 (JCRB, JCRB0834) was determined. In this example, the anti-CLDN18.2 IgG monoclonal antibody PR002726 was used as a positive control molecule, and was also the parent monoclonal antibody of the CLDN18.2 end of the CLDN18.2×4-1BB bispecific antibody molecule and the CLDN18.2×OX40 bispecific antibody molecule.

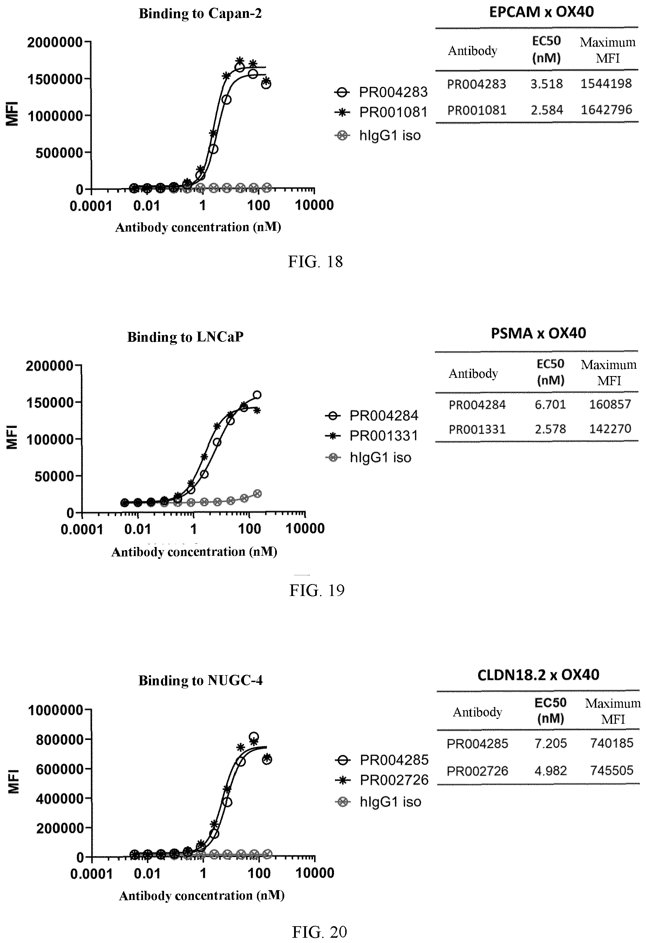

As shown in FIG. 20, the CLDN18.2×OX40 bispecific antibody molecule (PR004285) was able to bind to NUGC-4 cells, and had very close binding EC₅₀ and maximum fluorescence signal very close to those of the parent monoclonal antibody.

### Example 6.2 Binding to HER2

In this example, the binding ability of the antibodies targeting HER2 to SK-BR-3 cells highly expressing human HER2 (ATCC, HTB-30) was determined. In this example, the anti-HER2 IgG monoclonal antibody trastuzumab was used as a positive control molecule, and was also the parent monoclonal antibody of the HER2 end of the HER2×4-1BB bispecific antibody molecule.

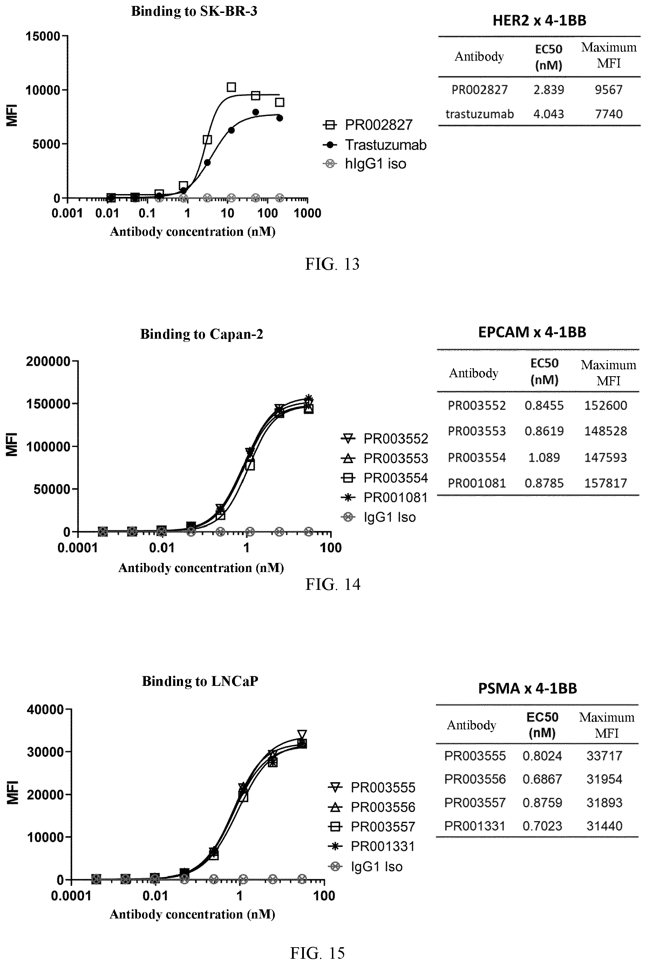

As shown in FIG. 13, the HER2×4-1BB bispecific antibody molecule (PR002827) was able to bind to SK-BR-3 cells, and had even slightly superior binding ability to that of the parent monoclonal antibody.

### Example 6.3 Binding to B7H4

In this example, the binding ability of the antibodies targeting B7H4 to SK-BR-3 cells highly expressing human B7H4 (ATCC, HTB-30) was determined. The anti-B7H4 IgG monoclonal antibody PR002408 or PR002410 was used as the parent monoclonal antibody of the B7H4 end of the B7H4×4-1BB bispecific antibody molecule and the B7H4×OX40 bispecific antibody molecule.

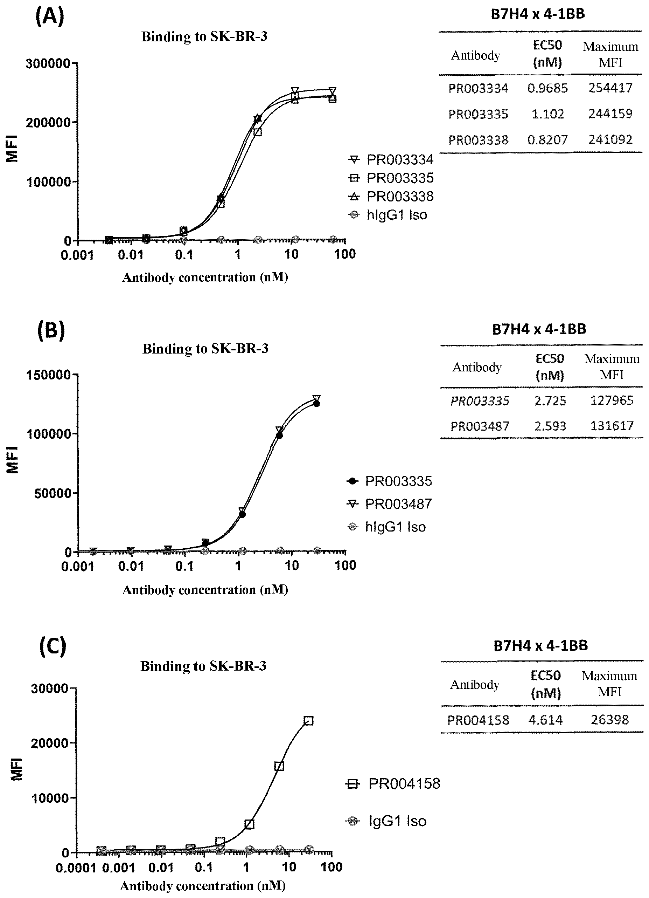

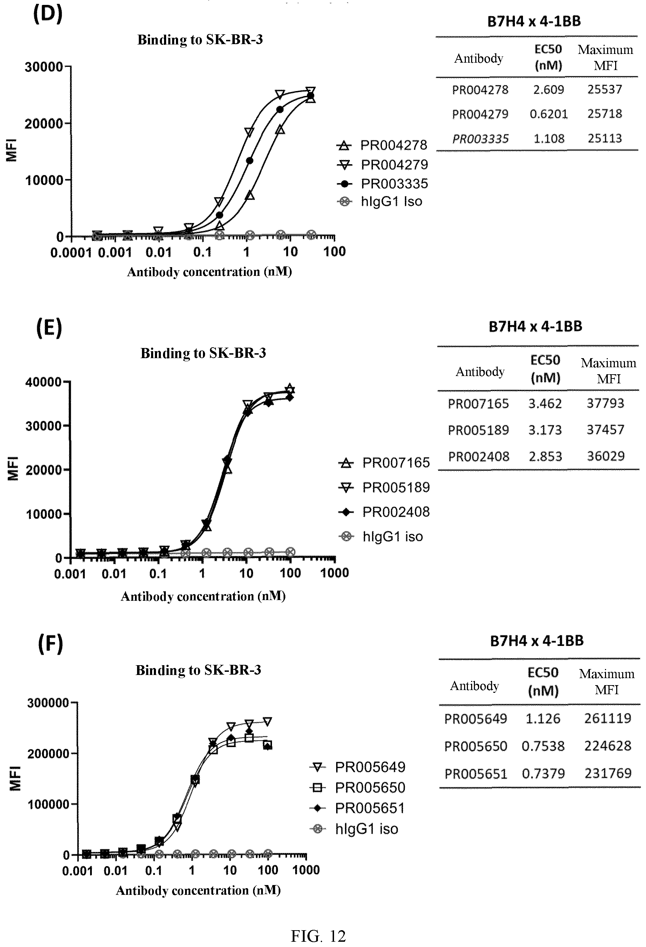

As shown in FIG. 12, the B7H4×4-1BB bispecific antibody molecules was able to bind to SK-BR-3 cells.

As shown in FIG. 12 (A), the B7H4×4-1BB bispecific antibody molecules with the IgG-VH tetravalent symmetric structure (PR003334, PR003335 and PR003338) were all able to bind to SK-BR-3, and had comparable binding ability.

As shown in FIG. 12 (B-C), the B7H4×4-1BB bispecific antibody molecules with the IgG-VH(2) hexavalent symmetric structure (PR003487 and PR004158) was able to bind to SK-BR-3.

As shown in FIG. 12 (D), the B7H4×4-1BB with the Fab-HCAb symmetric structure (PR004279) had slightly superior binding ability to that of the molecule with the VH-IgG_HC structure (PR004278).

As shown in FIG. 12 (E), the B7H4×4-1BB bispecific antibody molecules with the Fab-HCAb symmetric structure, whether the molecule with the structure (3) as described in Example 1.1.3 (PR005189) or the molecule with the structure (4) as described in Example 1.1.4 (PR007165), had almost identical binding activity, indicating that the fusion of the CL of the Fab to the heavy chain where VH_B is located does not affect the binding activity of the Fab end.

As shown in FIG. 12 (F), the first and second linker peptides of the Fab-HCAb structure did not affect the binding activity of the Fab end, for example, PR005649 had H1_15 sequence as the first linker peptide and human IgG2 hinge sequence as the second linker peptide, while PR005650 had the first linker peptide of 0 in length and human IgG1 hinge (C220S) sequence as the second linker peptide.

As shown in FIG. 12 (F), the bispecific antibody molecules with the Fab-VH-Fc-VH hexavalent symmetric structure (PR005650 and PR005651) were both able to bind to SK-BR-3.

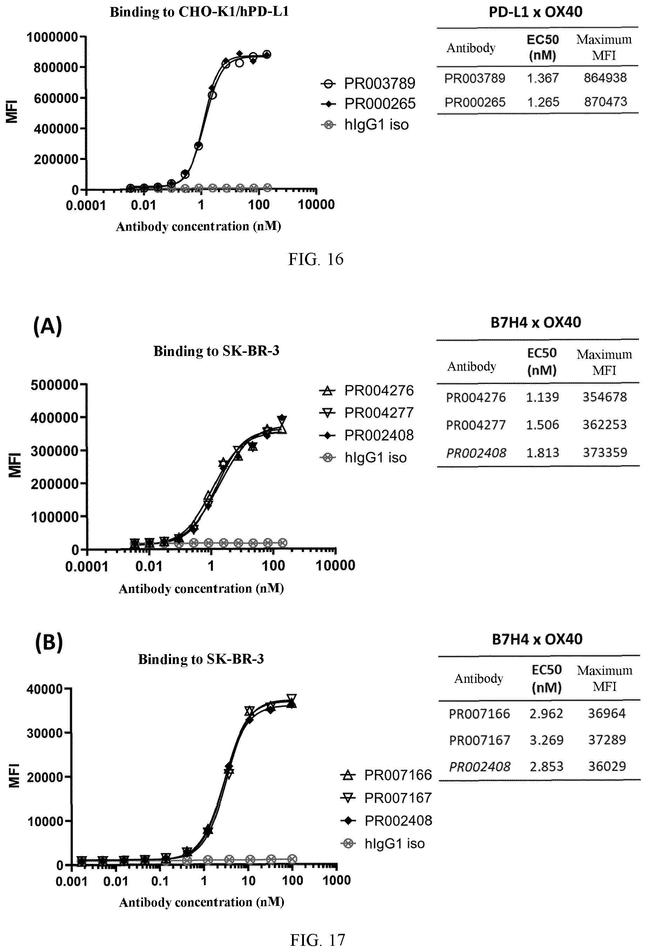

As shown in FIG. 17, the B7H4×OX40 bispecific antibody molecules with any structures, such as the molecule with the structure (1) (PR004276), the molecules with the structure (3) (PR004277 and PR007167) and the molecule with the structure (4) (PR007166), all had comparable binding ability to B7H4 to that of the parent monoclonal antibody PR002408.

### Example 6.4 Binding to PD-L1

In this example, the binding ability of the antibodies targeting PD-L1 to a CHO-K1 cell line CHO-K1/hPD-L1 (GenScript, M00543) highly expressing human PD-L1 was determined. In this example, the anti-PD-L1 IgG monoclonal antibody PR000265 was used as a positive control molecule, and was also the parent monoclonal antibody of the PD-L1 end of the PD-L1×4-1BB bispecific antibody molecule and the PD-L1×OX40 bispecific antibody molecule.

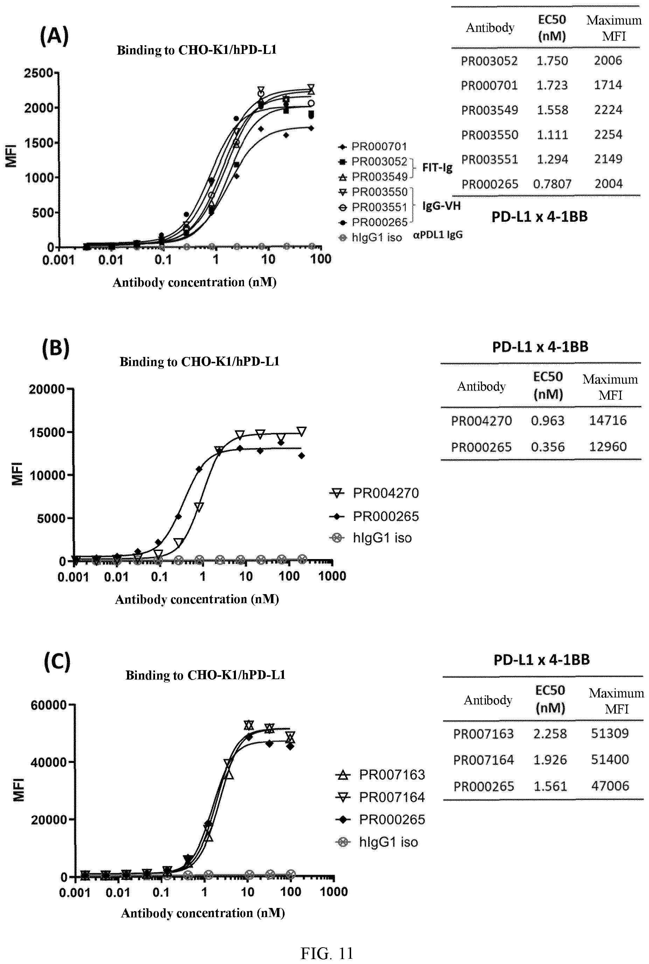

As shown in FIG. 11, the PD-L1×4-1BB bispecific antibody molecules were all able to bind to CHO-K1/hPD-L1 cells.

As shown in FIG. 11 (A), the PD-L1×4-1BB bispecific antibody molecules with the IgG-VH tetravalent symmetric structures (PR003549, PR003550, and PR003551) had similar binding ability to PD-L1 to the parent monoclonal antibody PR000265, and had slightly superior EC₅₀ values and maximum MFIs for binding to PD-L1 to that of the bispecific antibody molecules with the FIT-Ig structure (PR000701 and PR003052).

As shown in FIG. 11 (B), the PD-L1×4-1BB bispecific antibody molecule with the Fab-HCAb symmetric structure (PR004270) had similar binding ability to PD-L1 to the parent monoclonal antibody, and had higher binding maximum MFI than that of the parent monoclonal antibody.

As shown in FIG. 11 (C), the PD-L1×4-1BB bispecific antibody molecules with the Fab-HCAb symmetric structure, whether the molecule with the structure (3) as described in Example 1.1.3 (PR007164) or the molecule with the structure (4) as described in Example 1.1.4 (PR007163), had almost identical binding activity to PD-L1, indicating that the fusion of the CL of the Fab to the heavy chain where VH_B is located does not affect the binding activity of the Fab end.

As shown in FIG. 16, the PD-L1×OX40 bispecific antibody molecule (PR003789) was able to bind to CHO-K1/hPD-L1 cells, and had almost identical binding EC₅₀ and maximum fluorescence signal to those of the parent monoclonal antibody.

### Example 6.5 Binding to EPCAM

In this example, the binding ability of the antibodies targeting EPCAM to Capan-2 cells highly expressing human EPCAM (ATCC, HTB-80) was determined. In this example, the anti-EPCAM IgG monoclonal antibody PR001081 was used as a positive control molecule, and was also the parent monoclonal antibody of the EPCAM end of the EPCAM×4-1BB bispecific antibody molecule and the EPCAM×OX40 bispecific antibody molecule.

As shown in FIG. 14, the EPCAM×4-1BB bispecific antibody molecules (PR003552, PR003553 and PR003554) were all able to bind to Capan-2 cells, and had almost identical binding EC₅₀ and maximum fluorescence signals to those of the parent monoclonal antibody.

As shown in FIG. 18, the EPCAM×OX40 bispecific antibody molecule (PR004283) can bind to Capan-2 cells, and had very close binding EC₅₀ and maximum fluorescence signal to those of the parent monoclonal antibody.

### Example 6.6 Binding to PSMA

In this example, the binding ability of the antibodies targeting PSMA to LNCaP cells highly expressing human PSMA (Nanjing Cobioer, CBP60364) was determined. In this example, the anti-PSMA IgG monoclonal antibody PR001331 was used as a positive control molecule, and was also the parent monoclonal antibody of the PSMA end of the PSMA×4-1BB bispecific antibody molecule and the PSMA×OX40 bispecific antibody molecule.

As shown in FIG. 15, PSMA×4-1BB bispecific antibody molecules (PR003555, PR003556, and PR003557) were all able to bind to LNCaP cells, and had almost identical binding EC₅₀ and maximum fluorescence signals to those of the parent monoclonal antibody.

As shown in FIG. 19, the PSMA×OX40 bispecific antibody molecule (PR004284) was able to bind to LNCaP cells, and had very close binding EC₅₀ and maximum fluorescence signal to those of the parent monoclonal antibody.

### Example 7. T Cell Specific Activation Mediated by Target Cells

This example is intended to investigate the T cell activation activity of the bispecific immune cell engager binding protein targeting a costimulatory molecule on the surface of a T cell by binding to the costimulatory molecule in the presence of the target cell.

In this example, the bispecific immune cell engager binding protein may be a bispecific antibody molecule targeting 4-1BB or a bispecific antibody molecule targeting OX40. The target cell is a cell expressing a specific antigen (e.g., a tumor antigen), such as CHO-K1/hPD-L1 (GenScript, M00543) highly expressing human PD-L1, or MDA-MB-231 (ATCC, HTB-26) highly expressing human PD-L1, or SK-BR-3 (ATCC, HTB-30) highly expressing human B7H4, or CHO-K1/hB7H4 (produced in-house by Harbour BioMed) highly expressing human B7H4, or SK-BR-3 (ATCC, HTB-30) highly expressing human HER2, or LNCaP (Nanjing Cobioer, CBP60364) highly expressing human PSMA, or HEK293/hPSMA (produced in-house by Harbour BioMed) highly expressing human PSMA, or Capan-2 (ATCC, HTB-80) highly expressing human EPCAM, or NUGC4 (JCRB, JCRB0834) highly expressing human CLDN18.2. The effector cells may be isolated human PBMC or T cells.

Specifically, a 96-well plate (Corning, #3599) was coated first with 0.3 µg/mL anti-CD3 antibody OKT3 (Thermo, #16-0037-81) at 100 µL/well. Then, the density of human T cells (isolated from human PBMCs with a T cell isolation kit (Miltenyi, #130-096-535)) was adjusted to 2×10⁶ cells/mL, and the density of target cells was adjusted to 3×10⁵ cells/mL. The two cell suspensions were each seeded into a 96-well plate at 50 µL/well. Then, binding proteins at different concentrations were added at 100 µL/well, and two duplicate wells were set for each concentration. hIgG1 iso (CrownBio, #C0001) and hIgG4 iso (CrownBio, #C0045) were used as a control. The 96-well plate was incubated in an incubator at 37 °C with 5% CO₂ for 3 days. Supernatants after 48 h and 72 h of culture were each collected. The IL-2 concentration in the supernatant after 48 h was determined using an IL-2 ELISA kit (Thermo, #88-7025-88), and the IFN-γ concentration in the supernatant after 72 h was determined using an IFN-γ ELISA kit (Thermo, #88-7316-77). The ELISA assay was performed by referring to the instructions of relevant kit.

The data were processed and analyzed by plotting using GraphPad Prism 8 software.

### Example 7.1 T cell activation targeting-mediated by CLDN18.2

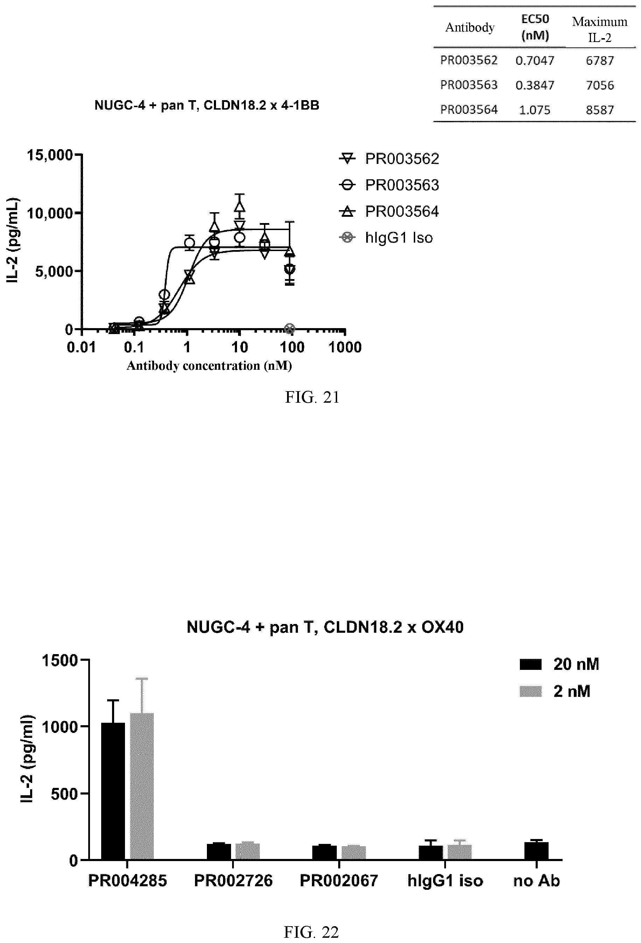

As shown in FIG. 21, in the presence of NUGC-4 cells highly expressing CLDN18.2, the CLDN18.2×4-1BB bispecific antibody molecules (PR003562, PR003563 and PR003564) were able to activate T cells and promote the production of cytokine IL-2 in an antibody concentration gradient-dependent manner.

As shown in FIG. 22, in the presence of NUGC-4 cells, the anti-OX40 HCAb monoclonal antibody PR002067 and the anti-CLDN18.2 IgG monoclonal antibody PR002726 were both unable to activate T cells. The CLDN18.2×OX40 bispecific antibody molecule PR004285 was able to activate T cells and promote the production of cytokine IL-2. This indicates that the T cell activation by the bispecific antibody molecules is dependent on the target cells.

### Example 7.2 T cell activation targeting-mediated by PSMA

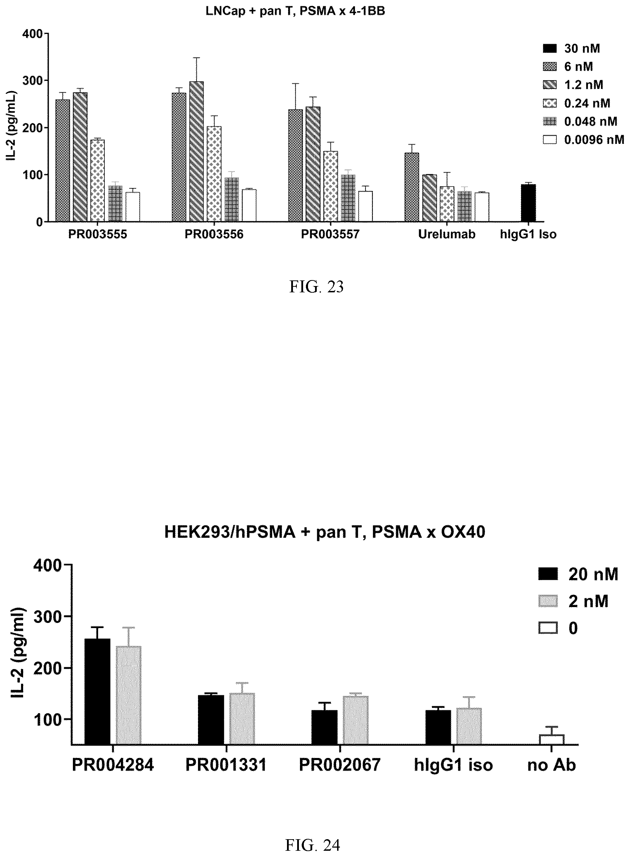

As shown in FIG. 23, in the presence of LNCaP cells highly expressing PSMA, the PSMA×4-1BB bispecific antibody molecules (PR003555, PR003556 and PR003557) had stronger T cell activation ability than that of urelumab, and were able to better promote the generation of IL-2.

As shown in FIG. 24, in the presence of HEK293/hPSMA cells highly expressing PSMA, the anti-OX40 HCAb monoclonal antibody PR002067 and the anti-PSMA IgG monoclonal antibody PR001331 were both unable to activate T cells. The PSMA×OX40 bispecific antibody molecule PR004284 was able to activate T cells and promote the production of cytokine IL-2. This indicates that the T cell activation by the bispecific antibody molecules is dependent on the target cells.

### Example 7.3 T cell activation targeting-mediated by EPCAM

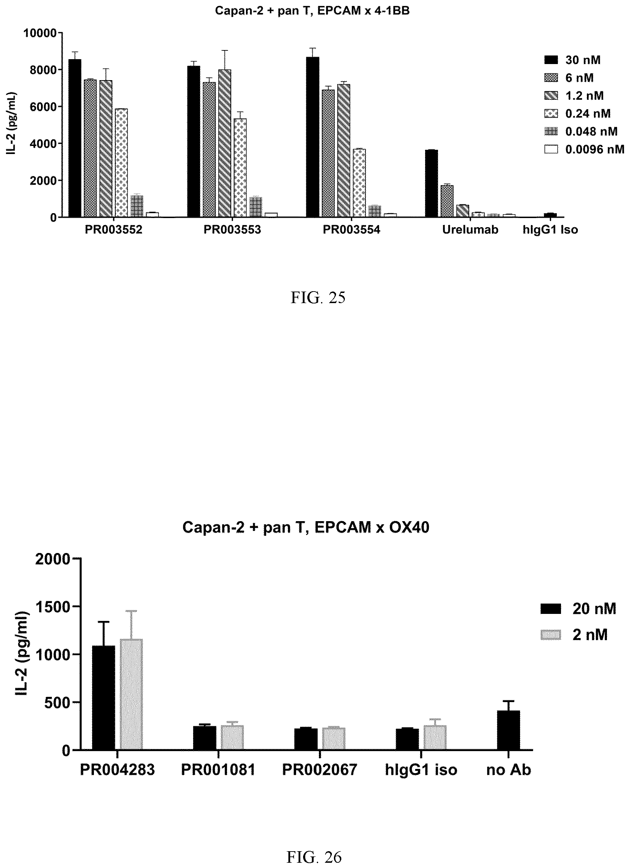

As shown in FIG. 25, in the presence of Capan-2 cells highly expressing EPCAM, the EPCAM×4-1BB bispecific antibody molecules (PR003552, PR003553 and PR003554) had T cell activation ability stronger than that of urelumab, and were able to better promote the generation of IL-2.

As shown in FIG. 26, in the presence of Capan-2 cells, the anti-OX40 HCAb monoclonal antibody PR002067 and the anti-EPCAM IgG monoclonal antibody PR001081 were both unable to activate T cells. The EPCAM×OX40 bispecific antibody molecule PR004283 was able to activate T cells and promote the production of cytokine IL-2. This indicates that the T cell activation by the bispecific antibody molecules is dependent on the target cells.

### Example 7.4 T cell activation targeting-mediated by PD-L1

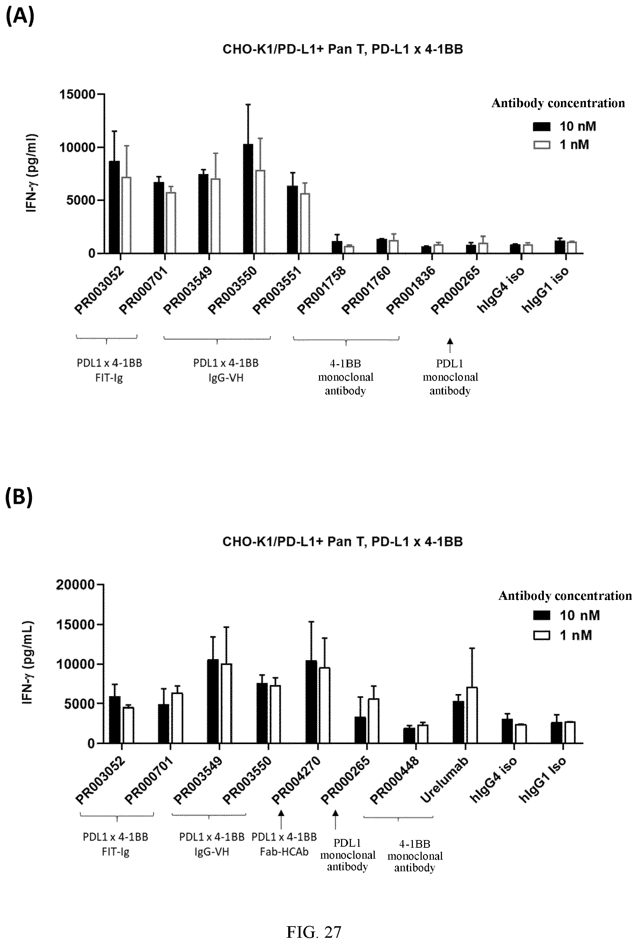

As shown in FIG. 27, in a system where a CHO-K1/hPD-L1 cell highly expressing PD-L1 was mixed with a T cell, crosslinking-independent anti-4-1BB monoclonal antibody urelumab was able to activate the T cell to release IFN-γ, while crosslinking-dependent anti-4-1BB monoclonal antibodies (PR000448, PR001758, PR001760 and PR001836) were hardly able to activate the T cell. The PD-L1×4-1BB bispecific antibody molecules with the FIT-Ig structure (PR003052 and PR000701), the PD-L1×4-1BB bispecific antibody molecules with the IgG-VH tetravalent symmetric structure (PR003549, PR003550 and PR003551) and the PD-L1×4-1BB bispecific antibody molecule with the Fab-HCAb structure (PR004270) were all able to activate T cells to release cytokines. This indicates that the T cell activation by PD-L1×4-1BB is dependent on the specific activation of target cells. Moreover, the bispecific antibody molecules with the IgG-VH tetravalent symmetric structure (PR003549 and PR003550) and the bispecific antibody molecule with the Fab-HCAb structure (PR004270) were able to cause higher release levels of cytokines and showed stronger T cell activation ability than the bispecific antibody molecules with the FIT-Ig structure (PR003052 and PR000701); and those bispecific antibody molecules were superior to urelumab.

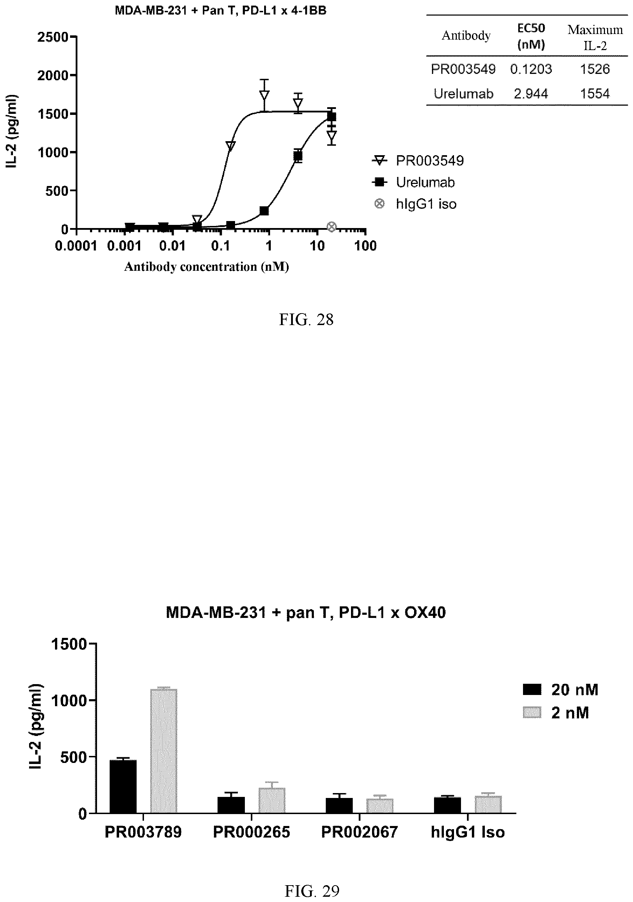

As shown in FIG. 28, in the presence of target cells MDA-MB-231 highly expressing PD-L1, the PD-L1×4-1BB bispecific antibody molecules with the IgG-VH tetravalent symmetric structure (PR003549) had stronger T cell activation ability than that of urelumab, and were able to better promote the generation of cytokine IL-2 in an antibody concentration gradient-dependent manner.

As shown in FIG. 29, in the presence of target cells MDA-MB-231 highly expressing PD-L1, the anti-OX40 HCAb monoclonal antibody PR002067 and the anti-PD-L1 IgG monoclonal antibody PR000265 were both unable to activate T cells. The PD-L1×OX40 bispecific antibody molecule PR003789 was able to activate T cells and promote the production of cytokine IL-2. This indicates that the T cell activation by the bispecific antibody molecules is dependent on the target cells.

### Example 7.5 T cell activation targeting-mediated by HER2

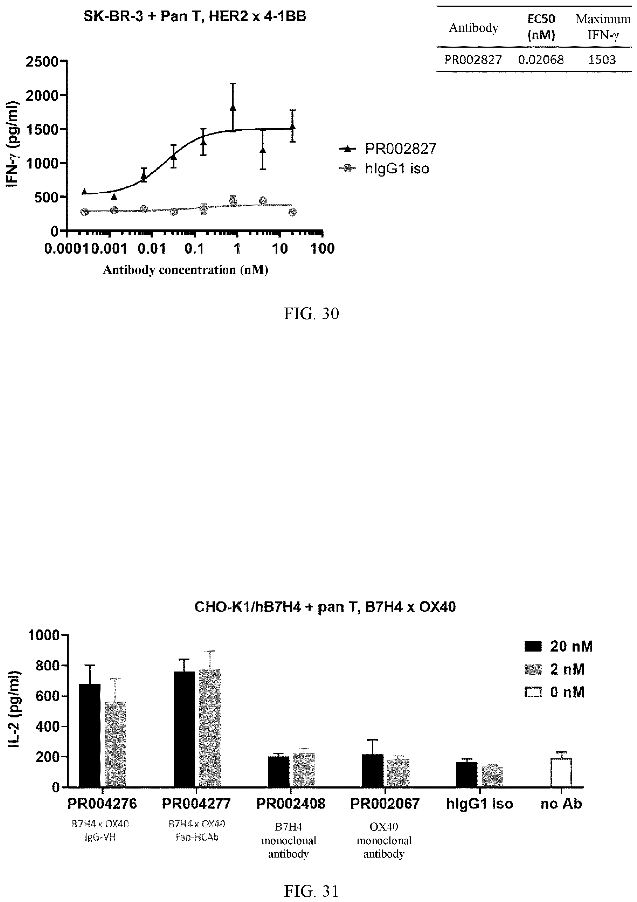

As shown in FIG. 30, in the presence of SK-BR-3 cells highly expressing HER2, the HER2×4-1BB bispecific antibody molecule (PR002827) was able to activate T cells and promote the production of cytokine IFN-γ in an antibody concentration gradient-dependent manner.

### Example 7.6 T cell activation targeting-mediated by B7H4

This example is intended to investigate the T cell activation ability of the B7H4×4-1BB or B7H4×OX40 bispecific antibody molecules targeting B7H4 in the presence of the target cells. The target cells may be cells expressing B7H4 to different degrees, such as SK-BR-3 (ATCC, HTB-30) highly expressing B7H4, or CHO-K1/hB7H4 (produced in-house by Harbour BioMed) highly expressing B7H4, or JIMT-1 (DSMZ, ACC 589) not expressing B7H4.

As shown in FIG. 31, in the presence of CHO-K1/hB7H4 cells highly expressing B7H4, the anti-OX40 HCAb monoclonal antibody PR002067 and the anti-B7H4 IgG monoclonal antibody PR002408 were both unable to activate T cells; the B7H4×OX40 bispecific antibody molecules PR004276 (IgG-VH structure) and PR004277 (Fab-HCAb structure) were both able to activate T cells and promote the production of cytokine IL-2. This indicates that the T cell activation by the bispecific antibody molecules is dependent on the target cells.

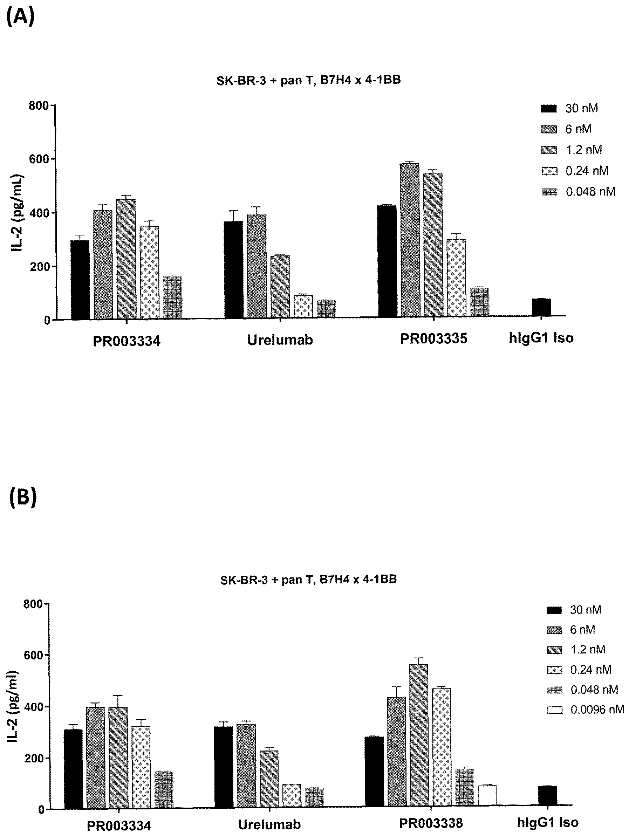

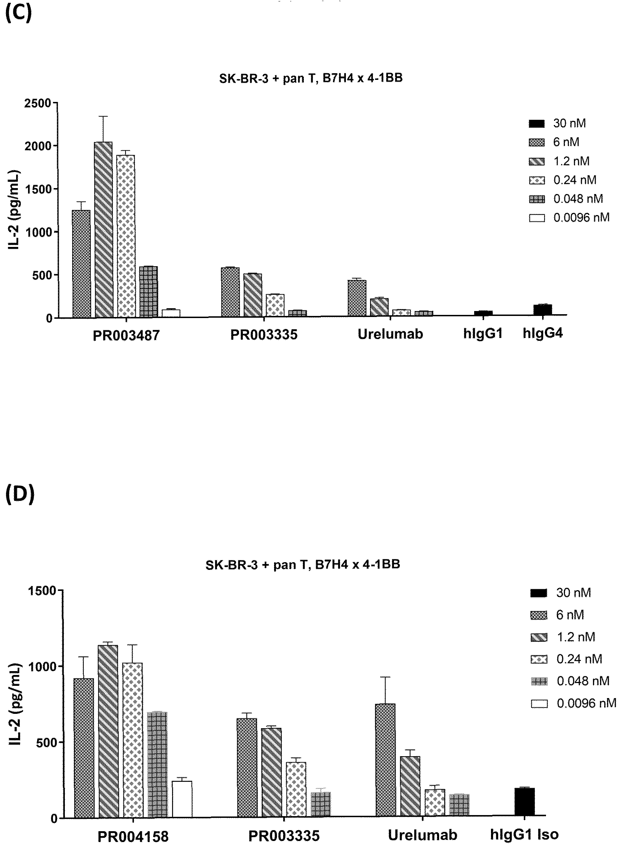

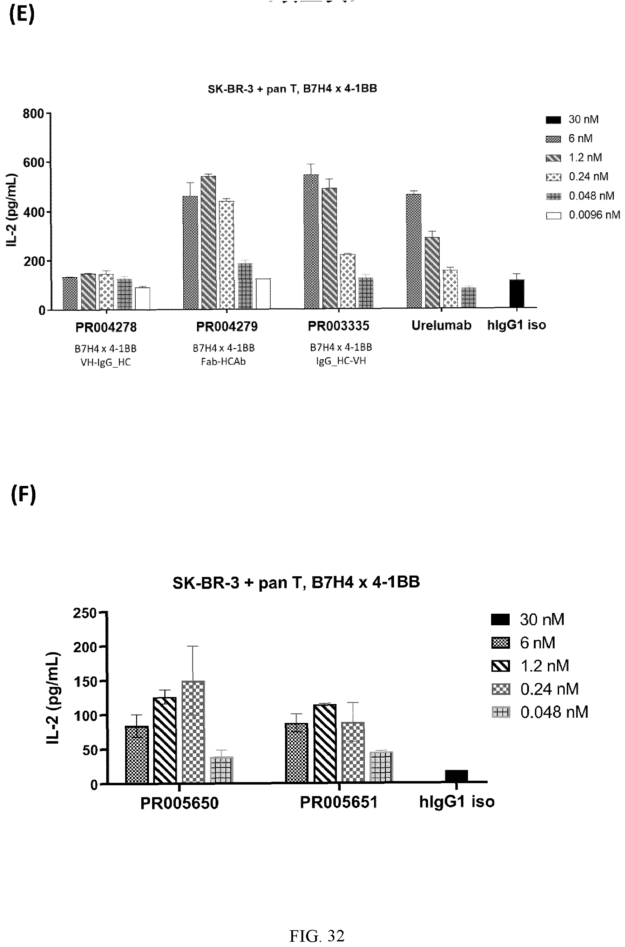

As shown in FIG. 32, in the presence of SK-BR-3 cells highly expressing B7H4, the B7H4×4-1BB bispecific antibody molecule had stronger T cell activation ability than that of urelumab, and was able to better promote the production of IL-2.

As shown in FIG. 32 (A-B), the bispecific antibody molecules with the IgG-VH tetravalent symmetric structure (PR003334, PR003335 and PR003338) had slightly stronger T cell activation ability than that of urelumab.

As shown in FIG. 32 (C-D), the bispecific antibodies with the IgG-VH(2) hexavalent symmetric structure (PR003487 and PR004158) had significantly stronger T cell activation ability than that of the IgG-VH tetravalent bispecific antibody and urelumab.

As shown in FIG. 32 (E), the bispecific antibody molecule with the Fab-HCAb symmetric structure (PR004279) had slightly stronger T cell activation ability than that of urelumab; when the 4-1BB-binding domain VH was at the C-terminus of the IgG heavy chain, the bispecific antibody molecule with the IgG-VH structure (PR003335) was able to activate T cells; while when the 4-1BB-binding domain VH was at the N-terminus of the IgG heavy chain, the bispecific antibody molecule with the VH-IgG structure (PR004278) was hardly able to activate T cells, although it was able to bind to 4-1BB (FIG. 3 (D)).

As shown in FIG. 32 (F), the bispecific antibodies with the Fab-VH-Fc-VH hexavalent symmetric structure (PR005650 and PR005651) were able to activate T cells.

This indicates that when the binding domain targeting 4-1BB is positioned between the Fab and the Fc (such as the Fab-HCAb structure, particularly the structure as described in Example 1.1.3 and FIG. 1 (3)) or at the C-terminus of Fc (such as the IgG-VH structures, particularly the structure as described in Example 1.1.1 and FIG. 1 (1)), the bispecific antibody molecule has the ability to crosslink 4-1BB and further activate T cells; while when the 4-1BB-binding domain is positioned at the N-terminus of the Fab (such as the VH-IgG structure, particularly the structure as described in Example 1.1.7 and FIG. 1 (7)), the bispecific antibody molecule is unable to activate T cells although it can bind to 4-1BB.

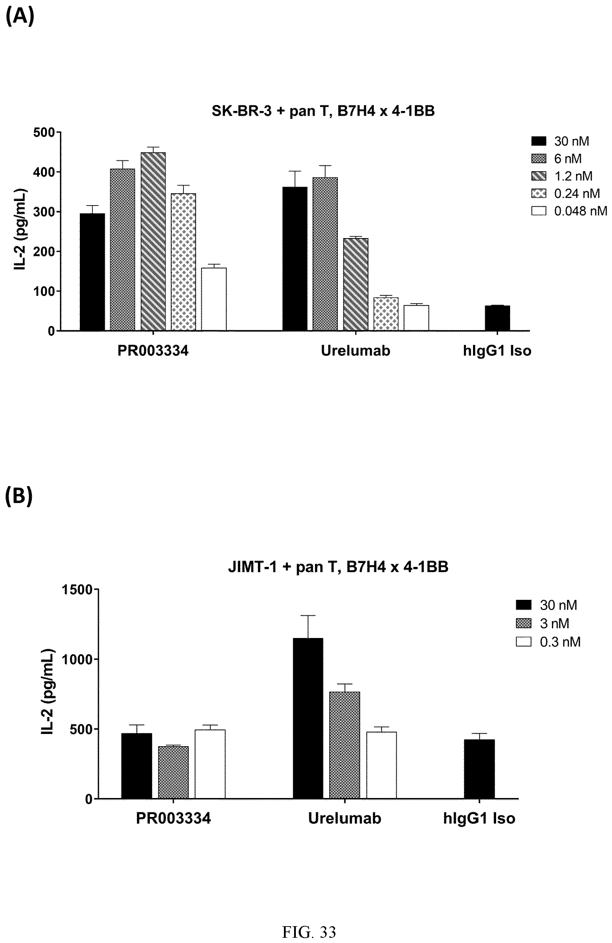

In another aspect, FIG. 33 shows that the T cell activation by B7H4×4-1BB is specifically dependent on the expression of B7H4. As shown in FIG. 33 (A), in the presence of SK-BR-3 cells highly expressing B7H4, the bispecific antibody molecule PR003334 was able to activate T cells; as shown in FIG. 33 (B), in the presence of JIMT-1 cells not expressing B7H4, PR003334 was unable to activate T cells. However, urelumab had no target specificity, so it was able to activate T cells even without expression of B7H4.

### Example 8. Mixed Lymphocyte Reaction (MLR)

This example is intended to investigate the T cell activation effects of the PD-L1×4-1BB bispecific antibody molecules by the mixed lymphocyte reaction (MLR).

In the first step, monocytes were isolated from PBMC cells (MT-Bio) of a first donor using CD14 magnetic beads (Meltenyi, #130-050-201) by referring to the instructions of the relevant kit. Then, 50 ng/mL of recombinant human IL-4 (PeproTech, #200-02-A) and 100 ng/mL of recombinant human GM-CSF (PeproTech, #300-03-A) were added, and after 7 days of induction at 37 °C, immature dendritic cells (iDC cells) were obtained. 1 µg/mL lipopolysaccharide (LPS, Sigma, #L6529) was then added, and after 24 h of induction, mature dendritic cells (mDC cells) were obtained. In the second step, T lymphocytes were isolated from PBMC cells (MT-Bio) of a second donor using a T cell isolation kit (Meltenyi, #130-096-535). In the third step, the obtained T cells and mDC cells were seeded in a 96-well plate (T cells at 1×10⁵/well and mDC cells at 2×10⁴/well) at a ratio of 5:1. Then, binding proteins at different concentrations were added at 50 µL/well, wherein the antibody concentration may be the final concentration of (10 nM, 1 nM), or a total of 8 concentrations obtained by a 3-fold gradient dilution from the highest final concentration of 50 nM; and two duplicate wells were set for each concentration. hIgG1 iso (CrownBio, #C0001) or a blank well was used as a control. The cells were incubated in an incubator at 37 °C with 5% CO₂ for 5 days. In the fourth step, supernatants on day 3 and on day 5 were each collected. The IL-2 concentration in the 3-day supernatant was determined using an IL-2 ELISA kit (Thermo, #88-7025-77), and the IFN-γ concentration in the 5-day supernatant was determined using an IFN-γ ELISA kit (Thermo, #88-7316-88). The ELISA assay was performed by referring to the instructions of relevant kit.

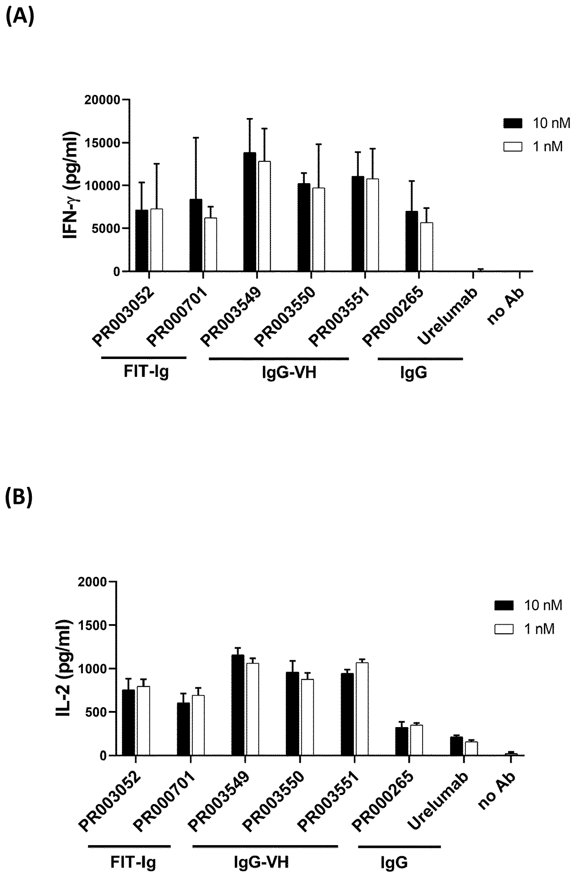

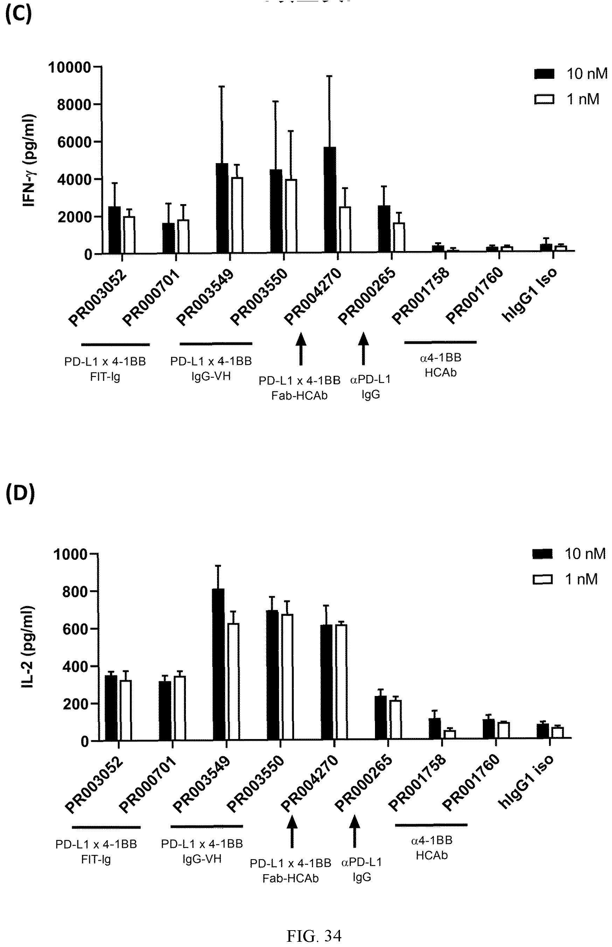

FIG. 34 shows two independent MLR assays using different donor PBMCs. In the two MLR assays, the anti-4-1BB monoclonal antibody urelumab had a limited T cell activation effect and weak cytokine production ability; the anti-PD-L1 monoclonal antibody PR000265 had a relatively significant activation effect; while the PD-L1×4-1BB bispecific antibody molecules were able to further improve the functions of the T cells.

In the first MLR assay (FIG. 34, A-B), the bispecific antibody molecules with the IgG-VH tetravalent symmetric structure (PR003549, PR003550 and PR003551) were able to cause higher release levels of cytokines and showed stronger T cell activation ability than the antibody molecules with the FIT-Ig structure (PR003052 and PR000701); and those bispecific antibody molecules were superior to the PD-L1 monoclonal antibodies.

In the second MLR assay (FIG. 34, C-D), the bispecific antibody molecules with the IgG-VH tetravalent symmetric structure (PR003549 and PR003550) and the bispecific antibody molecule with the Fab-HCAb structure (PR004270) were able to cause higher release levels of cytokines and showed stronger T cell activation ability than the bispecific antibody molecules with the FIT-Ig structure (PR003052 and PR000701); and those bispecific antibody molecules were superior to the PD-L1 monoclonal antibodies.

### Example 9. Pharmacokinetic Study

In this example, the pharmacokinetic properties of the bispecific immune cell engager binding protein molecules with different structures in mice were investigated.

Administration and blood collection: for each test binding protein molecule, 6 female BALB/C or C57BL/6 mice weighing 18-22 g were selected and administered with the test binding protein molecule intravenously at a dose of 5 mg/kg. The whole blood of 3 mice in one group was collected before the administration and 15 min, 24 h (1 day), 4 days and 10 days after the administration, and the whole blood of 3 mice in the other group was collected before the administration and 5 h, 2 days, 7 days and 14 days after the administration. The whole blood was left to stand for 30 min for coagulation, and then centrifuged, and the isolated serum sample was cryopreserved at -80 °C until it was taken for analysis.

Analysis method: the drug concentration in the serum of mice was quantitatively determined by two ELISA methods. ELISA method I, namely the Fc end detection method, was performed by capturing human Fc-containing antibodies in the serum of mice using a goat anti-human Fc polyclonal antibody coating a 96-well plate, and then adding an HRP-labeled goat anti-human Fc secondary antibody. ELISA method II, namely the functional domain detection method, was performed by capturing the antibodies specifically recognizing the antigens in the serum of mice using an antigenic protein coating a 96-well plate, and then adding an HRP-labeled goat anti-human Fc secondary antibody. For example, in this example, a human PD-L1 protein was used to capture the bispecific antibody molecule PR004270; or a human 4-1BB protein was used to capture the bispecific antibody molecules PR003334 and PR003335. Pharmacokinetic parameters were analyzed using Phoenix WinNonlin software version 8.2 by non-compartmental analysis (NCA).

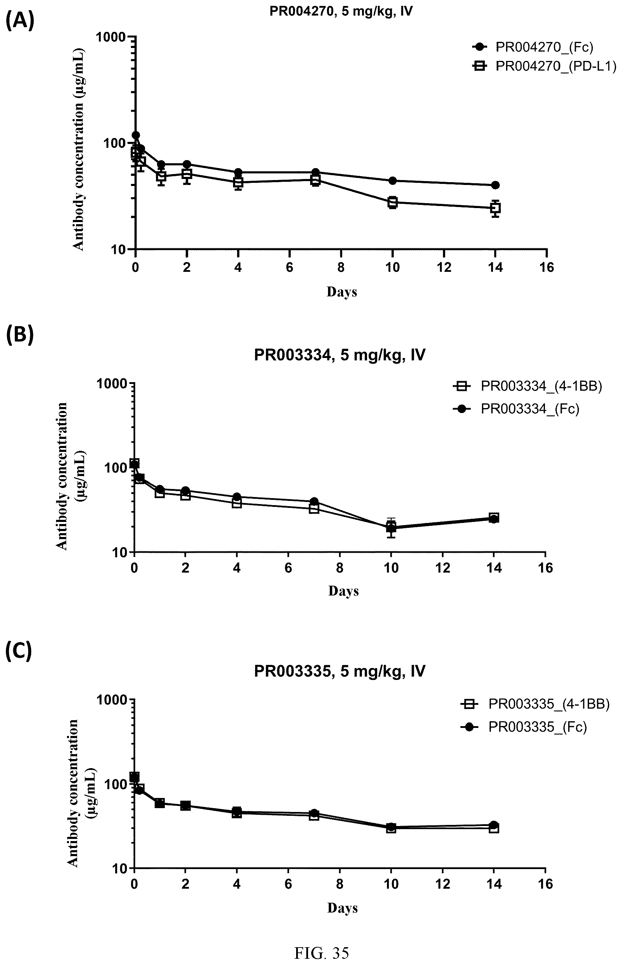

As shown in FIG. 35 and Table 13, the bispecific antibody molecule with the Fab-HCAb structure (PR004270) had a similar serum half-life t_{1/2} value to a conventional IgG antibody, and the t_{1/2} value was more than 10 days as measured by the PD-L1 end detection method. The bispecific antibody molecules with the IgG-VH structure (PR003334 and PR003335) had similar pharmacokinetics to conventional IgG antibodies: under the Fc end detection method, the serum half-life t_{1/2} values of PR003334 and PR003335 in mice were about 8 days and 14 days, respectively; and under the 4-1BB end detection method, the serum half-life t_{1/2} values of PR003334 and PR003335 in mice were about 9 days and 12 days, respectively.

**Table 13. Pharmacokinetic parameters of bispecific antibody molecules in mice**

| Bispecific antibody molecule | PR003334 | | PR003335 | | PR004270 | |
|---|---|---|---|---|---|---|
| Animals (number) | C57BL/6 mice (n = 6) | | C57BL/6 mice (n = 6) | | BALB/c mice (n = 6) | |
| Antibody dosage | 5 mg/kg, I.V. | | 5 mg/kg, I.V. | | 5 mg/kg, I.V. | |
| PK parameters | Fc end detection | 4-1BB end detection | Fc end detection | 4-1BB end detection | Fc end detection | PD-L1 end detection |
| T_{1/2} (hour) | 193 | 218 | 336 | 295 | 465.6 | 256.5 |
| V_{d} (mL/kg) | 76 | 89 | 81 | 81 | 75.7 | 83.9 |
| AUC(µg^{∗}hour/mL ) | 12,680 | 11,471 | 14,813 | 14,286 | 17,536 | 13,126 |
| Cl (mL/hour/kg) | 0.28 | 0.28 | 0.17 | 0.19 | 0.11 | 0.23 |
| C₀ (µg/mL) | 111 | 114 | 123 | 124 | 119.7 | 81.7 |

### Example 10. Anti-Tumor Efficacy of Bispecific Antibody Molecules

In this example, the anti-tumor effect of B7H4×4-1BB bispecific antibody molecule PR003334 in a BALB/c-hCD137/CT26-hB7H4 mouse tumor model was investigated.

The test was performed as follows: female BALB/c-hCD137 mice (BALB/c mice introduced with exogenous human 4-1BB transgene, GemPharmatech) aged 6-8 weeks were selected, and were inoculated subcutaneously with CT26-hB7H4 tumor cells (mouse colon cancer cells CT26 introduced with exogenous human B7H4 transgene, Harbour BioMed) in the logarithmic growth phase on the right dorsal side of the experimental mice at a dose of 5×10⁵ cells/mouse. When the mean tumor volume reached 80 mm³, the mice were randomized into groups of 6. On the day of grouping, the drug diluted with PBS at a specific concentration was administered by intraperitoneal injection (i.p.), twice a week for a total of 6 doses (BIW×3), with PBS as a blank control group. Tumor volume and body weight of mice were measured on the day of the first administration and on days 4, 7, 11, 14 and 18 thereafter. The tumor volume was calculated as follows: tumor volume (mm³) = 0.5 × (long diameter of tumor × short diameter of tumor²). After the experiment was completed, tumor-bearing mice were euthanized, stripped of tumors and weighed. The tumor volume, body weight, and the like of mice in each group were calculated and the results were expressed as mean ± standard error (Mean ± SEM). The different treatment groups were separately compared with the control group for any significant difference by one-way analysis of variance (one-way ANOVA).

In this example, the anti-4-1BB monoclonal antibody urelumab was used as a positive control molecule. The dose of 6 mpk (mg/kg) for the bispecific antibody molecule PR003334 and the dose of 5 mpk for urelumab were equivalent doses at the same molar concentration (converted according to molecular weight); and the dose of 18 mpk for the bispecific antibody molecule PR003334 and the dose of 15 mpk for urelumab were equivalent doses at the same molar concentration. An anti-mouse PD-1 antibody (Bio X Cell, clone RMP1-14, #BE0146) at a dose of 10 mpk was also used as a control molecule. PBS was used as a blank control group.

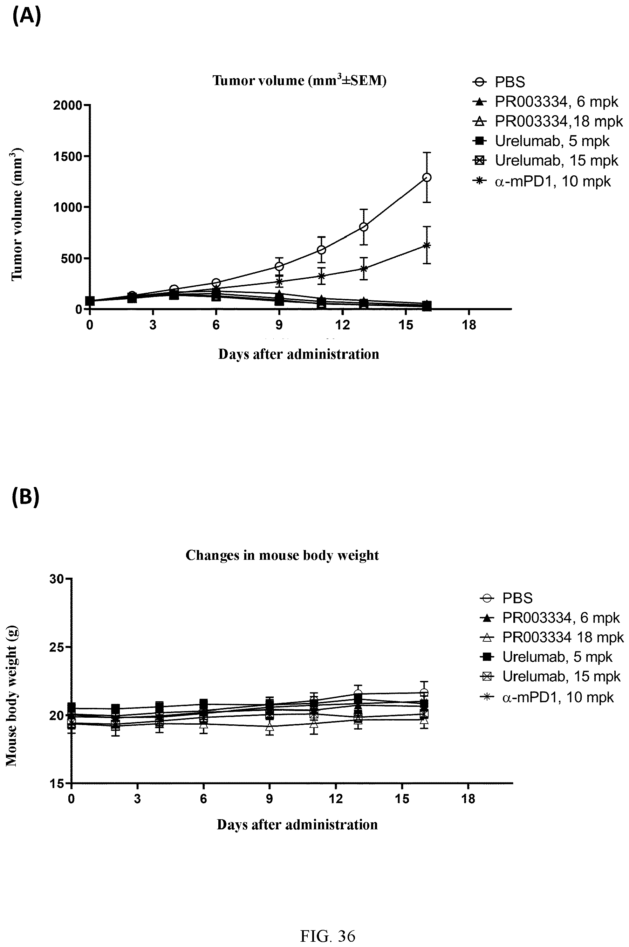

As shown in FIG. 36, the bispecific antibody molecule PR003334 and urelumab both had the efficacy of inhibiting the tumor growth in each dose group, and were superior to the PD-1 antibody (A). The change in body weight of mice in each test group was within the normal range (B).

### Example 11. Determination of Stability and Physicochemical Properties of Bispecific Antibody Molecules by UNcle

In this example, the physicochemical properties such as molecular stability of the B7H4×4-1BB bispecific antibody molecules (PR003334, PR003335 and PR003338) were determined by the method described in [0312]. As shown in Table 14, those bispecific antibody molecules had good stability.

**Table 14. Determination parameters for bispecific antibody molecules in UNcle**

| | **PR003334** | **PR003335** | **PR003338** |
|---|---|---|---|
| Tₘ (°C) | 60.32 | 65.59 | 62.17 |
| T_{agg} (°C) at 266 nm | 52.00 | 50.39 | 54.14 |
| T_{agg} (°C at 473 nm | / | / | / |
| Initial SLS at 266 nm (counts x10⁴) | 0.26 | 0.22 | 0.20 |
| Max SLS at 266 nm (counts x10⁴) | 1.00 | 0.40 | 0.34 |
| Initial SLS at 473 nm (counts) | < 0 | < 0 | < 0 |
| Max SLS at 473 nm (counts) | < 0 | < 0 | < 0 |
| Initial diameter (nm) (25°C) | 16.22 | 16.38 | 11.74 |
| Final diameter (nm) (95°C) | 23.85 | 17.51 | 16.52 |
| Initial PDI | 0.267 | 0.229 | 0.043 |
| Final PDI | 0.089 | 0.194 | 0.185 |

### Example 12. Summary

In the present application, several bispecific immune cell engager binding protein molecules were constructed, one end of which can bind to a costimulatory molecule on the surface of an immune cell (e.g., 4-1BB, OX40, etc.), and the other end of which can bind to a specific antigen molecule on the surface of a tumor cell (e.g., HER2, PSMA, EPCAM, CLDN18.2, B7H4, etc.) or a co-inhibitory molecule on the surface of an immune cell (e.g., PD-L1, etc.). Those bispecific antibody molecules can bring the immune cells and the tumor cells together, efficiently and selectively activate the immune cells in a tumor microenvironment, and reduce toxic and side effects caused by non-specific activation of peripheral immune cells. By utilizing the crosslinking of the costimulatory molecules mediated by target cell antigens and the activation of downstream signaling pathways, the bispecific antibody molecules promote the activation and proliferation of the immune cells, and improve the efficacy of killing the tumors.

In the present application, several bispecific immune cell engager binding protein molecules with different structures are constructed. The IgG-VH symmetric structure (particularly the structure as described in Example 1.1.1 and FIG. 1(1)) and the Fab-HCAb symmetric structure (particularly the structure as described in Example 1.1.3 and FIG. 1 (3)) show excellent universality and druggability, and show excellent biological activity, molecular stability and pharmaceutical properties on a plurality of bispecific antibody molecules with different combinations of targets.

In some embodiments, it is shown that the T cell activation by the bispecific immune cell engager binding protein molecules is dependent on the target cells. As reflected in Example 7.6 and FIG. 33, in the presence of SK-BR-3 cells highly expressing B7H4, the B7H4×4-1BB bispecific antibody molecules are able to specifically activate T cells to release IL-2; however, in the presence of JIMT-1 cells not expressing B7H4, the B7H4×4-1BB bispecific antibody molecules are unable to activate T cells. Urelumab had no target specificity, so it was able to activate T cells even without expression of B7H4. This may indicate that the B7H4×4-1BB bispecific antibody molecule has better safety than urelumab.

In some embodiments, it is shown that increasing the number of the antigen-binding fragments targeting immune cell costimulatory molecules (e.g., 4-1BB) (effector moieties) can significantly improve the immune cell activation ability of the bispecific antibody molecules, such as the potent T cell activation ability of the bispecific antibody molecules with the IgG-VH(2) symmetric structure (particularly the structure as described in Example 1.1.2 and FIG. 1 (2)) embodied in Example 7.6 and FIG. 32 (C-D).

In some embodiments, it is shown that the position and distance of the antigen-binding fragment targeting the immune cell costimulatory molecule (e.g., 4-1BB) (effector moiety) relative to the antigen-binding fragment targeting the tumor cell (targeting moiety) are critical to the functional activity of the bispecific antibody molecule. As reflected in Example 7.6 and FIG. 32 (E), when the binding domain targeting 4-1BB is positioned between the Fab and the Fc (such as the Fab-HCAb structure, particularly the structure as described in Example 1.1.3 and FIG. 1 (3)) or at the C-terminus of Fc (such as the IgG-VH structures, particularly the structure as described in Example 1.1.1 and FIG. 1 (1)), the bispecific antibody molecule have the ability to crosslink 4-1BB and further activate T cells; while when the 4-1BB-binding domain is positioned at the N-terminus of the Fab (such as the VH-IgG structure, particularly the structure as described in Example 1.1.7 and FIG. 1 (7)), the bispecific antibody molecule is unable to activate T cells although it can bind to 4-1BB.

Based on the findings described above, in one aspect, the number of the antigen-binding fragments VHs (as shown in the black domain in FIG. 37) targeting the immune cell costimulatory molecule can be increased based on the IgG-VH structure or Fab-HCAb structure to improve the ability to activate the immune cells; in another aspect, the Fc region can be deleted to make the molecule smaller (e.g., the VH domain in FIG. 37 (E) is linked to the C'-terminus of the CH1 of the Fab and the C'-terminus of the CL of the Fab, respectively). The structures of other derived bi- (multi-) specific binding proteins based on heavy-chain antibodies or single-domain antibodies are listed in FIG. 37.

## Claims

1. A binding protein comprising at least two protein functional regions, comprising a first protein functional region and a second protein functional region, wherein the first protein functional region comprises a targeting moiety binding to an antigen on a target cell, and the second protein functional region comprises an effector moiety binding to an antigen on an immune effector cell;
the binding protein is selected from the following structures:
(I) the first protein functional region is of a Fab structure, the second protein functional region is of a VH structure, and the first protein functional region and the second protein functional region are arranged from the N-terminus to the C-terminus of the binding protein; and
(II) the first protein functional region is of a Fab structure, the second protein functional region is of a Fab structure, and the first protein functional region and the second protein functional region are arranged from the N-terminus to the C-terminus of the binding protein;
preferably, the binding protein is of (a) a monomeric structure, or (b) a dimeric structure consisting of two monomers.

2. The binding protein according to claim 1, wherein
in the monomeric structure of the (a), the binding protein has two second protein functional regions, one of which is linked to the C-terminus of the heavy chain CH1 of the Fab structure of the first protein functional region, and the other of which is linked to the C-terminus of the light chain CL of the Fab structure of the first protein functional region;
in the dimeric structure of the (b), the binding protein further comprises an Fc;
preferably, the Fc is positioned between the first protein functional region and the second protein functional region; or, the Fc is positioned at the C-terminus of the second protein functional region; or, the Fc is positioned between the two second protein functional regions;
more preferably, in each monomer, the number of the first protein functional region is 1, the number of the Fc is 1, and the number of the second protein functional region is 1 or more than one, e.g., 1, 2 or 3;
even more preferably, the binding protein further comprises a third protein functional region, wherein the third protein functional region is positioned at the C- or N-terminus of the binding protein, or at the C- or N-terminus of the Fc structure of the binding protein;
even more preferably, the number of the third protein functional region is 1 or more than one, e.g., 1, 2 or 3.

3. The binding protein according to claim 1 or 2, wherein
the binding protein comprises a polypeptide chain 1 and a polypeptide chain 2, wherein the polypeptide chain 1 is as shown in a formula of N'-VL_A-CL-C', and the polypeptide chain 2 is as shown in a formula of N'-VH_A-CH1-h-CH2-CH3-L-VH_B-C', wherein the L is a linker peptide being preferably 0 in length or having an amino acid sequence preferably as set forth in any one of SEQ ID NOs: 244-265 or an amino acid sequence of GS;
or, the binding protein comprises a polypeptide chain 1 and a polypeptide chain 2, wherein the polypeptide chain 1 is as shown in a formula of N'-VL_A-CL-C', and the polypeptide chain 2 is as shown in a formula of N'-VH_A-CH1-h-CH2-CH3-L1-VH_B-L2-VH_B-C', wherein the L1 and L2 are linker peptides being preferably 0 in length or having an amino acid sequence preferably as set forth in any one of SEQ ID NOs: 244-265 or an amino acid sequence of GS;
or, the binding protein comprises a polypeptide chain 1 and a polypeptide chain 2, wherein the polypeptide chain 1 is as shown in a formula of N'-VH_A-CH1-C', and the polypeptide chain 2 is as shown in a formula of N'-VL_A-CL-L1-VH_B-L2-CH2-CH3-C', wherein the L1 and L2 are linker peptides being preferably 0 in length or having an amino acid sequence preferably as set forth in any one of SEQ ID NOs: 244-265 or an amino acid sequence of GS, and the L2 further has an amino acid sequence preferably as set forth in SEQ ID NO: 253, SEQ ID NO: 262 or SEQ ID NO: 263;
or, the binding protein comprises a polypeptide chain 1 and a polypeptide chain 2, wherein the polypeptide chain 1 is as shown in a formula of N'-VL_A-CL-C', and the polypeptide chain 2 is as shown in a formula of N'-VH_A-CH1-L1-VH_B-L2-CH2-CH3-C', wherein the L1 and L2 are linker peptides being preferably 0 in length or having an amino acid sequence preferably as set forth in any one of SEQ ID NOs: 244-265 or an amino acid sequence of GS, and the L2 further has an amino acid sequence preferably as set forth in SEQ ID NO: 253, SEQ ID NO: 262 or SEQ ID NO: 263;
or, the binding protein comprises a polypeptide chain 1 and a polypeptide chain 2, wherein the polypeptide chain 1 is as shown in a formula of N'-VH_A-CH1-C', and the polypeptide chain 2 is as shown in a formula of N'-VL_A-CL-L1-VH_B-L2-CH2-CH3-L3-VH_B-C', wherein the L1, L2 and L3 are linker peptides being preferably 0 in length or having an amino acid sequence preferably as set forth in any one of SEQ ID NOs: 244-265 or an amino acid sequence of GS, and the L2 further has an amino acid sequence preferably as set forth in SEQ ID NO: 253, SEQ ID NO: 262 or SEQ ID NO: 263;
or, the binding protein comprises a polypeptide chain 1 and a polypeptide chain 2, wherein the polypeptide chain 1 is as shown in a formula of N'-VL_A-CL-C', and the polypeptide chain 2 is as shown in a formula of N'-VH_A-CH1-L1-VH_B-L2-CH2-CH3-L3-VH_B-C', wherein the L1, L2 and L3 are linker peptides being preferably 0 in length or having an amino acid sequence preferably as set forth in any one of SEQ ID NOs: 244-265 or an amino acid sequence of GS, and the L2 further has an amino acid sequence preferably as set forth in SEQ ID NO: 253, SEQ ID NO: 262 or SEQ ID NO: 263;
or, the binding protein comprises a polypeptide chain 1, a polypeptide chain 2 and a polypeptide chain 3, wherein the polypeptide chain 1 is as shown in a formula of N'-VL_A-CL-L-VH_B-CH1-h-CH2-CH3-C', the polypeptide chain 2 is as shown in a formula of N'-VH_A-CH1-C', and the polypeptide chain 3 is as shown in a formula of N'-VL_B-CL-C', wherein the L is a linker peptide being preferably 0 in length or having an amino acid sequence preferably as set forth in any one of SEQ ID NOs: 244-265 or an amino acid sequence of GS;
wherein the VL_B and/or the VH_B are VL and/or VH of the second protein functional region, respectively, and the VL_A and/or the VH_A are VL and/or VH of the first protein functional region, respectively; the h is a hinge region; the CL is a light chain constant region domain; the CH1, CH2 and CH3 are a first domain, a second domain and a third domain of a heavy chain constant region, respectively; and the L or L1 or L2 or L3 is a linker peptide.

4. The binding protein according to any one of claims 1-3, wherein the antigen on the target cell is a tumor antigen and/or a co-inhibitory molecule antigen;
wherein the tumor antigen is CD19, BCMA, TSHR, CD171, CS-1, CLL-1, GD3, Tn Ag, FLT3, CD38, CD123, CD44v6, B7H3, B7H4, KIT, IL-13Ra2, IL-11Ra, PSCA, PSMA, PRSS21, VEGFR2, LewisY, CD24, PDGFR-beta, SSEA-4, MUC1, EGFR, NCAM, CAIX, LMP2, EphA2, fucosyl GM1, sLe, GM3, TGS5, HMWMAA, GD2, FOLR1, FOLR2, TEM1/CD248, TEM7R, CLDN6, CLDN18.2, GPRC5D, CXORF61, CD97, CD179a, ALK, polysialic acid, PLAC1, GloboH, NY-BR-1, UPK2, HAVCR1, ADRB3, PANX3, GPR20, LY6K, OR51E2, TAARP, WT1, ETV6-AML, SPA17, XAGE1, Tie 2, MAD-CT-1, MAD-CT-2, FOSL1, hTERT, ML-IAP, ERG, NA17, PAX3, AR, cyclin B1, MYCN, RhoC, CYP1B1, BORIS, SART3, PAX5, OY-TES1, LCK, AKAP-4, SSX2, CD79a, CD79b, CD72, LAIR1, FCAR, LILRA2, CD300LF, CLEC12A, BST2, EMR2, LY75, GPC3, FCRL5, IGLL1, CD20, CD30, HER2, ROR1, FLT3, TAAG72, CD22, CD33, GD2, gp100Tn, FAP, tyrosinase, EPCAM, CEA, IGF-1R, EphB2, mesothelin, cadherin17, CD32b, EGFRvIII, GPNMB, GPR64, HER3, LRP6, LYPD8, NKG2D, SLC34A2, SLC39A6, SLITRK6, GUCY2C or TACSTD2; preferably B7H3, B7H4, PSMA, CLDN18.2, GPC3, HER2, ROR1, CD22, EPCAM, mesothelin, EGFRvIII or TACSTD2;
and/or, the co-inhibitory molecule antigen is PD-L1, PD-L2, CTLA4, B7H3, B7H4, VISTA, HHLA2 or CD155, preferably PD-L1, B7H3, B7H4 or HHLA2;
and/or the antigen on the immune effector cell is a stimulatory antigen or a costimulatory molecule antigen, e.g., TCR/CD3, CD28, 4-1BB, OX40, GITR, CD27, ICOS, CD2, CD40, CD226, CD16, NKp30, NKp44, NKp46, NKG2D or 2B4, preferably TCR/CD3, CD28, 4-1BB, OX40, GITR, CD40, CD226, CD16 orNKp30;
preferably:
the antigen on the target cell is CLDN18.2, HER2, B7H4, EPCAM, PSMA or PD-L1; and/or the antigen on the immune effector cell is 4-1BB or OX40.

5. The binding protein according to any one of claims 1-4, wherein the first protein functional region is an antibody targeting CLDN18.2 or an antigen-binding fragment thereof, an antibody targeting HER2 or an antigen-binding fragment thereof, an antibody targeting B7H4 or an antigen-binding fragment thereof, an antibody targeting EPCAM or an antigen-binding fragment thereof, an antibody targeting PSMA or an antigen-binding fragment thereof, and/or an antibody targeting PD-L1 or an antigen-binding fragment thereof; and
the second protein functional region is an antibody targeting OX40 or an antigen-binding fragment thereof, or an antibody targeting 4-1BB or an antigen-binding fragment thereof;
preferably:
the antibody targeting CLDN18.2 or the antigen-binding fragment thereof comprises a heavy chain variable region (VH) and a light chain variable region (VL), wherein the VH comprises VH CDR1 with a sequence as set forth in SEQ ID NO: 22, VH CDR2 with a sequence as set forth in SEQ ID NO: 55 and VH CDR3 with a sequence as set forth in SEQ ID NO: 88, and the VL comprises VL CDR1 with a sequence as set forth in SEQ ID NO: 108, VL CDR2 with a sequence as set forth in SEQ ID NO: 121 and VL CDR3 with a sequence as set forth in SEQ ID NO: 142;
the antibody targeting HER2 or the antigen-binding fragment thereof comprises a heavy chain variable region (VH) and a light chain variable region (VL), wherein the VH comprises VH CDR1 with a sequence as set forth in SEQ ID NO: 13, VH CDR2 with a sequence as set forth in SEQ ID NO: 42 and VH CDR3 with a sequence as set forth in SEQ ID NO: 77, and the VL comprises VL CDR1 with a sequence as set forth in SEQ ID NO: 105, VL CDR2 with a sequence as set forth in SEQ ID NO: 117 and VL CDR3 with a sequence as set forth in SEQ ID NO: 134;
the antibody targeting PD-L1 or the antigen-binding fragment thereof comprises a heavy chain variable region (VH) and a light chain variable region (VL), wherein the VH comprises VH CDR1 with a sequence as set forth in SEQ ID NO: 11, VH CDR2 with a sequence as set forth in SEQ ID NO: 40 and VH CDR3 with a sequence as set forth in SEQ ID NO: 75, and the VL comprises VL CDR1 with a sequence as set forth in SEQ ID NO: 103, VL CDR2 with a sequence as set forth in SEQ ID NO: 117 and VL CDR3 with a sequence as set forth in SEQ ID NO: 132; or, the VH comprises VH CDR1 with a sequence as set forth in SEQ ID NO: 14, VH CDR2 with a sequence as set forth in SEQ ID NO: 43 and VH CDR3 with a sequence as set forth in SEQ ID NO: 78, and the VL comprises VL CDR1 with a sequence as set forth in SEQ ID NO: 106, VL CDR2 with a sequence as set forth in SEQ ID NO: 119 and VL CDR3 with a sequence as set forth in SEQ ID NO: 135;
the antibody targeting B7H4 or the antigen-binding fragment thereof comprises a heavy chain variable region (VH) and a light chain variable region (VL), wherein the VH comprises VH CDR1 with a sequence as set forth in SEQ ID NO: 20, VH CDR2 with a sequence as set forth in SEQ ID NO: 53 and VH CDR3 with a sequence as set forth in SEQ ID NO: 86, and the VL comprises VL CDR1 with a sequence as set forth in SEQ ID NO: 110, VL CDR2 with a sequence as set forth in SEQ ID NO: 118 and VL CDR3 with a sequence as set forth in SEQ ID NO: 140; or the VH comprises VH CDR1 with a sequence as set forth in SEQ ID NO: 21, VH CDR2 with a sequence as set forth in SEQ ID NO: 54 and VH CDR3 with a sequence as set forth in SEQ ID NO: 87, and the VL comprises VL CDR1 with a sequence as set forth in SEQ ID NO: 111, VL CDR2 with a sequence as set forth in SEQ ID NO: 118 and VL CDR3 with a sequence as set forth in SEQ ID NO: 141;
the antibody targeting EPCAM or the antigen-binding fragment thereof comprises heavy chain variable region (VH) and light chain variable region (VL), wherein the VH comprises VH CDR1 with a sequence as set forth in SEQ ID NO: 16, VH CDR2 with a sequence as set forth in SEQ ID NO: 47 and VH CDR3 with a sequence as set forth in SEQ ID NO: 81, and the VL comprises VL CDR1 with a sequence as set forth in SEQ ID NO: 108, VL CDR2 with a sequence as set forth in SEQ ID NO: 121 and VL CDR3 with a sequence as set forth in SEQ ID NO: 138;
the antibody targeting PSMA or the antigen-binding fragment thereof comprises a heavy chain variable region (VH) and a light chain variable region (VL), wherein the VH comprises VH CDR1 with a sequence as set forth in SEQ ID NO: 17, VH CDR2 with a sequence as set forth in SEQ ID NO: 48 and VH CDR3 with a sequence as set forth in SEQ ID NO: 82, and the VL comprises VL CDR1 with a sequence as set forth in SEQ ID NO: 109, VL CDR2 with a sequence as set forth in SEQ ID NO: 122 and VL CDR3 with a sequence as set forth in SEQ ID NO: 139;
the antibody targeting 4-1BB or the antigen-binding fragment thereof comprises a heavy chain variable region (VH), wherein the VH comprises VH CDR1 with a sequence as set forth in SEQ ID NO: 16, VH CDR2 with a sequence as set forth in SEQ ID NO: 49 and VH CDR3 with a sequence as set forth in SEQ ID NO: 83; or the VH comprises VH CDR1 with a sequence as set forth in SEQ ID NO: 18, VH CDR2 with a sequence as set forth in SEQ ID NO: 50 and VH CDR3 with a sequence as set forth in SEQ ID NO: 84; or, the VH comprises VH CDR1 with a sequence as set forth in SEQ ID NO: 19, VH CDR2 with a sequence as set forth in SEQ ID NO: 51 and VH CDR3 with a sequence as set forth in SEQ ID NO: 84; or the VH comprises VH CDR1 with a sequence as set forth in SEQ ID NO: 18, VH CDR2 with a sequence as set forth in SEQ ID NO: 57 and VH CDR3 with a sequence as set forth in SEQ ID NO: 90;
or, the antibody targeting 4-1BB or the antigen-binding fragment thereof comprises a heavy chain variable region (VH) and a light chain variable region (VL), wherein the VH comprises VH CDR1 with a sequence as set forth in SEQ ID NO: 12, VH CDR2 with a sequence as set forth in SEQ ID NO: 41 and VH CDR3 with a sequence as set forth in SEQ ID NO: 76, and the VL comprises VL CDR1 with a sequence as set forth in SEQ ID NO: 104, VL CDR2 with a sequence as set forth in SEQ ID NO: 118 and VL CDR3 with a sequence as set forth in SEQ ID NO: 133; or, the VH comprises VH CDR1 with a sequence as set forth in SEQ ID NO: 12, VH CDR2 with a sequence as set forth in SEQ ID NO: 44 and VH CDR3 with a sequence as set forth in SEQ ID NO: 76, and the VL comprises VL CDR1 with a sequence as set forth in SEQ ID NO: 104, VL CDR2 with a sequence as set forth in SEQ ID NO: 118 and VL CDR3 with a sequence as set forth in SEQ ID NO: 133; and
the antibody targeting OX40 or the antigen-binding fragment thereof comprises a heavy chain variable region (VH), wherein the VH comprises VH CDR1 with a sequence as set forth in SEQ ID NO: 14, VH CDR2 with a sequence as set forth in SEQ ID NO: 52 and VH CDR3 with a sequence as set forth in SEQ ID NO: 85;
more preferably:
the antibody targeting CLDN18.2 or the antigen-binding fragment thereof comprises VH with a sequence as set forth in SEQ ID NO: 165 and VL with a sequence as set forth in SEQ ID NO: 179;
the antibody targeting HER2 or the antigen-binding fragment thereof comprises VH with a sequence as set forth in SEQ ID NO: 152 and VL with a sequence as set forth in SEQ ID NO: 170;
the antibody targeting PD-L1 or the antigen-binding fragment thereof comprises VH with a sequence as set forth in SEQ ID NO: 150 and VL with a sequence as set forth in SEQ ID NO: 168; or comprises VH with a sequence as set forth in SEQ ID NO: 153 and VL with a sequence as set forth in SEQ ID NO: 171;
the antibody targeting B7H4 or the antigen-binding fragment thereof comprises VH with a sequence as set forth in SEQ ID NO: 163 and VL with a sequence as set forth in SEQ ID NO: 177; or comprises VH with a sequence as set forth in SEQ ID NO: 164 and VL with a sequence as set forth in SEQ ID NO: 178;
the antibody targeting EPCAM or the antigen-binding fragment thereof comprises VH with a sequence as set forth in SEQ ID NO: 157 and VL with a sequence as set forth in SEQ ID NO: 175;
the antibody targeting PSMA or the antigen-binding fragment thereof comprises VH with a sequence as set forth in SEQ ID NO: 158 and VL with a sequence as set forth in SEQ ID NO: 176;
the antibody targeting 4-1BB or the antigen-binding fragment thereof comprises VH with a sequence as set forth in SEQ ID NO: 159; or, comprises VH with a sequence as set forth in SEQ ID NO: 160; or, comprises VH with a sequence as set forth in SEQ ID NO: 161; or, comprises VH with a sequence as set forth in SEQ ID NO: 167; or, comprises VH with a sequence as set forth in SEQ ID NO: 151 and VL with a sequence as set forth in SEQ ID NO: 169; or, comprises VH with a sequence as set forth in SEQ ID NO: 154 and VL with a sequence as set forth in SEQ ID NO: 172; and
the antibody targeting OX40 or the antigen-binding fragment thereof comprises VH with a sequence as set forth in SEQ ID NO: 162;
even more preferably:
the antibody targeting CLDN18.2 or the antigen-binding fragment thereof comprises a heavy chain with a sequence as set forth in SEQ ID NO: 196 and a light chain with a sequence as set forth in SEQ ID NO: 210;
the antibody targeting HER2 or the antigen-binding fragment thereof comprises a heavy chain with a sequence as set forth in SEQ ID NO: 183 and a light chain with a sequence as set forth in SEQ ID NO: 201;
the antibody targeting PD-L1 or the antigen-binding fragment thereof comprises a heavy chain with a sequence as set forth in SEQ ID NO: 181 and a light chain with a sequence as set forth in SEQ ID NO: 199; or comprises a heavy chain with a sequence as set forth in SEQ ID NO: 184 and a light chain with a sequence as set forth in SEQ ID NO: 202;
the antibody targeting B7H4 or the antigen-binding fragment thereof comprises a heavy chain with a sequence as set forth in SEQ ID NO: 194 and a light chain with a sequence as set forth in SEQ ID NO: 208; or comprises a heavy chain with a sequence as set forth in SEQ ID NO: 195 and a light chain with a sequence as set forth in SEQ ID NO: 209;
the antibody targeting EPCAM or the antigen-binding fragment thereof comprises a heavy chain with a sequence as set forth in SEQ ID NO: 188 and a light chain with a sequence as set forth in SEQ ID NO: 206;
the antibody targeting PSMA or the antigen-binding fragment thereof comprises a heavy chain with a sequence as set forth in SEQ ID NO: 189 and a light chain with a sequence as set forth in SEQ ID NO: 207;
the antibody targeting 4-1BB or the antigen-binding fragment thereof comprises a heavy chain with a sequence as set forth in SEQ ID NO: 190; or, comprises a heavy chain with a sequence as set forth in SEQ ID NO: 191; or, comprises a heavy chain with a sequence as set forth in SEQ ID NO: 192; or, comprises a heavy chain with a sequence as set forth in SEQ ID NO: 198; or, comprises a heavy chain with a sequence as set forth in SEQ ID NO: 182 and a light chain with a sequence as set forth in SEQ ID NO: 200; or, comprises a heavy chain with a sequence as set forth in SEQ ID NO: 185 and a light chain with a sequence as set forth in SEQ ID NO: 203; and
the antibody targeting OX40 or the antigen-binding fragment thereof comprises a heavy chain with a sequence as set forth in SEQ ID NO: 193.

6. The binding protein according to any one of claims 1-5, wherein
the first protein functional region comprises a heavy chain variable region (VH) and a light chain variable region (VL), wherein the VH comprises VH CDR1 with a sequence as set forth in SEQ ID NO: 13, VH CDR2 with a sequence as set forth in SEQ ID NO: 42 and VH CDR3 with a sequence as set forth in SEQ ID NO: 77, and the VL comprises VL CDR1 with a sequence as set forth in SEQ ID NO: 105, VL CDR2 with a sequence as set forth in SEQ ID NO: 117 and VL CDR3 with a sequence as set forth in SEQ ID NO: 134; and the second protein functional region comprises a heavy chain variable region (VH), wherein the VH comprises VH CDR1 with a sequence as set forth in SEQ ID NO: 19, VH CDR2 with a sequence as set forth in SEQ ID NO: 51 and VH CDR3 with a sequence as set forth in SEQ ID NO: 84;
or, the first protein functional region comprises a heavy chain variable region (VH) and a light chain variable region (VL), wherein the VH comprises VH CDR1 with a sequence as set forth in SEQ ID NO: 20, VH CDR2 with a sequence as set forth in SEQ ID NO: 53 and VH CDR3 with a sequence as set forth in SEQ ID NO: 86, and the VL comprises VL CDR1 with a sequence as set forth in SEQ ID NO: 110, VL CDR2 with a sequence as set forth in SEQ ID NO: 118 and VL CDR3 with a sequence as set forth in SEQ ID NO: 140; and the second protein functional region comprises a heavy chain variable region (VH), wherein the VH comprises VH CDR1 with a sequence as set forth in SEQ ID NO: 16, VH CDR2 with a sequence as set forth in SEQ ID NO: 49 and VH CDR3 with a sequence as set forth in SEQ ID NO: 83;
or, the first protein functional region comprises a heavy chain variable region (VH) and a light chain variable region (VL), wherein the VH comprises VH CDR1 with a sequence as set forth in SEQ ID NO: 20, VH CDR2 with a sequence as set forth in SEQ ID NO: 53 and VH CDR3 with a sequence as set forth in SEQ ID NO: 86, and the VL comprises VL CDR1 with a sequence as set forth in SEQ ID NO: 110, VL CDR2 with a sequence as set forth in SEQ ID NO: 118 and VL CDR3 with a sequence as set forth in SEQ ID NO: 140; and the second protein functional region comprises a heavy chain variable region (VH), wherein the VH comprises VH CDR1 with a sequence as set forth in SEQ ID NO: 18, VH CDR2 with a sequence as set forth in SEQ ID NO: 50 and VH CDR3 with a sequence as set forth in SEQ ID NO: 84;
or, the first protein functional region comprises a heavy chain variable region (VH) and a light chain variable region (VL), wherein the VH comprises VH CDR1 with a sequence as set forth in SEQ ID NO: 20, VH CDR2 with a sequence as set forth in SEQ ID NO: 53 and VH CDR3 with a sequence as set forth in SEQ ID NO: 86, and the VL comprises VL CDR1 with a sequence as set forth in SEQ ID NO: 110, VL CDR2 with a sequence as set forth in SEQ ID NO: 118 and VL CDR3 with a sequence as set forth in SEQ ID NO: 140; and the second protein functional region comprises a heavy chain variable region (VH), wherein the VH comprises VH CDR1 with a sequence as set forth in SEQ ID NO: 18, VH CDR2 with a sequence as set forth in SEQ ID NO: 57 and VH CDR3 with a sequence as set forth in SEQ ID NO: 90;
or, the first protein functional region comprises a heavy chain variable region (VH) and a light chain variable region (VL), wherein the VH comprises VH CDR1 with a sequence as set forth in SEQ ID NO: 14, VH CDR2 with a sequence as set forth in SEQ ID NO: 43 and VH CDR3 with a sequence as set forth in SEQ ID NO: 78, and the VL comprises VL CDR1 with a sequence as set forth in SEQ ID NO: 106, VL CDR2 with a sequence as set forth in SEQ ID NO: 119 and VL CDR3 with a sequence as set forth in SEQ ID NO: 135; and the second protein functional region comprises a heavy chain variable region (VH), wherein the VH comprises a VH CDR1 with a sequence as set forth in SEQ ID NO: 16, a VH CDR2 with a sequence as set forth in SEQ ID NO: 49 and a VH CDR3 with a sequence as set forth in SEQ ID NO: 83;
or, the first protein functional region comprises a heavy chain variable region (VH) and a light chain variable region (VL), wherein the VH comprises VH CDR1 with a sequence as set forth in SEQ ID NO: 14, VH CDR2 with a sequence as set forth in SEQ ID NO: 43 and VH CDR3 with a sequence as set forth in SEQ ID NO: 78, and the VL comprises VL CDR1 with a sequence as set forth in SEQ ID NO: 106, VL CDR2 with a sequence as set forth in SEQ ID NO: 119 and VL CDR3 with a sequence as set forth in SEQ ID NO: 135; and the second protein functional region comprises a heavy chain variable region (VH), wherein the VH comprises VH CDR1 with a sequence as set forth in SEQ ID NO: 18, VH CDR2 with a sequence as set forth in SEQ ID NO: 50 and VH CDR3 with a sequence as set forth in SEQ ID NO: 84;
or, the first protein functional region comprises a heavy chain variable region (VH) and a light chain variable region (VL), wherein the VH comprises VH CDR1 with a sequence as set forth in SEQ ID NO: 14, VH CDR2 with a sequence as set forth in SEQ ID NO: 43 and VH CDR3 with a sequence as set forth in SEQ ID NO: 78, and the VL comprises VL CDR1 with a sequence as set forth in SEQ ID NO: 106, VL CDR2 with a sequence as set forth in SEQ ID NO: 119 and VL CDR3 with a sequence as set forth in SEQ ID NO: 135; and the second protein functional region comprises a heavy chain variable region (VH), wherein the VH comprises VH CDR1 with a sequence as set forth in SEQ ID NO: 19, VH CDR2 with a sequence as set forth in SEQ ID NO: 51 and VH CDR3 with a sequence as set forth in SEQ ID NO: 84;
or, the first protein functional region comprises a heavy chain variable region (VH) and a light chain variable region (VL), wherein the VH comprises VH CDR1 with a sequence as set forth in SEQ ID NO: 16, VH CDR2 with a sequence as set forth in SEQ ID NO: 47 and VH CDR3 with a sequence as set forth in SEQ ID NO: 81, and the VL comprises VL CDR1 with a sequence as set forth in SEQ ID NO: 108, VL CDR2 with a sequence as set forth in SEQ ID NO: 121 and VL CDR3 with a sequence as set forth in SEQ ID NO: 138; and the second protein functional region comprises a heavy chain variable region (VH), wherein the VH comprises VH CDR1 with a sequence as set forth in SEQ ID NO: 16, VH CDR2 with a sequence as set forth in SEQ ID NO: 49 and VH CDR3 with a sequence as set forth in SEQ ID NO: 83;
or, the first protein functional region comprises a heavy chain variable region (VH) and a light chain variable region (VL), wherein the VH comprises VH CDR1 with a sequence as set forth in SEQ ID NO: 16, VH CDR2 with a sequence as set forth in SEQ ID NO: 47 and VH CDR3 with a sequence as set forth in SEQ ID NO: 81, and the VL comprises VL CDR1 with a sequence as set forth in SEQ ID NO: 108, VL CDR2 with a sequence as set forth in SEQ ID NO: 121 and VL CDR3 with a sequence as set forth in SEQ ID NO: 138; and the second protein functional region comprises a heavy chain variable region (VH), wherein the VH comprises VH CDR1 with a sequence as set forth in SEQ ID NO: 18, VH CDR2 with a sequence as set forth in SEQ ID NO: 50 and VH CDR3 with a sequence as set forth in SEQ ID NO: 84;
or, the first protein functional region comprises a heavy chain variable region (VH) and a light chain variable region (VL), wherein the VH comprises VH CDR1 with a sequence as set forth in SEQ ID NO: 16, VH CDR2 with a sequence as set forth in SEQ ID NO: 47 and VH CDR3 with a sequence as set forth in SEQ ID NO: 81, and the VL comprises VL CDR1 with a sequence as set forth in SEQ ID NO: 108, VL CDR2 with a sequence as set forth in SEQ ID NO: 121 and VL CDR3 with a sequence as set forth in SEQ ID NO: 138; and the second protein functional region comprises a heavy chain variable region (VH), wherein the VH comprises VH CDR1 with a sequence as set forth in SEQ ID NO: 19, VH CDR2 with a sequence as set forth in SEQ ID NO: 51 and VH CDR3 with a sequence as set forth in SEQ ID NO: 84;
or, the first protein functional region comprises a heavy chain variable region (VH) and a light chain variable region (VL), wherein the VH comprises VH CDR1 with a sequence as set forth in SEQ ID NO: 17, VH CDR2 with a sequence as set forth in SEQ ID NO: 48 and VH CDR3 with a sequence as set forth in SEQ ID NO: 82, and the VL comprises VL CDR1 with a sequence as set forth in SEQ ID NO: 109, VL CDR2 with a sequence as set forth in SEQ ID NO: 122 and VL CDR3 with a sequence as set forth in SEQ ID NO: 139; and the second protein functional region comprises a heavy chain variable region (VH), wherein the VH comprises VH CDR1 with a sequence as set forth in SEQ ID NO: 16, VH CDR2 with a sequence as set forth in SEQ ID NO: 49 and VH CDR3 with a sequence as set forth in SEQ ID NO: 83;
or, the first protein functional region comprises a heavy chain variable region (VH) and a light chain variable region (VL), wherein the VH comprises VH CDR1 with a sequence as set forth in SEQ ID NO: 17, VH CDR2 with a sequence as set forth in SEQ ID NO: 48 and VH CDR3 with a sequence as set forth in SEQ ID NO: 82, and the VL comprises VL CDR1 with a sequence as set forth in SEQ ID NO: 109, VL CDR2 with a sequence as set forth in SEQ ID NO: 122 and VL CDR3 with a sequence as set forth in SEQ ID NO: 139; and the second protein functional region comprises a heavy chain variable region (VH), wherein the VH comprises VH CDR1 with a sequence as set forth in SEQ ID NO: 18, VH CDR2 with a sequence as set forth in SEQ ID NO: 50 and VH CDR3 with a sequence as set forth in SEQ ID NO: 84;
or, the first protein functional region comprises a heavy chain variable region (VH) and a light chain variable region (VL), wherein the VH comprises VH CDR1 with a sequence as set forth in SEQ ID NO: 17, VH CDR2 with a sequence as set forth in SEQ ID NO: 48 and VH CDR3 with a sequence as set forth in SEQ ID NO: 82, and the VL comprises VL CDR1 with a sequence as set forth in SEQ ID NO: 109, VL CDR2 with a sequence as set forth in SEQ ID NO: 122 and VL CDR3 with a sequence as set forth in SEQ ID NO: 139; and the second protein functional region comprises a heavy chain variable region (VH), wherein the VH comprises VH CDR1 with a sequence as set forth in SEQ ID NO: 19, VH CDR2 with a sequence as set forth in SEQ ID NO: 51 and VH CDR3 with a sequence as set forth in SEQ ID NO: 84;
or, the first protein functional region comprises a heavy chain variable region (VH) and a light chain variable region (VL), wherein the VH comprises VH CDR1 with a sequence as set forth in SEQ ID NO: 22, VH CDR2 with a sequence as set forth in SEQ ID NO: 55 and VH CDR3 with a sequence as set forth in SEQ ID NO: 88, and the VL comprises VL CDR1 with a sequence as set forth in SEQ ID NO: 108, VL CDR2 with a sequence as set forth in SEQ ID NO: 121 and VL CDR3 with a sequence as set forth in SEQ ID NO: 142; and the second protein functional region comprises a heavy chain variable region (VH), wherein the VH comprises VH CDR1 with a sequence as set forth in SEQ ID NO: 16, VH CDR2 with a sequence as set forth in SEQ ID NO: 49 and VH CDR3 with a sequence as set forth in SEQ ID NO: 83;
or, the first protein functional region comprises a heavy chain variable region (VH) and a light chain variable region (VL), wherein the VH comprises VH CDR1 with a sequence as set forth in SEQ ID NO: 22, VH CDR2 with a sequence as set forth in SEQ ID NO: 55 and VH CDR3 with a sequence as set forth in SEQ ID NO: 88, and the VL comprises VL CDR1 with a sequence as set forth in SEQ ID NO: 108, VL CDR2 with a sequence as set forth in SEQ ID NO: 121 and VL CDR3 with a sequence as set forth in SEQ ID NO: 142; and the second protein functional region comprises a heavy chain variable region (VH), wherein the VH comprises VH CDR1 with a sequence as set forth in SEQ ID NO: 18, VH CDR2 with a sequence as set forth in SEQ ID NO: 50 and VH CDR3 with a sequence as set forth in SEQ ID NO: 84;
or, the first protein functional region comprises a heavy chain variable region (VH) and a light chain variable region (VL), wherein the VH comprises VH CDR1 with a sequence as set forth in SEQ ID NO: 22, VH CDR2 with a sequence as set forth in SEQ ID NO: 55 and VH CDR3 with a sequence as set forth in SEQ ID NO: 88, and the VL comprises VL CDR1 with a sequence as set forth in SEQ ID NO: 108, VL CDR2 with a sequence as set forth in SEQ ID NO: 121 and VL CDR3 with a sequence as set forth in SEQ ID NO: 142; and the second protein functional region comprises a heavy chain variable region (VH), wherein the VH comprises VH CDR1 with a sequence as set forth in SEQ ID NO: 19, VH CDR2 with a sequence as set forth in SEQ ID NO: 51 and VH CDR3 with a sequence as set forth in SEQ ID NO: 84;
or, the first protein functional region comprises a heavy chain variable region (VH) and a light chain variable region (VL), wherein the VH comprises VH CDR1 with a sequence as set forth in SEQ ID NO: 14, VH CDR2 with a sequence as set forth in SEQ ID NO: 43 and VH CDR3 with a sequence as set forth in SEQ ID NO: 78, and the VL comprises VL CDR1 with a sequence as set forth in SEQ ID NO: 106, VL CDR2 with a sequence as set forth in SEQ ID NO: 119 and VL CDR3 with a sequence as set forth in SEQ ID NO: 135; and the second protein functional region comprises a heavy chain variable region (VH), wherein the VH comprises VH CDR1 with a sequence as set forth in SEQ ID NO: 14, VH CDR2 with a sequence as set forth in SEQ ID NO: 52 and VH CDR3 with a sequence as set forth in SEQ ID NO: 85;
or, the first protein functional region comprises a heavy chain variable region (VH) and a light chain variable region (VL), wherein the VH comprises VH CDR1 with a sequence as set forth in SEQ ID NO: 20, VH CDR2 with a sequence as set forth in SEQ ID NO: 53 and VH CDR3 with a sequence as set forth in SEQ ID NO: 86, and the VL comprises VL CDR1 with a sequence as set forth in SEQ ID NO: 110, VL CDR2 with a sequence as set forth in SEQ ID NO: 118 and VL CDR3 with a sequence as set forth in SEQ ID NO: 140; and the second protein functional region comprises a heavy chain variable region (VH), wherein the VH comprises VH CDR1 with a sequence as set forth in SEQ ID NO: 14, VH CDR2 with a sequence as set forth in SEQ ID NO: 52 and VH CDR3 with a sequence as set forth in SEQ ID NO: 85;
or, the first protein functional region comprises a heavy chain variable region (VH) and a light chain variable region (VL), wherein the VH comprises VH CDR1 with a sequence as set forth in SEQ ID NO: 16, VH CDR2 with a sequence as set forth in SEQ ID NO: 47 and VH CDR3 with a sequence as set forth in SEQ ID NO: 81, and the VL comprises VL CDR1 with a sequence as set forth in SEQ ID NO: 108, VL CDR2 with a sequence as set forth in SEQ ID NO: 121 and VL CDR3 with a sequence as set forth in SEQ ID NO: 138; and the second protein functional region comprises a heavy chain variable region (VH), wherein the VH comprises VH CDR1 with a sequence as set forth in SEQ ID NO: 14, VH CDR2 with a sequence as set forth in SEQ ID NO: 52 and VH CDR3 with a sequence as set forth in SEQ ID NO: 85;
or, the first protein functional region comprises a heavy chain variable region (VH) and a light chain variable region (VL), wherein the VH comprises VH CDR1 with a sequence as set forth in SEQ ID NO: 17, VH CDR2 with a sequence as set forth in SEQ ID NO: 48 and VH CDR3 with a sequence as set forth in SEQ ID NO: 82, and the VL comprises VL CDR1 with a sequence as set forth in SEQ ID NO: 109, VL CDR2 with a sequence as set forth in SEQ ID NO: 122 and VL CDR3 with a sequence as set forth in SEQ ID NO: 139; and the second protein functional region comprises a heavy chain variable region (VH), wherein the VH comprises VH CDR1 with a sequence as set forth in SEQ ID NO: 14, VH CDR2 with a sequence as set forth in SEQ ID NO: 52 and VH CDR3 with a sequence as set forth in SEQ ID NO: 85;
or, the first protein functional region comprises a heavy chain variable region (VH) and a light chain variable region (VL), wherein the VH comprises VH CDR1 with a sequence as set forth in SEQ ID NO: 22, VH CDR2 with a sequence as set forth in SEQ ID NO: 55 and VH CDR3 with a sequence as set forth in SEQ ID NO: 88, and the VL comprises VL CDR1 with a sequence as set forth in SEQ ID NO: 108, VL CDR2 with a sequence as set forth in SEQ ID NO: 121 and VL CDR3 with a sequence as set forth in SEQ ID NO: 142; and the second protein functional region comprises a heavy chain variable region (VH), wherein the VH comprises VH CDR1 with a sequence as set forth in SEQ ID NO: 14, VH CDR2 with a sequence as set forth in SEQ ID NO: 52 and VH CDR3 with a sequence as set forth in SEQ ID NO: 85;
or, the first protein functional region comprises a heavy chain variable region (VH) and a light chain variable region (VL), wherein the VH comprises VH CDR1 with a sequence as set forth in SEQ ID NO: 21, VH CDR2 with a sequence as set forth in SEQ ID NO: 54 and VH CDR3 with a sequence as set forth in SEQ ID NO: 87, and the VL comprises VL CDR1 with a sequence as set forth in SEQ ID NO: 111, VL CDR2 with a sequence as set forth in SEQ ID NO: 118 and VL CDR3 with a sequence as set forth in SEQ ID NO: 141; and the second protein functional region comprises a heavy chain variable region (VH), wherein the VH comprises VH CDR1 with a sequence as set forth in SEQ ID NO: 18, VH CDR2 with a sequence as set forth in SEQ ID NO: 50 and VH CDR3 with a sequence as set forth in SEQ ID NO: 84;
or, the first protein functional region comprises a heavy chain variable region (VH) and a light chain variable region (VL), wherein the VH comprises VH CDR1 with a sequence as set forth in SEQ ID NO: 14, VH CDR2 with a sequence as set forth in SEQ ID NO: 43 and VH CDR3 with a sequence as set forth in SEQ ID NO: 78, and the VL comprises VL CDR1 with a sequence as set forth in SEQ ID NO: 106, VL CDR2 with a sequence as set forth in SEQ ID NO: 119 and VL CDR3 with a sequence as set forth in SEQ ID NO: 135; and the second protein functional region comprises a heavy chain variable region (VH) and a light chain variable region (VL), wherein the VH comprises VH CDR1 with a sequence as set forth in SEQ ID NO: 12, VH CDR2 with a sequence as set forth in SEQ ID NO: 41 and VH CDR3 with a sequence as set forth in SEQ ID NO: 76, and the VL comprises VL CDR1 with a sequence as set forth in SEQ ID NO: 104, VL CDR2 with a sequence as set forth in SEQ ID NO: 118 and VL CDR3 with a sequence as set forth in SEQ ID NO: 133;
or, the first protein functional region comprises a heavy chain variable region (VH) and a light chain variable region (VL), wherein the VH comprises VH CDR1 with a sequence as set forth in SEQ ID NO: 11, VH CDR2 with a sequence as set forth in SEQ ID NO: 40 and VH CDR3 with a sequence as set forth in SEQ ID NO: 75, and the VL comprises VL CDR1 with a sequence as set forth in SEQ ID NO: 103, VL CDR2 with a sequence as set forth in SEQ ID NO: 117 and VL CDR3 with a sequence as set forth in SEQ ID NO: 132; and the second protein functional region comprises a heavy chain variable region (VH) and a light chain variable region (VL), wherein the VH comprises VH CDR1 with a sequence as set forth in SEQ ID NO: 12, VH CDR2 with a sequence as set forth in SEQ ID NO: 44 and VH CDR3 with a sequence as set forth in SEQ ID NO: 76, and the VL comprises VL CDR1 with a sequence as set forth in SEQ ID NO: 104, VL CDR2 with a sequence as set forth in SEQ ID NO: 118 and VL CDR3 with a sequence as set forth in SEQ ID NO: 133;
preferably:
the first protein functional region comprises VH with a sequence as set forth in SEQ ID NO: 152 and VL with a sequence as set forth in SEQ ID NO: 170; and the second protein functional region comprises VH with a sequence as set forth in SEQ ID NO: 161;
or, the first protein functional region comprises VH with a sequence as set forth in SEQ ID NO: 163 and VL with a sequence as set forth in SEQ ID NO: 177; and the second protein functional region comprises VH with a sequence as set forth in SEQ ID NO: 159;
or, the first protein functional region comprises VH with a sequence as set forth in SEQ ID NO: 163 and VL with a sequence as set forth in SEQ ID NO: 177; and the second protein functional region comprises VH with a sequence as set forth in SEQ ID NO: 160;
or, the first protein functional region comprises VH with a sequence as set forth in SEQ ID NO: 163 and VL with a sequence as set forth in SEQ ID NO: 177; and the second protein functional region comprises VH with a sequence as set forth in SEQ ID NO: 167;
or, the first protein functional region comprises VH with a sequence as set forth in SEQ ID NO: 153 and VL with a sequence as set forth in SEQ ID NO: 171; and the second protein functional region comprises VH with a sequence as set forth in SEQ ID NO: 159;
or, the first protein functional region comprises VH with a sequence as set forth in SEQ ID NO: 153 and VL with a sequence as set forth in SEQ ID NO: 171; and the second protein functional region comprises VH with a sequence as set forth in SEQ ID NO: 160;
or, the first protein functional region comprises VH with a sequence as set forth in SEQ ID NO: 153 and VL with a sequence as set forth in SEQ ID NO: 171; and the second protein functional region comprises VH with a sequence as set forth in SEQ ID NO: 161;
or, the first protein functional region comprises VH with a sequence as set forth in SEQ ID NO: 157 and VL with a sequence as set forth in SEQ ID NO: 175; and the second protein functional region comprises VH with a sequence as set forth in SEQ ID NO: 159;
or, the first protein functional region comprises VH with a sequence as set forth in SEQ ID NO: 157 and VL with a sequence as set forth in SEQ ID NO: 175; and the second protein functional region comprises VH with a sequence as set forth in SEQ ID NO: 160;
or, the first protein functional region comprises VH with a sequence as set forth in SEQ ID NO: 157 and VL with a sequence as set forth in SEQ ID NO: 175; and the second protein functional region comprises VH with a sequence as set forth in SEQ ID NO: 161;
or, the first protein functional region comprises VH with a sequence as set forth in SEQ ID NO: 158 and VL with a sequence as set forth in SEQ ID NO: 176; and the second protein functional region comprises VH with a sequence as set forth in SEQ ID NO: 159;
or, the first protein functional region comprises VH with a sequence as set forth in SEQ ID NO: 158 and VL with a sequence as set forth in SEQ ID NO: 176; and the second protein functional region comprises VH with a sequence as set forth in SEQ ID NO: 160;
or, the first protein functional region comprises VH with a sequence as set forth in SEQ ID NO: 158 and VL with a sequence as set forth in SEQ ID NO: 176; and the second protein functional region comprises VH with a sequence as set forth in SEQ ID NO: 161;
or, the first protein functional region comprises VH with a sequence as set forth in SEQ ID NO: 165 and VL with a sequence as set forth in SEQ ID NO: 179; and the second protein functional region comprises VH with a sequence as set forth in SEQ ID NO: 159;
or, the first protein functional region comprises VH with a sequence as set forth in SEQ ID NO: 165 and VL with a sequence as set forth in SEQ ID NO: 179; and the second protein functional region comprises VH with a sequence as set forth in SEQ ID NO: 160;
or, the first protein functional region comprises VH with a sequence as set forth in SEQ ID NO: 165 and VL with a sequence as set forth in SEQ ID NO: 179; and the second protein functional region comprises VH with a sequence as set forth in SEQ ID NO: 161;
or, the first protein functional region comprises VH with a sequence as set forth in SEQ ID NO: 153 and VL with a sequence as set forth in SEQ ID NO: 171; and the second protein functional region comprises VH with a sequence as set forth in SEQ ID NO: 162;
or, the first protein functional region comprises VH with a sequence as set forth in SEQ ID NO: 163 and VL with a sequence as set forth in SEQ ID NO: 177; and the second protein functional region comprises VH with a sequence as set forth in SEQ ID NO: 162;
or, the first protein functional region comprises VH with a sequence as set forth in SEQ ID NO: 157 and VL with a sequence as set forth in SEQ ID NO: 175; and the second protein functional region comprises VH with a sequence as set forth in SEQ ID NO: 162;
or, the first protein functional region comprises VH with a sequence as set forth in SEQ ID NO: 158 and VL with a sequence as set forth in SEQ ID NO: 176; and the second protein functional region comprises VH with a sequence as set forth in SEQ ID NO: 162;
or, the first protein functional region comprises VH with a sequence as set forth in SEQ ID NO: 165 and VL with a sequence as set forth in SEQ ID NO: 179; and the second protein functional region comprises VH with a sequence as set forth in SEQ ID NO: 162;
or, the first protein functional region comprises VH with a sequence as set forth in SEQ ID NO: 164 and VL with a sequence as set forth in SEQ ID NO: 178; and the second protein functional region comprises VH with a sequence as set forth in SEQ ID NO: 160;
or, the first protein functional region comprises VH with a sequence as set forth in SEQ ID NO: 153 and VL with a sequence as set forth in SEQ ID NO: 171; and the second protein functional region comprises VH with a sequence as set forth in SEQ ID NO: 151 and VL with a sequence as set forth in SEQ ID NO: 169;
or, the first protein functional region comprises VH with a sequence as set forth in SEQ ID NO: 150 and VL with a sequence as set forth in SEQ ID NO: 168; and the second protein functional region comprises VH with a sequence as set forth in SEQ ID NO: 154 and VL with a sequence as set forth in SEQ ID NO: 172;
more preferably, the binding protein comprises the following polypeptide chains:
a polypeptide chain 1 with a sequence as set forth in SEQ ID NO: 201, and a polypeptide chain 2 with a sequence as set forth in SEQ ID NO: 214;
or, a polypeptide chain 1 with a sequence as set forth in SEQ ID NO: 208, and a polypeptide chain 2 with a sequence as set forth in SEQ ID NO: 217;
or, a polypeptide chain 1 with a sequence as set forth in SEQ ID NO: 208, and a polypeptide chain 2 with a sequence as set forth in SEQ ID NO: 218;
or, a polypeptide chain 1 with a sequence as set forth in SEQ ID NO: 208, and a polypeptide chain 2 with a sequence as set forth in SEQ ID NO: 219;
or, a polypeptide chain 1 with a sequence as set forth in SEQ ID NO: 202, and a polypeptide chain 2 with a sequence as set forth in SEQ ID NO: 221;
or, a polypeptide chain 1 with a sequence as set forth in SEQ ID NO: 202, and a polypeptide chain 2 with a sequence as set forth in SEQ ID NO: 222;
or, a polypeptide chain 1 with a sequence as set forth in SEQ ID NO: 202, and a polypeptide chain 2 with a sequence as set forth in SEQ ID NO: 223;
or, a polypeptide chain 1 with a sequence as set forth in SEQ ID NO: 206, and a polypeptide chain 2 with a sequence as set forth in SEQ ID NO: 224;
or, a polypeptide chain 1 with a sequence as set forth in SEQ ID NO: 206, and a polypeptide chain 2 with a sequence as set forth in SEQ ID NO: 225;
or, a polypeptide chain 1 with a sequence as set forth in SEQ ID NO: 206, and a polypeptide chain 2 with a sequence as set forth in SEQ ID NO: 226;
or, a polypeptide chain 1 with a sequence as set forth in SEQ ID NO: 207, and a polypeptide chain 2 with a sequence as set forth in SEQ ID NO: 227;
or, a polypeptide chain 1 with a sequence as set forth in SEQ ID NO: 207, and a polypeptide chain 2 with a sequence as set forth in SEQ ID NO: 228;
or, a polypeptide chain 1 with a sequence as set forth in SEQ ID NO: 207, and a polypeptide chain 2 with a sequence as set forth in SEQ ID NO: 229;
or, a polypeptide chain 1 with a sequence as set forth in SEQ ID NO: 210, and a polypeptide chain 2 with a sequence as set forth in SEQ ID NO: 230;
or, a polypeptide chain 1 with a sequence as set forth in SEQ ID NO: 210, and a polypeptide chain 2 with a sequence as set forth in SEQ ID NO: 231;
or, a polypeptide chain 1 with a sequence as set forth in SEQ ID NO: 210, and a polypeptide chain 2 with a sequence as set forth in SEQ ID NO: 232;
or, a polypeptide chain 1 with a sequence as set forth in SEQ ID NO: 202, and a polypeptide chain 2 with a sequence as set forth in SEQ ID NO: 233;
or, a polypeptide chain 1 with a sequence as set forth in SEQ ID NO: 208, and a polypeptide chain 2 with a sequence as set forth in SEQ ID NO: 236;
or, a polypeptide chain 1 with a sequence as set forth in SEQ ID NO: 206, and a polypeptide chain 2 with a sequence as set forth in SEQ ID NO: 241;
or, a polypeptide chain 1 with a sequence as set forth in SEQ ID NO: 207, and a polypeptide chain 2 with a sequence as set forth in SEQ ID NO: 242;
or, a polypeptide chain 1 with a sequence as set forth in SEQ ID NO: 210, and a polypeptide chain 2 with a sequence as set forth in SEQ ID NO: 243;
or, a polypeptide chain 1 with a sequence as set forth in SEQ ID NO: 208, and a polypeptide chain 2 with a sequence as set forth in SEQ ID NO: 220;
or, a polypeptide chain 1 with a sequence as set forth in SEQ ID NO: 209, and a polypeptide chain 2 with a sequence as set forth in SEQ ID NO: 234;
or, a polypeptide chain 1 with a sequence as set forth in SEQ ID NO: 212, and a polypeptide chain 2 with a sequence as set forth in SEQ ID NO: 235;
or, a polypeptide chain 1 with a sequence as set forth in SEQ ID NO: 237, and a polypeptide chain 2 with a sequence as set forth in SEQ ID NO: 240;
or, a polypeptide chain 1 with a sequence as set forth in SEQ ID NO: 237, and a polypeptide chain 2 with a sequence as set forth in SEQ ID NO: 238;
or, a polypeptide chain 1 with a sequence as set forth in SEQ ID NO: 213, a polypeptide chain 2 with a sequence as set forth in SEQ ID NO: 212, and a polypeptide chain 3 with a sequence as set forth in SEQ ID NO: 203;
or, a polypeptide chain 1 with a sequence as set forth in SEQ ID NO: 216, a polypeptide chain 2 with a sequence as set forth in SEQ ID NO: 215, and a polypeptide chain 3 with a sequence as set forth in SEQ ID NO: 203;
or, a polypeptide chain 1 with a sequence as set forth in SEQ ID NO: 237, and a polypeptide chain 2 with a sequence as set forth in SEQ ID NO: 266;
or, a polypeptide chain 1 with a sequence as set forth in SEQ ID NO: 237, and a polypeptide chain 2 with a sequence as set forth in SEQ ID NO: 267;
or, a polypeptide chain 1 with a sequence as set forth in SEQ ID NO: 237, and a polypeptide chain 2 with a sequence as set forth in SEQ ID NO: 268;
or, a polypeptide chain 1 with a sequence as set forth in SEQ ID NO: 237, and a polypeptide chain 2 with a sequence as set forth in SEQ ID NO: 269;
or, a polypeptide chain 1 with a sequence as set forth in SEQ ID NO: 202, and a polypeptide chain 2 with a sequence as set forth in SEQ ID NO: 270;
or, a polypeptide chain 1 with a sequence as set forth in SEQ ID NO: 212, and a polypeptide chain 2 with a sequence as set forth in SEQ ID NO: 271;
or, a polypeptide chain 1 with a sequence as set forth in SEQ ID NO: 208, and a polypeptide chain 2 with a sequence as set forth in SEQ ID NO: 272;
or, a polypeptide chain 1 with a sequence as set forth in SEQ ID NO: 208, and a polypeptide chain 2 with a sequence as set forth in SEQ ID NO: 273;
or, a polypeptide chain 1 with a sequence as set forth in SEQ ID NO: 237, and a polypeptide chain 2 with a sequence as set forth in SEQ ID NO: 274.

7. An isolated nucleic acid encoding the binding protein according to any one of claims 1-6.

8. A recombinant expression vector comprising the isolated nucleic acid according to claim 7;
preferably, the expression vector comprises a eukaryotic cell expression vector and/or a prokaryotic cell expression vector.

9. A transformant comprising the isolated nucleic acid according to claim 7 or the recombinant expression vector according to claim 8;
preferably, the transformant has a host cell being a prokaryotic cell and/or a eukaryotic cell, wherein the prokaryotic cell is preferably an *E. coli* cell such as TG1 and BL21, and the eukaryotic cell is preferably an HEK293 cell or a CHO cell.

10. A method for preparing a binding protein, comprising culturing the transformant according to claim 9, and obtaining the binding protein from a culture.

11. A pharmaceutical composition comprising the binding protein according to any one of claims 1-6 and optionally a pharmaceutically acceptable carrier;
preferably, the pharmaceutical composition further comprises an additional anti-tumor antibody as an active ingredient.

12. A kit comprising the binding protein according to any one of claims 1-6 and/or the pharmaceutical composition according to claim 11;
preferably, the kit further comprises (i) a device for administering the binding protein or the pharmaceutical composition; and/or (ii) instructions.

13. A combination of kits comprising a kit I comprising the binding protein according to any one of claims 1-6 and/or the pharmaceutical composition according to claim 11, and a kit II comprising an additional antibody or pharmaceutical composition.

14. An administration device comprising the binding protein according to any one of claims 1-6 and/or the pharmaceutical composition according to claim 11;
preferably, the administration device further comprises a component, such as a syringe, an infusion device or an implantable administration device, for containing or administering to a subject the binding protein and/or the pharmaceutical composition.

15. Use of the binding protein according to any one of claims 1-6, the pharmaceutical composition according to claim 11, the kit according to claim 12, the combination of kits according to claim 13 and/or the administration device according to claim 14 in the preparation of a medicament for the diagnosis, prevention and/or treatment of a tumor;
preferably, the tumor is breast cancer, ovarian cancer, endometrial cancer, renal cancer, melanoma, pancreatic cancer, prostate cancer, lung cancer, gastric cancer, liver cancer, esophageal cancer, cervical cancer, head and neck neoplasm, cholangiocarcinoma, gallbladder cancer, bladder cancer, sarcoma and/or colorectal cancer.

16. A method for detecting a specific antigen *in vitro* or *in vivo,* comprising detection with the binding protein according to any one of claims 1-6.

17. A method for diagnosing, preventing and/or treating a tumor, comprising the step of administering to a patient in need thereof the binding protein according to any one of claims 1-6, the pharmaceutical composition according to claim 11, the kit according to claim 12, the combination of kits according to claim 13 and/or the administration device according to claim 14;
preferably, the tumor is selected from one or more of breast cancer, ovarian cancer, endometrial cancer, renal cancer, melanoma, lung cancer, gastric cancer, liver cancer, esophageal cancer, cervical cancer, head and neck neoplasm, cholangiocarcinoma, gallbladder cancer, bladder cancer, sarcoma, colorectal cancer, lymphoma and multiple myeloma.
